# EUROPEAN PATENT APPLICATION

(11) **EP 4 763 854 A2**
(43) Date of publication of application: **24.06.2026**
(21) Application number: 26163405.9
(22) Date of filing: 14.08.2024
(51) Int. Cl.: C07H 15/203

(54) **CONJUGATES COMPRISING CLEAVABLE LINKERS**

(30) Priority: 15.08.2023 US 202363519567 P; 22.07.2024 US 202463673817 P
(62) Divisional of application: 24758462.6
(71) Applicant: AstraZeneca AB, 151 85 Södertälje (SE)
(72) Inventor: BEADLE, Jonathan David, Cambridge CB2 0AA (GB); GALAN COCA, Albano, Cambridge CB2 0AA (GB); COCCO, Mattia, Cambridge CB2 0AA (GB); GOUNDRY, William Robert Fraser, Cambridge CB2 0AA (GB)
(74) Representative: AstraZeneca Intellectual Property

(57) **Abstract**

The specification relates to conjugates comprising a linker of Formula (IA): 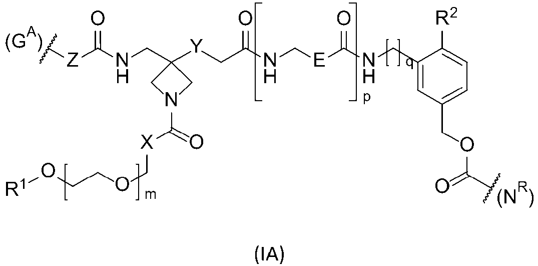 and pharmaceutically acceptable salts thereof. The specification also relates the use of the conjugates for the treatment of diseases such as cancer, and intermediates useful for the synthesis of the conjugates.

## Description

### Cross-Reference to Related Patent Applications

This specification claims the benefit of priority to U.S. Provisional Patent Applications No. 63/519,567 (filed August 15 2023) and 63/673,817 (filed 22 July 2024). The entire text of the above-referenced patent applications are incorporated by reference into this specification.

### Field

This specification relates to certain conjugates comprising cleavable linkers, and to pharmaceutical compositions containing them. This specification also relates to the use of the conjugates in methods of treating diseases such as cancer. This specification further relates to processes and intermediate compounds involved in the preparation of the conjugates.

### Background

Antibody drug conjugates (ADCs) are an established method to deliver a drug to biological target. It is possible to use an enzymatically cleavable linker to release free drug at the target, so that the free drug can have a therapeutic effect. Through transcriptomic and proteomic profiling of various solid tumour cell lines, β-glucuronidase expression has been identified as being upregulated and typically localised to the lysosome. WO2007011968 and WO2015182984 disclose certain antibody drug conjugates comprising β-glucuronidase-cleavable linkers.

There remains a need for conjugates having β-glucuronidase-cleavable linkers that can selectively deliver a drug to a biological target and that have favourable physicochemical properties, including solubility and lipophilicity. The conjugates of the disclosure may be used for the treatment of diseases such as cancer.

### General Description

The specification relates to conjugates comprising a linker of Formula (IA): 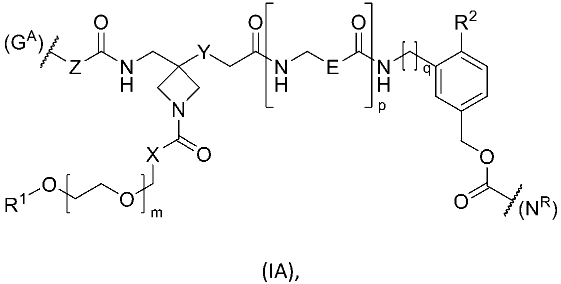
and pharmaceutically acceptable salts thereof, as defined herein.

In a first aspect there is provided a conjugate of Formula (I)

Ab - (G^{A}-J^{A}-D^{R})ₖ (I)

or a pharmaceutically acceptable salt thereof, wherein
Ab is an antibody or antigen-binding fragment thereof,
k is an integer from 1 to 10,
each G^{A} is independently a conjugation group conjugated to the antibody or antigen-binding fragment thereof,
each D^{R} is independently a drug comprising a nitrogen atom N^{R},
each J^{A} is independently a group of Formula (IA) 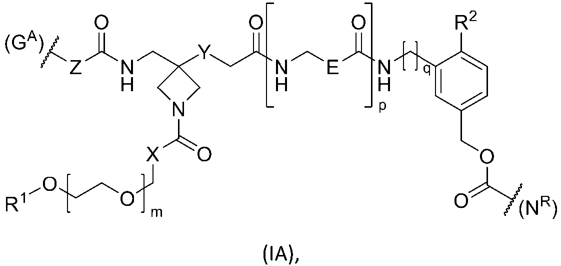
E is (CH₂)ₙ₁, wherein n1 is 0, 1, 2 or 3,
R¹ is C₁₋₄ alkyl or H,
R² is 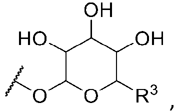 wherein R³ is CO₂H or CH₂OH,
X is (CH₂)_{n2,} 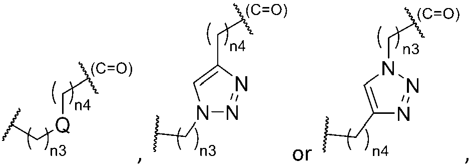 wherein n2 and n4 are 0, 1, 2 or 3, n3 is 1, 2 or 3, and Q is O or NR^{A}, wherein R^{A} is H, C₁₋₄ alkyl or C₃₋₄ cycloalkyl,
Y is O or NR^{B}, wherein R^{B} is H, C₁₋₄ alkyl or C₃₋₄ cycloalkyl,
Z is (CH₂)ₙ₅, wherein n5 is 0, 1, 2, 3, 4 or 5,
m is an integer from 2 to 17,
p is 1 or 0,
q is 1 or 0,
(G^{A}) indicates the point of attachment to G^{A}, and
(N^{R}) indicates the point of attachment to the nitrogen atom N^{R}.

In a further aspect there is provided a conjugate of Formula (IM)

Ab - (G^{A}-J^{A}-D^{M})ₖ (IM)

or a pharmaceutically acceptable salt thereof, wherein
each D^{M} is 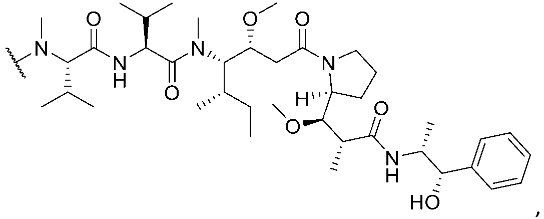
each J^{A} is independently a group of Formula (IMA) 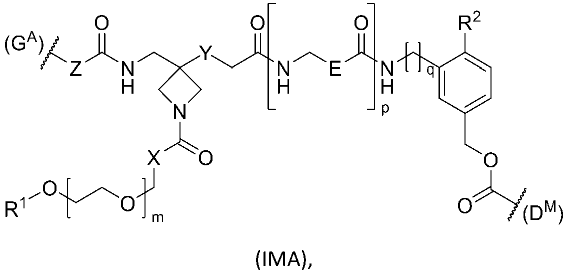
wherein Ab, E, G^{A}, R¹, R², X, Y, Z, k, m, p and q are as defined above for a conjugate of Formula (I), (G^{A}) indicates the point of attachment to G^{A}, and (D^{M}) indicates the point of attachment to D^{M}.

In a further aspect there is provided a conjugate of Formula (IC)

Ab - (G^{A-}J^{A}-D^{C})ₖ (IC)

or a pharmaceutically acceptable salt thereof, wherein
each D^{C} is 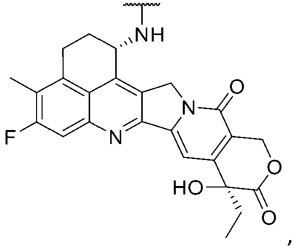
each J^{A} is independently a group of Formula (ICA) 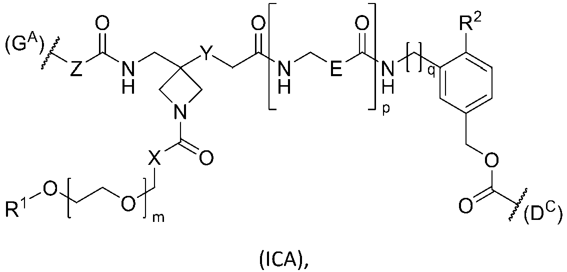
wherein Ab, E, G^{A}, R¹, R², X, Y, Z, k, m, p and q are as defined above for a conjugate of Formula (I), (G^{A}) indicates the point of attachment to G^{A}, and (D^{C}) indicates the point of attachment to D^{C}.

In a further aspect there is provided a pharmaceutical composition comprising a conjugate of Formula (I), (IM) or (IC), or a pharmaceutically acceptable salt thereof and a pharmaceutically acceptable excipient.

In a further aspect there is provided a conjugate of Formula (I), (IM) or (IC), or a pharmaceutically acceptable salt thereof, for use in therapy.

In a further aspect there is provided a conjugate of Formula (I), (IM) or (IC), or a pharmaceutically acceptable salt thereof, for use in the treatment of cancer.

In a further aspect there is provided the use of a conjugate of Formula (I), (IM) or (IC), or a pharmaceutically acceptable salt thereof, in the manufacture of a medicament.

In a further aspect there is provided the use of a conjugate of Formula (I), (IM) or (IC), or a pharmaceutically acceptable salt thereof, in the manufacture of a medicament for the treatment of cancer.

In a further aspect there is provided a method of treating cancer in a patient comprising administering to the patient an effective amount of a conjugate of Formula (I), (IM) or (IC), or a pharmaceutically acceptable salt thereof.

In a further aspect there is provided a compound of Formula (II)

G^{B}-J^{B}-D^{R} (II)

or a salt thereof, wherein G^{B} is a conjugation group for conjugation to an antibody or antigen-binding fragment thereof,
D^{R} is a drug comprising a nitrogen atom N^{R},
J^{B} is a group of Formula (IIA) 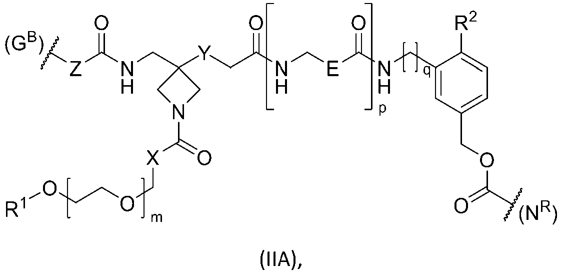
wherein E, R¹, R², X, Y, Z, m, p and q are as defined above for a conjugate of Formula (I), (G^{B}) indicates the point of attachment to G^{B}, and (N^{R}) indicates the point of attachment to the nitrogen atom N^{R}.

In a further aspect there is provided a compound of Formula (IIM)

G^{B}-J^{B}-D^{M} (IIM)

or a salt thereof, wherein G^{B} is a conjugation group for conjugation to an antibody or antigen-binding fragment thereof,
J^{B} is a group of Formula (IIMA) 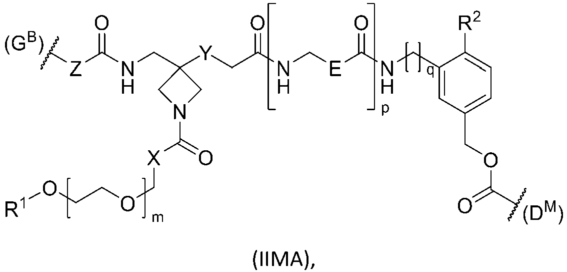
wherein D^{M}, E, R¹, R², X, Y, Z, m, p and q are as defined above for a conjugate of Formula (IM), (G^{B}) indicates the point of attachment to G^{B}, and (D^{M}) indicates the point of attachment to D^{M}.

In a further aspect there is provided a compound of Formula (IIC)

G^{B-}J^{B}-D^{C} (IIC)

or a salt thereof, wherein G^{B} is a conjugation group for conjugation to an antibody or antigen-binding fragment thereof,
J^{B} is a group of Formula (IICA) 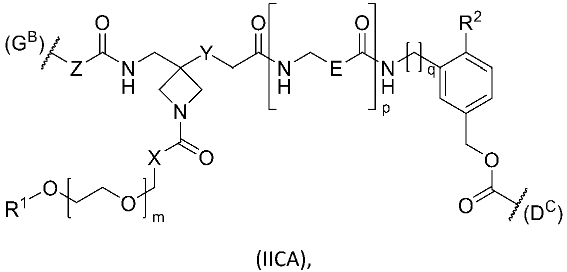
wherein D^{C}, E, R¹, R², X, Y, Z, m, p and q are as defined above for a conjugate of Formula (IC), (G^{B}) indicates the point of attachment to G^{B}, and (D^{C}) indicates the point of attachment to D^{C}.

In a further aspect there is provided intermediates useful for the synthesis of a compound of Formula (II), (IIM) or (IIC), or a salt thereof.

A conjugate of Formula (I), (IM) or (IC), or a pharmaceutically acceptable salt thereof, may undergo enzymatic cleavage to release a free drug. Conjugates of Formula (I), (IM) or (IC) may exhibit improved efficacy and/or advantageous physical properties (for example, higher stability, lower lipophilicity, higher aqueous solubility, higher permeability and/or lower plasma protein binding), and/or favourable toxicity profiles (for example reduced off target toxicity), and/or favourable metabolic or pharmacokinetic profiles, in comparison with other conjugates. Conjugates of Formula (I), (IM) or (IC) may exhibit improved colloidal stability in comparison with other conjugates. As such, conjugates of Formula (I), (IM) or (IC) may be especially suitable for use in therapy, such as the treatment of cancer.

As described herein, conjugates of the present disclosure may undergo slower cleavage in the presence of β-glucuronidase relative to other conjugates. Without wishing to be bound by theory, it is believed that slower cleavage of the linker to release a free drug may result in improved tolerability of the conjugate by reducing the proportion of free drug released in off-target tissue.

### Definitions

So that the present specification may be more readily understood, certain terms are explicitly defined below. In addition, definitions are set forth as appropriate throughout the detailed description. Where examples are provided for a definition, they are not limiting.

The prefix C_{x-y}, where x and y are integers, indicates the numerical range of carbon atoms that are present in a group.

As used herein the term "alkyl" refers to a saturated, linear or branched hydrocarbon radical having the specified number of carbon atoms. Examples of C₁₋₄alkyl groups include methyl (Me), ethyl (Et), n-propyl (*ⁿ*Pr), i-propyl (*ⁱ*Pr), n-butyl (*ⁿ*Bu), i-butyl (*ⁱ*Bu), s-butyl (*^{s}*Bu), and t-butyl (*^{t}*Bu). Examples of C₁₋₆ alkyl groups include methyl, ethyl, n-propyl, i-propyl, n-butyl, i-butyl, s-butyl, t-butyl, n-pentyl and n-hexyl.

As used herein the term "conjugation group for conjugation to an antibody, or antigen-binding fragment thereof" refers to an atom or group of atoms capable of forming a covalent bond to an antibody, or antigen-binding fragment thereof, through a chemical reaction.

The use of " 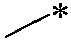 " in formulas of this specification indicates the point of attachment to the antibody or antigen-binding fragment thereof. By way of illustration 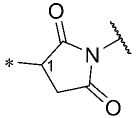 indicates that that there is a covalent bond connecting the antibody, or antigen-binding fragment thereof, to the carbon atom marked 1.

For the avoidance of doubt, the use of " 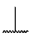 " in formulas of this specification denotes the point of covalent attachment to a group, where the group is other than the antibody or antigen-binding fragment thereof.

Units, prefixes, and symbols are denoted in their International System of Units (SI) accepted form. Numeric ranges are inclusive of the numbers defining the range.

Unless defined otherwise, all technical and scientific terms used herein have the same meaning as commonly understood by one of ordinary skill in the art to which this disclosure is related. For example, the Concise Dictionary of Biomedicine and Molecular Biology, Juo, Pei-Show, 2nd ed., 2002, CRC Press; The Dictionary of Cell and Molecular Biology, 3rd ed., 1999, Academic Press; and the Oxford Dictionary of Biochemistry and Molecular Biology, Revised, 2000, Oxford University Press, provide one of skill with a general dictionary of many of the terms used in this disclosure.

### Description of Figures

Embodiments and experiments illustrating the principles of the disclosure will now be discussed with reference to the accompanying figures in which:
**Figure 1A** shows enzymatic Linker-Payload cleavage for **LP2** and **LP3** with β-glucuronidase.
**Figure 1B** shows enzymatic Linker-Payload cleavage for **LP2** and **LP3** with cathepsin B.
**Figure 1C** shows enzymatic Linker-Payload cleavage for **LP2** and **LP3** with human neutrophil elastase.
**Figure 1D** shows enzymatic Linker-Payload cleavage for **LP2, LP7** (Reference) and **LP8** (Reference) with human neutrophil elastase.
**Figure 1E** shows enzymatic Linker-Payload cleavage for **LP2, LP7** (Reference) and **LP8** (Reference) with β-glucuronidase.

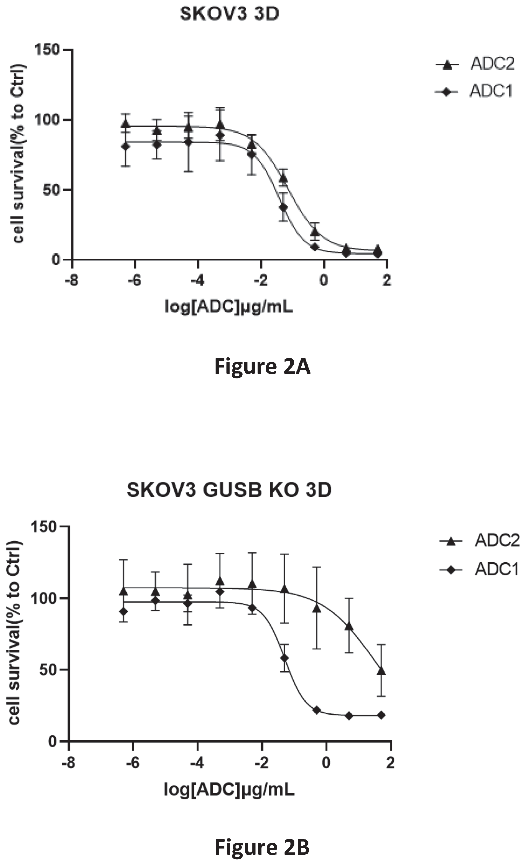

**Figure 2A** shows cytotoxicity data for **ADC1** (control) and **ADC2** in a 3D Her2+++ SKOV3 cell line.
**Figure 2B** shows cytotoxicity data for **ADC1** (control) and **ADC2** in a 3D Her2+++ SKOV3 GUSB KO (β-Glucuronidase knockout) cell line.

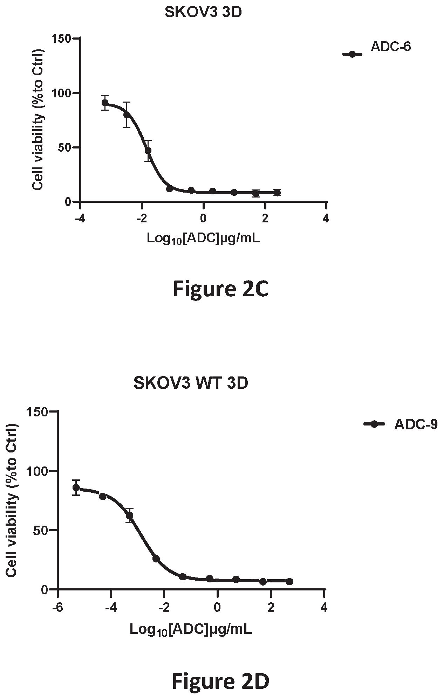

**Figure 2C** shows cytotoxicity data for **ADC6** in a 3D Her2+++ SKOV3 cell line.
**Figure 2D** shows cytotoxicity data for **ADC9** in a 3D Her2+++ SKOV3 cell line.

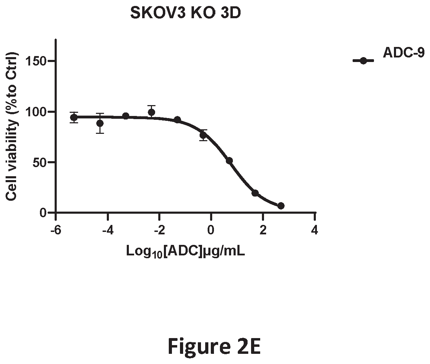

**Figure 2E** shows cytotoxicity data for **ADC9** in a 3D Her2+++ SKOV3 GUSB KO (β-Glucuronidase knockout) cell line.

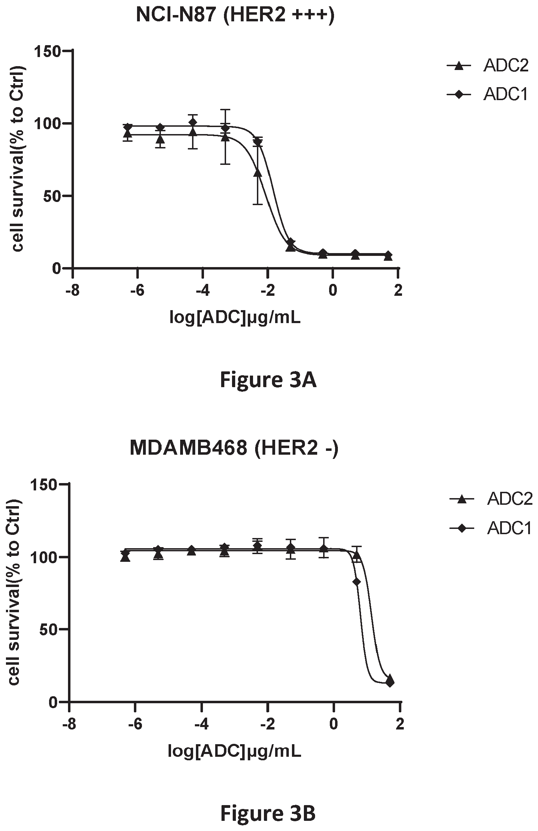

**Figure 3A** shows cytotoxicity data for **ADC1** (control) and **ADC2** in a 2D Her2+++ SKOV3 cell line.
**Figure 3B** shows cytotoxicity data for **ADC1** (control) and **ADC2** in a 2D Her2+++ SKOV3 GUSB KO (β-Glucuronidase knockout) cell line.

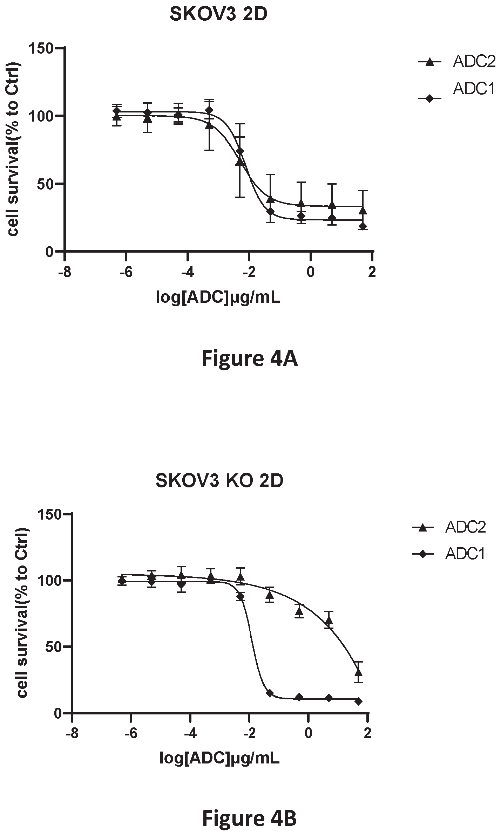

**Figure 4A** shows cytotoxicity data for **ADC1** (control) and **ADC2** in a Her2+++ NCI-N87 cell line.
**Figure 4B** shows cytotoxicity data for **ADC1** (control) and **ADC2** in a Her2- MDAMB468 cell line.

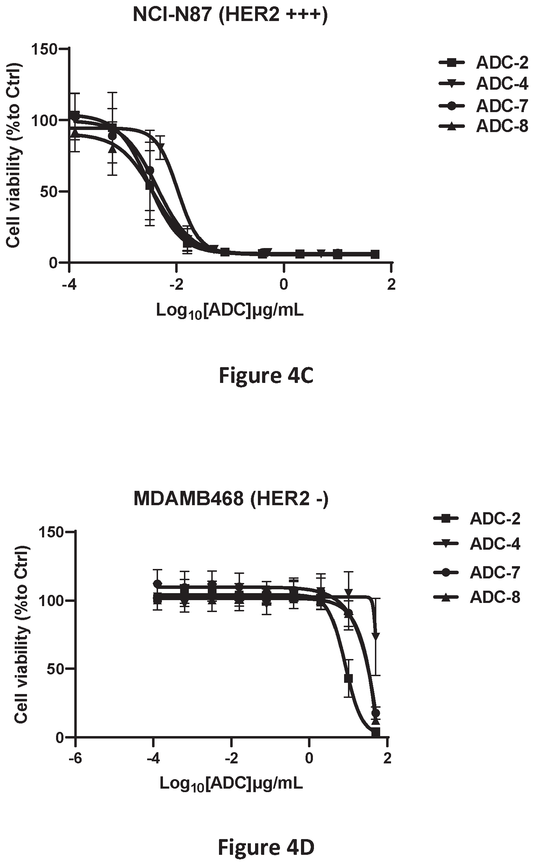

**Figure 4C** shows cytotoxicity data for **ADC2, ADC4, ADC7** (Reference) and **ADC8** (Reference) in a Her2+++ NCI-N87 cell line.
**Figure 4D** shows cytotoxicity data for **ADC2, ADC4, ADC7** (Reference) and **ADC8** (Reference) in a Her2- MDAMB468 cell line.

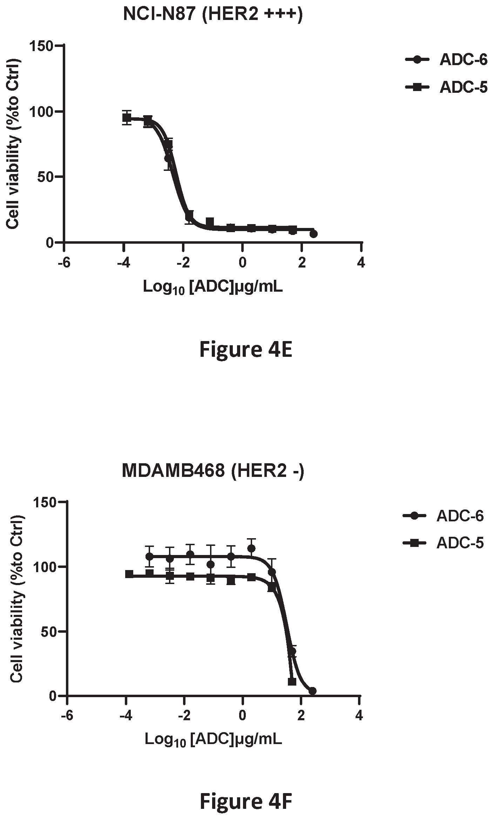

**Figure 4E** shows cytotoxicity data for **ADCS** and **ADC6** in a Her2+++ NCI-N87 cell line.
**Figure 4F** shows cytotoxicity data for **ADC5** and **ADC6** in a Her2- MDAMB468 cell line.

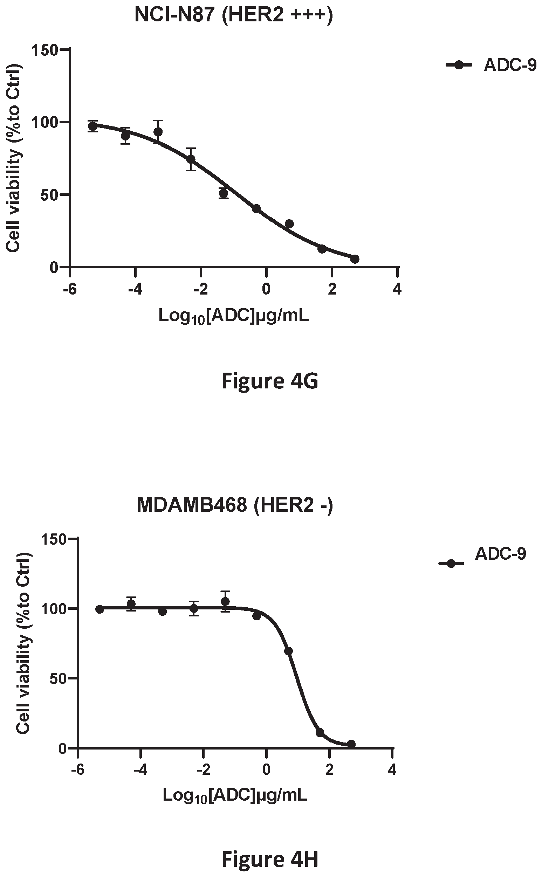

**Figure 4G** shows cytotoxicity data for **ADC9** in a Her2+++ NCI-N87 cell line.
**Figure 4H** shows cytotoxicity data for **ADC9** in a Her2- MDAMB468 cell line.

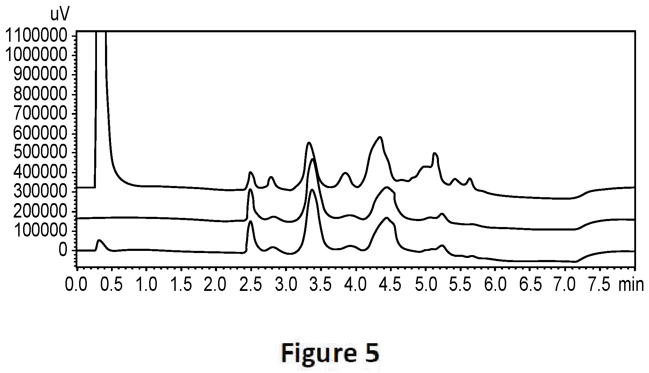

**Figure 5** shows a Hydrophobic Interaction Chromatography (HIC) plot (Run 1) for **ADC2** (top), **ADC7** (Reference) (middle) and **ADC8** (Reference) (bottom).

### Detailed Description

In one aspect, this specification provides a conjugate of Formula (I), (IM) or (IC), or a pharmaceutically acceptable salt thereof, as defined above.

In embodiments there is provided a conjugate of Formula (I), (IM) or (IC), or a pharmaceutically acceptable salt thereof, wherein k is 1, 2, 3, 4, 5, 6, 7, 8, 9 or 10. In further embodiments k is an integer from 2 to 10. In further embodiments k is an integer from 2 to 8. In further embodiments k is 4. In further embodiments k is 8.

In embodiments there is provided a conjugate of Formula (I), (IM) or (IC), or a pharmaceutically acceptable salt thereof, wherein G^{A} is selected from 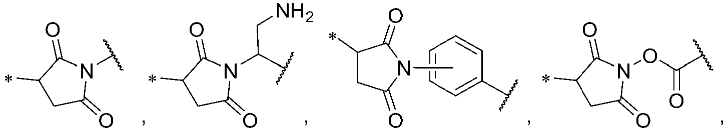 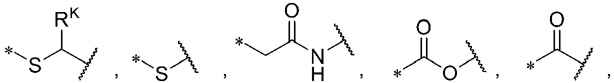 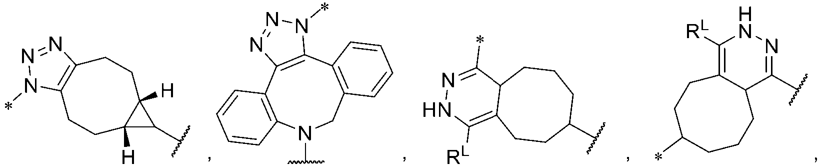 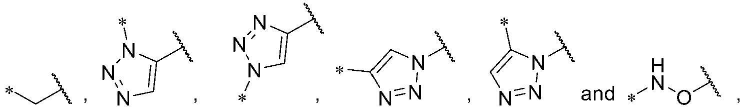 wherein R^{K} is H or CH₃, R^{L} is C₁₋₆ alkyl, and 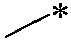 indicates the point of attachment to the antibody, or antigen-binding fragment thereof.

In embodiments there is provided a conjugate of Formula (I), (IM) or (IC), or a pharmaceutically acceptable salt thereof, wherein G^{A} is selected from

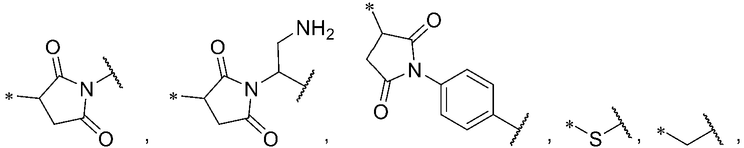

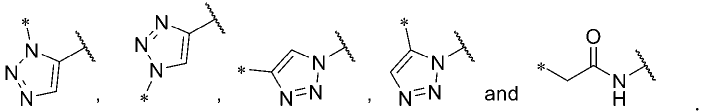

In embodiments there is provided a conjugate of Formula (I), (IM) or (IC), or a pharmaceutically acceptable salt thereof, wherein G^{A} is

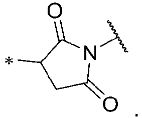

In embodiments there is provided a conjugate of Formula (I), (IM) or (IC), or a pharmaceutically acceptable salt thereof, wherein G^{A} is

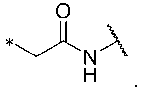

In embodiments there is provided a conjugate of Formula (I), (IM) or (IC), or a pharmaceutically acceptable salt thereof, wherein G^{A} is

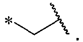

In embodiments there is provided a conjugate of Formula (I), (IM) or (IC), or a pharmaceutically acceptable salt thereof, wherein G^{A} is

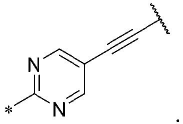

In embodiments there is provided a conjugate of Formula (I), (IM) or (IC), or a pharmaceutically acceptable salt thereof, wherein E is (CH₂)ₙ₁, wherein n1 is 0, 1, 2 or 3. In further embodiments E is a covalent bond. In further embodiments E is CH₂. In further embodiments E is (CH₂)₂. In further embodiments E is (CH₂)₃.

In embodiments there is provided a conjugate of Formula (I), (IM) or (IC), or a pharmaceutically acceptable salt thereof, wherein R¹ is C₁₋₄ alkyl or H. In further embodiments R¹ is C₁₋₄ alkyl. In further embodiments R¹ is CH₃.

In embodiments there is provided a conjugate of Formula (I), (IM) or (IC), or a pharmaceutically acceptable salt thereof, wherein R² is 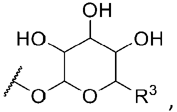 wherein R³ is CO₂H or CH₂OH. In further embodiments, R³ is CO₂H.

In embodiments there is provided a conjugate of Formula (I), (IM) or (IC), or a pharmaceutically acceptable salt thereof, wherein R² is 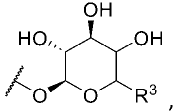 wherein R³ is CO₂H or CH₂OH. In further embodiments, R³ is CO₂H.

In embodiments there is provided a conjugate of Formula (I), (IM) or (IC), or a pharmaceutically acceptable salt thereof, wherein R² is 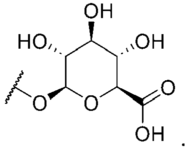 In embodiments there is provided a conjugate of Formula (I), (IM) or (IC), or a pharmaceutically acceptable salt thereof, wherein R² is

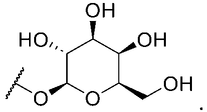

In embodiments there is provided a conjugate of Formula (I), (IM) or (IC), or a pharmaceutically acceptable salt thereof, wherein X is (CH₂)ₙ₂, wherein n2 is 0, 1, 2 or 3. In further embodiments X is a covalent bond. In further embodiments X is CH₂. In further embodiments X is (CH₂)₂. In further embodiments X is (CH₂)₃.

In embodiments there is provided a conjugate of Formula (I), (IM) or (IC), or a pharmaceutically acceptable salt thereof, wherein X is 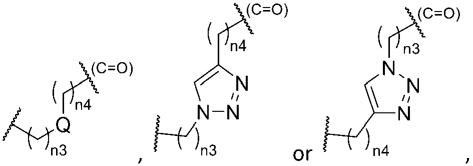 wherein n4 is 0, 1, 2 or 3, n3 is 1, 2 or 3, and Q is O or NR^{A}, and wherein R^{A} is H, C₁₋₄ alkyl or C₃₋₄ cycloalkyl. As used herein (C=O) indicates the point of attachment to C=O.

In embodiments there is provided a conjugate of Formula (I), (IM) or (IC), or a pharmaceutically acceptable salt thereof, wherein Y is O.

In embodiments there is provided a conjugate of Formula (I), (IM) or (IC), or a pharmaceutically acceptable salt thereof, wherein Y is NR^{B}, wherein R^{B} is H, C₁₋₄ alkyl or C₃₋₄ cycloalkyl. In further embodiments, Y is NH.

In embodiments there is provided a conjugate of Formula (I), (IM) or (IC), or a pharmaceutically acceptable salt thereof, wherein Z is (CH₂)ₙ₅, wherein n5 is 1, 2, 3, 4 or 5. In further embodiments Z is CH₂. In further embodiments Z is (CH₂)₂. In further embodiments Z is (CH₂)₃. In further embodiments Z is (CH₂)₄. In further embodiments Z is (CH₂)₅.

In embodiments there is provided a conjugate of Formula (I), (IM) or (IC), or a pharmaceutically acceptable salt thereof, wherein m is 2, 3, 4, 5, 6, 7, 8, 9, 10, 11, 12, 13, 14, 15, 16 or 17. In further embodiments m is an integer from 6 to 16. In further embodiments m is an integer from 7 to 15. In further embodiments m is an integer from 8 to 14. In further embodiments m is an integer from 9 to 13. In further embodiments m is an integer from 10 to 12. In further embodiments m is 11.

In embodiments there is provided a conjugate of Formula (I), (IM) or (IC), or a pharmaceutically acceptable salt thereof, wherein p is 1.

In embodiments there is provided a conjugate of Formula (I), (IM) or (IC), or a pharmaceutically acceptable salt thereof, wherein p is 0.

In embodiments there is provided a conjugate of Formula (I), (IM) or (IC), or a pharmaceutically acceptable salt thereof, wherein q is 1.

In embodiments there is provided a conjugate of Formula (I), (IM) or (IC), or a pharmaceutically acceptable salt thereof, wherein q is 0.

In embodiments there is provided a conjugate of Formula (I), or a pharmaceutically acceptable salt thereof, wherein each J^{A} is a group of Formula (IB)

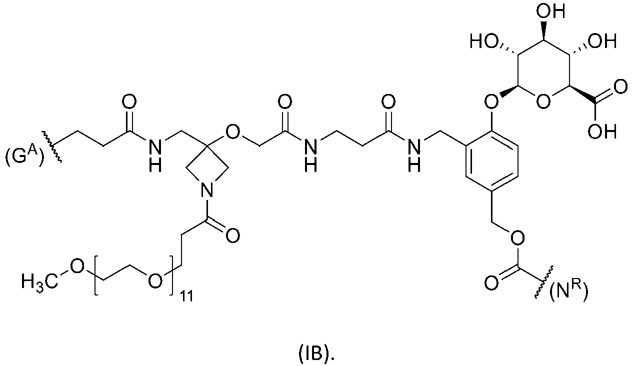

In embodiments there is provided a conjugate of Formula (I), or a pharmaceutically acceptable salt thereof, wherein each J^{A} is a group of Formula (IB1)

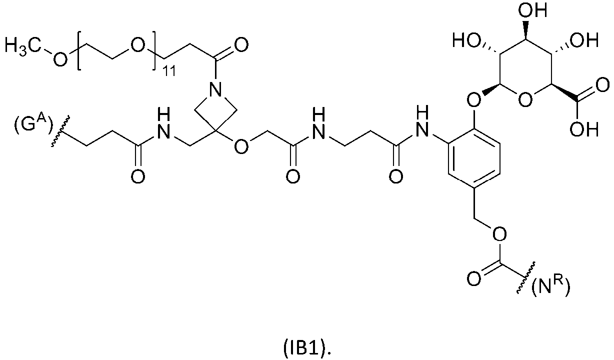

In embodiments there is provided a conjugate of Formula (I), or a pharmaceutically acceptable salt thereof, wherein each J^{A} is a group of Formula (IB2)

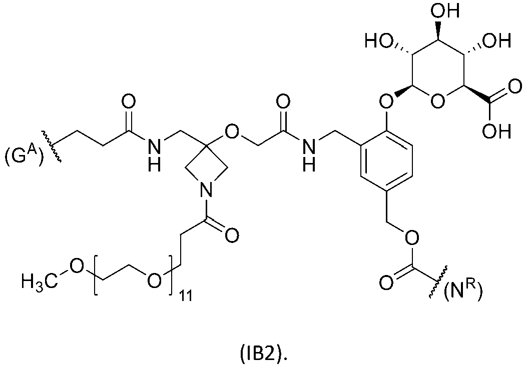

In embodiments there is provided a conjugate of Formula (I), or a pharmaceutically acceptable salt thereof, wherein each J^{A} is a group of Formula (IB3)

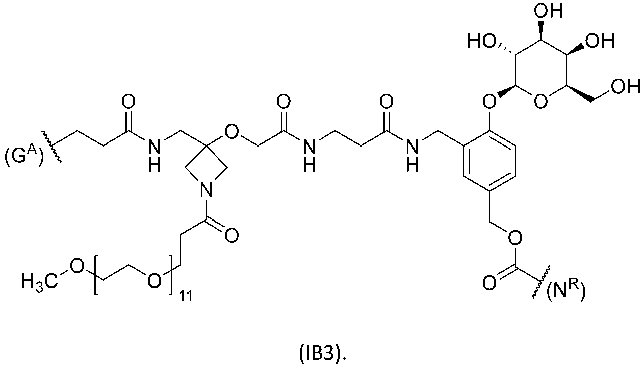

In embodiments there is provided a conjugate of Formula (I), or a pharmaceutically acceptable salt thereof, wherein each J^{A} is a group of Formula (IB4)

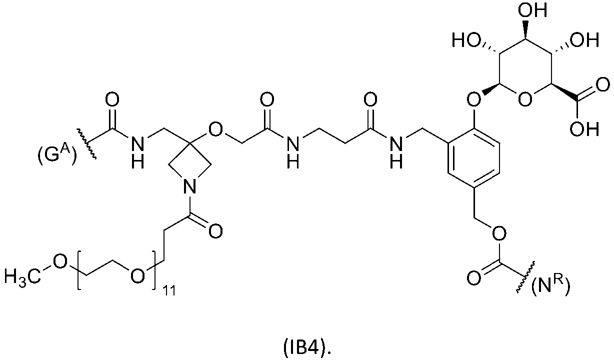

In embodiments there is provided a conjugate of Formula (IM), or a pharmaceutically acceptable salt thereof, wherein each J^{A} is a group of Formula (IMB)

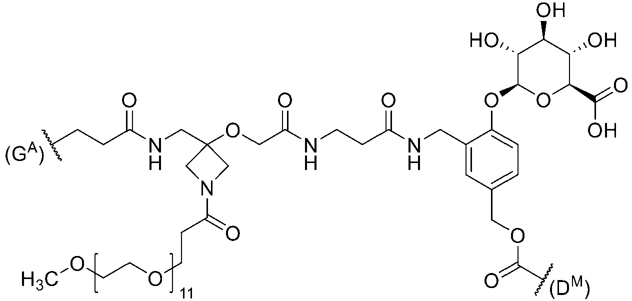

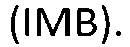

In embodiments there is provided a conjugate of Formula (IM), or a pharmaceutically acceptable salt thereof, wherein each J^{A} is a group of Formula (IMB1)

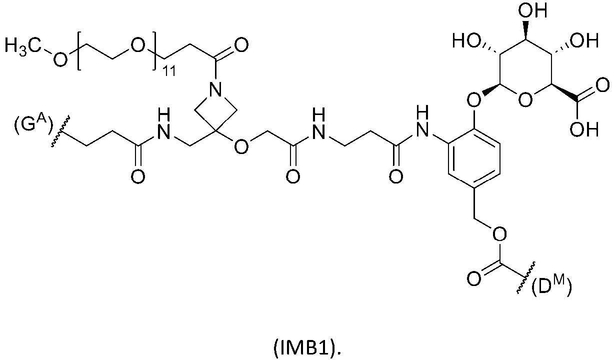

In embodiments there is provided a conjugate of Formula (IM), or a pharmaceutically acceptable salt thereof, wherein each J^{A} is a group of Formula (IMB2)

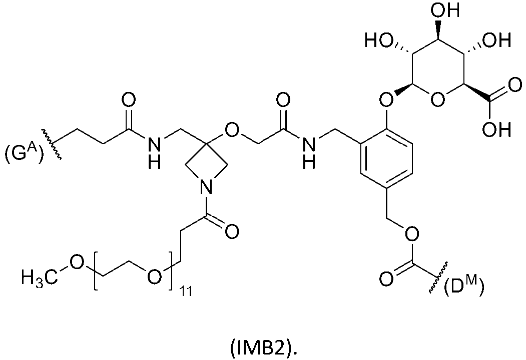

In embodiments there is provided a conjugate of Formula (IM), or a pharmaceutically acceptable salt thereof, wherein each J^{A} is a group of Formula (IMB3)

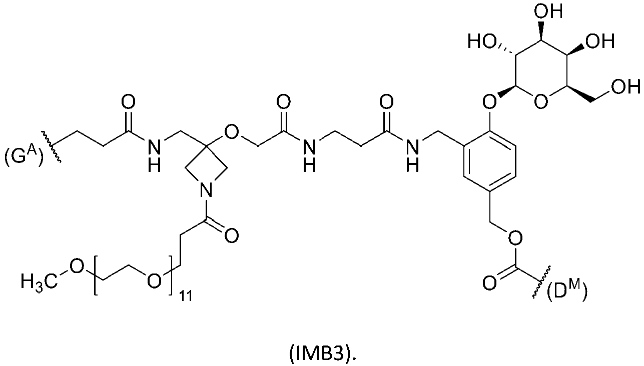

In embodiments there is provided a conjugate of Formula (IM), or a pharmaceutically acceptable salt thereof, wherein each J^{A} is a group of Formula (IMB4)

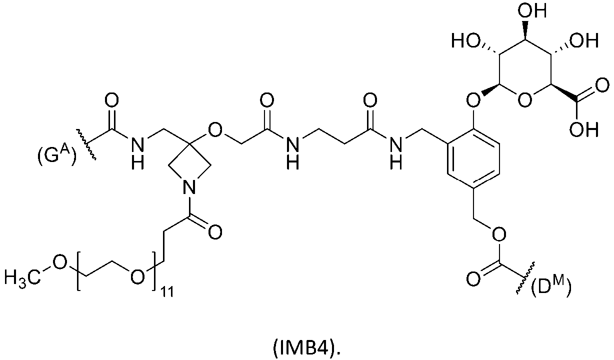

In embodiments there is provided a conjugate of Formula (IC), or a pharmaceutically acceptable salt thereof, wherein each J^{A} is a group of Formula (ICB)

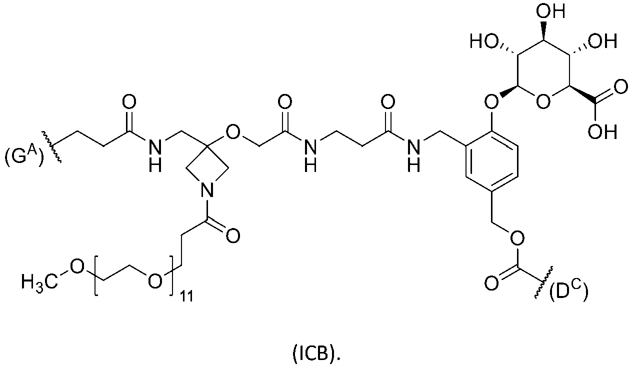

In embodiments there is provided a conjugate of Formula (IC), or a pharmaceutically acceptable salt thereof, wherein each J^{A} is a group of Formula (ICB1)

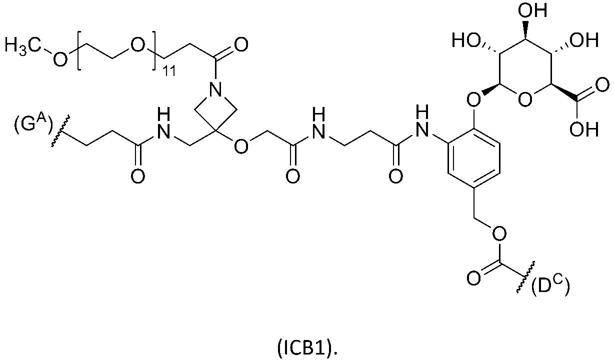

In embodiments there is provided a conjugate of Formula (IC), or a pharmaceutically acceptable salt thereof, wherein each J^{A} is a group of Formula (ICB2)

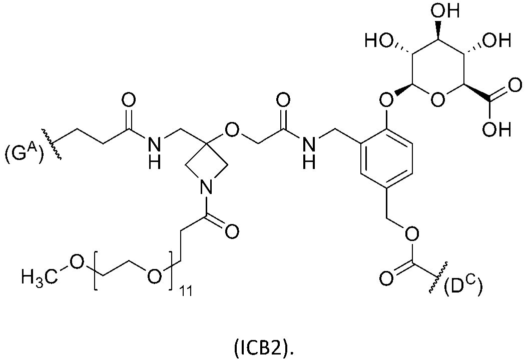

In embodiments there is provided a conjugate of Formula (IC), or a pharmaceutically acceptable salt thereof, wherein each J^{A} is a group of Formula (ICB3)

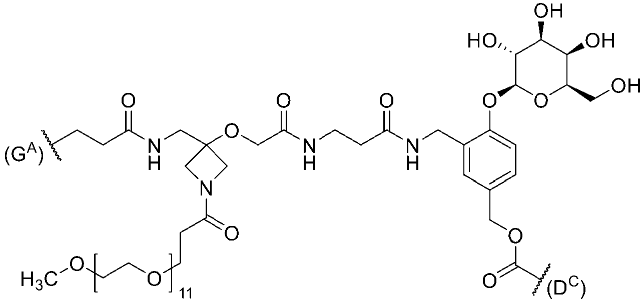

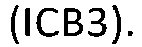

In embodiments there is provided a conjugate of Formula (IC), or a pharmaceutically acceptable salt thereof, wherein each J^{A} is a group of Formula (ICB4)

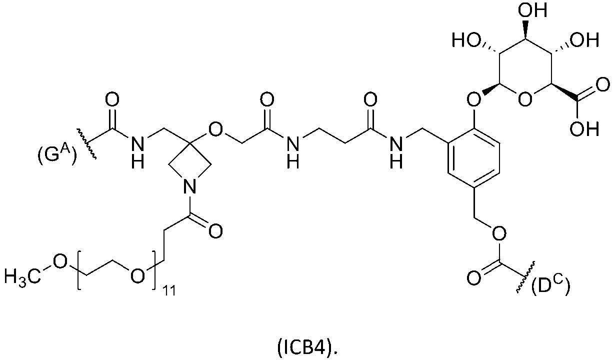

In embodiments there is provided a conjugate of Formula (IM), or a pharmaceutically acceptable salt thereof, wherein each (G^{A}-J^{A}-D^{M}) is

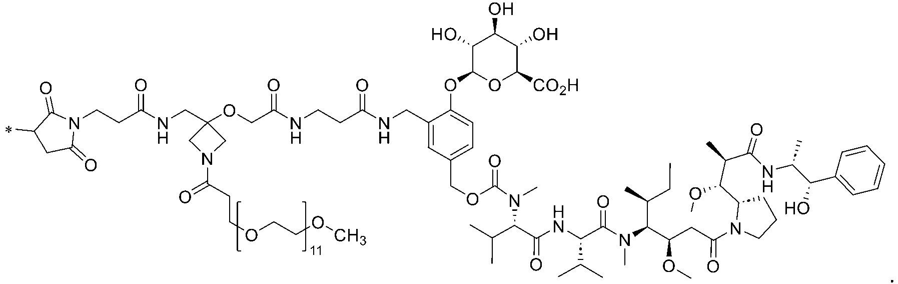

In embodiments there is provided a conjugate of Formula (IM), or a pharmaceutically acceptable salt thereof, wherein each (G^{A}-J^{A}-D^{M}) is

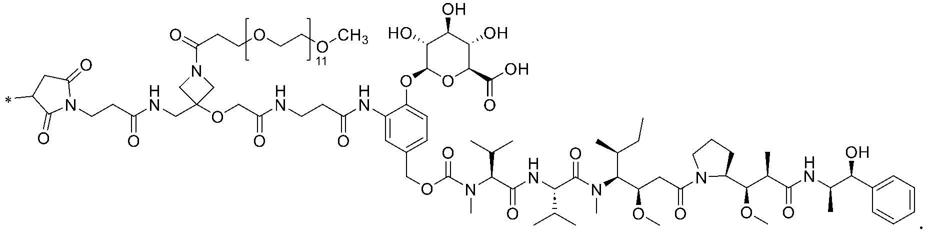

In embodiments there is provided a conjugate of Formula (IM), or a pharmaceutically acceptable salt thereof, wherein each (G^{A}-J^{A}-D^{M}) is

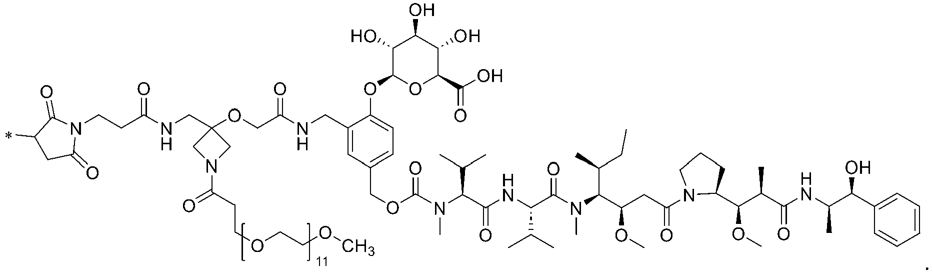

In embodiments there is provided a conjugate of Formula (IM), or a pharmaceutically acceptable salt thereof, wherein each (G^{A}-J^{A}-D^{M}) is

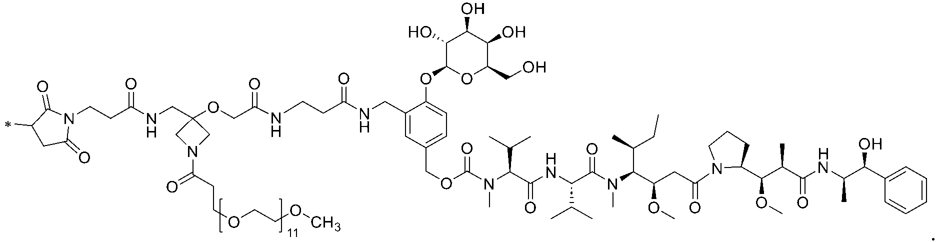

In embodiments there is provided a conjugate of Formula (IM), or a pharmaceutically acceptable salt thereof, wherein each (G^{A}-J^{A}-D^{M}) is

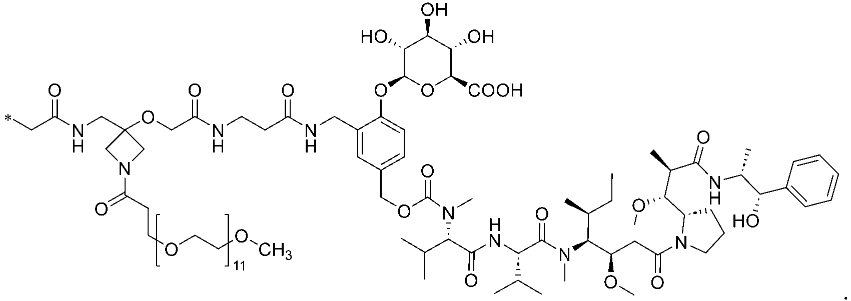

In embodiments there is provided a conjugate of Formula (IC), or a pharmaceutically acceptable salt thereof, wherein each (G^{A}-J^{A}-D^{C}) is

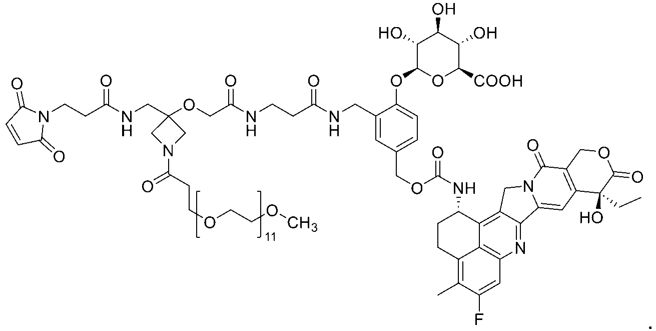

In a further aspect there is provided a compound of Formula (II)

G^{B}-J^{B}-D^{R} (II)

or a salt thereof, wherein G^{B} is a conjugation group for conjugation to an antibody or antigen-binding fragment thereof,
D^{R} is a drug comprising a nitrogen atom N^{R},
J^{B} is a group of Formula (IIA) 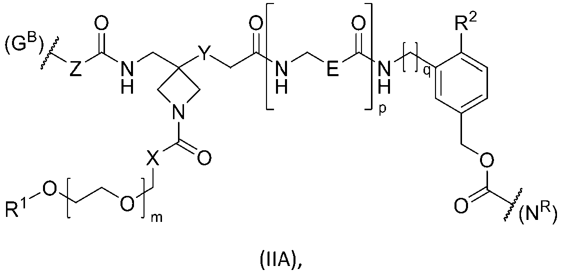
wherein E, R¹, R², X, Y, Z, m, p and q are as defined in any of the embodiments of a conjugate of Formula (I) disclosed herein, (G^{B}) indicates the point of attachment to G^{B}, and (N^{R}) indicates the point of attachment to the nitrogen atom N^{R}.

In a further aspect there is provided a compound of Formula (IIM)

G^{B}-J^{B}-D^{M} (IIM)

or a salt thereof, wherein G^{B} is a conjugation group for conjugation to an antibody or antigen-binding fragment thereof,
J^{B} is a group of Formula (IIMA) 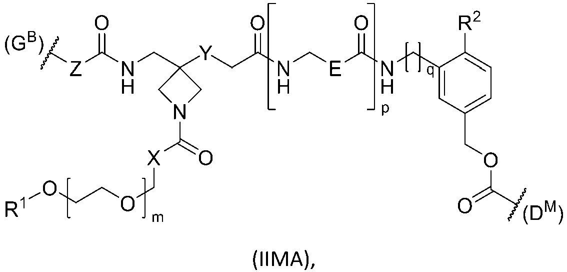
wherein D^{M}, E, R¹, R², X, Y, Z, m, p and q are as defined in any embodiment of a conjugate of Formula (IM) disclosed herein, (G^{B}) indicates the point of attachment to G^{B}, and (D^{M}) indicates the point of attachment to D^{M}.

In a further aspect there is provided a compound of Formula (IIC)

G^{B}-J^{B}-D^{C} (IIC)

or a salt thereof, wherein G^{B} is a conjugation group for conjugation to an antibody or antigen-binding fragment thereof,
J^{B} is a group of Formula (IICA) 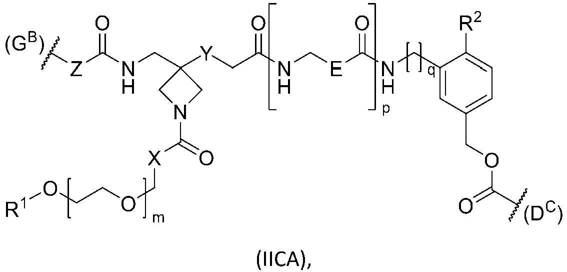
wherein D^{C}, E, R¹, R², X, Y, Z, m, p and q are as defined in any embodiment of a conjugate of Formula (IC) disclosed herein, (G^{B}) indicates the point of attachment to G^{B}, and (D^{C}) indicates the point of attachment to D^{C}.

In embodiments there is provided a compound of Formula (II), (IIM) or (IIC), or a salt thereof, wherein G^{B} is selected from 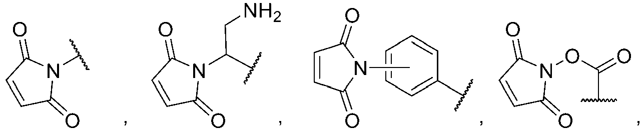 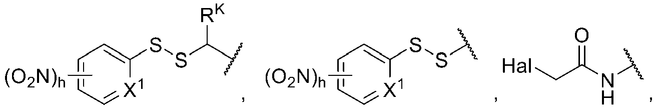 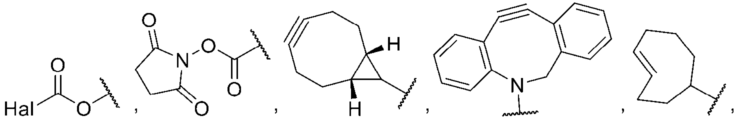 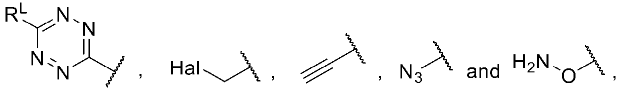 wherein X¹ is CH or N, h is 0 or 1, Hal is Cl, Br or I, R^{K} is H or CH₃, and R^{L} is C₁₋₆ alkyl.

In embodiments there is provided a compound of Formula (II), (IIM) or (IIC), or a salt thereof, wherein G^{B} is selected from

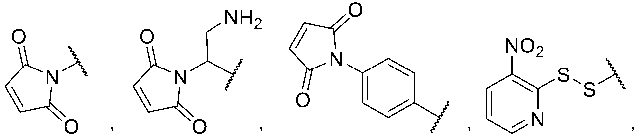

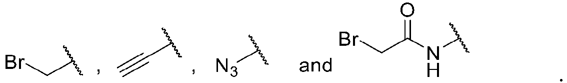

In embodiments there is provided a compound of Formula (II), (IIM) or (IIC), or a salt thereof, wherein G^{B} is

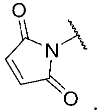

In embodiments there is provided a compound of Formula (II), (IIM) or (IIC), or a salt thereof, wherein G^{B} is

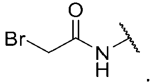

In embodiments there is provided a compound of Formula (II), (IIM) or (IIC), or a salt thereof, wherein G^{B} is

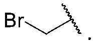

In embodiments there is provided a compound of Formula (II), (IIM) or (IIC), or a salt thereof, wherein G^{B} is

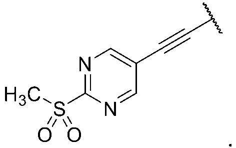

In embodiments there is provided a compound of Formula (II) or a salt thereof, wherein J^{B} is a group of Formula (IIB)

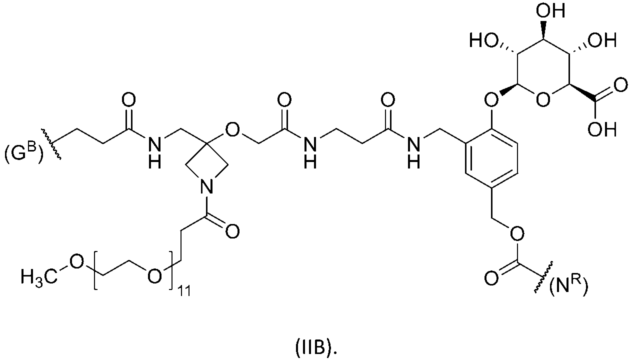

In embodiments there is provided a compound of Formula (II) or a salt thereof, wherein J^{B} is a group of Formula (IIB1)

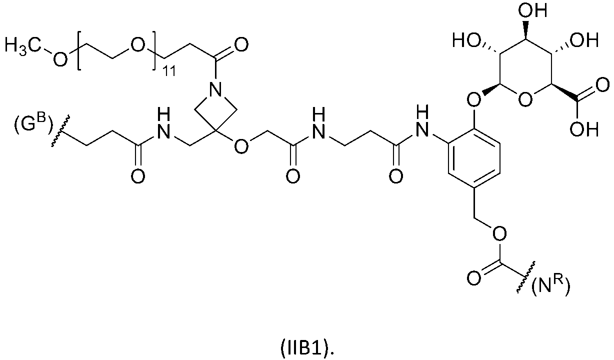

In embodiments there is provided a compound of Formula (II) or a salt thereof, wherein J^{B} is a group of Formula (IIB2)

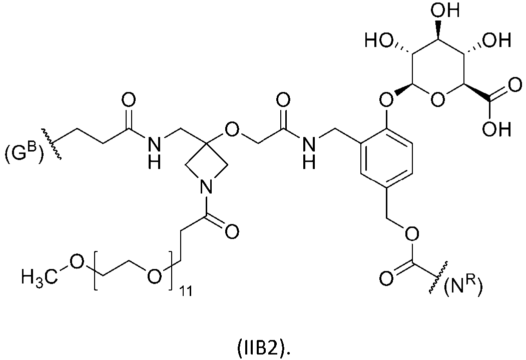

In embodiments there is provided a compound of Formula (II) or a salt thereof, wherein J^{B} is a group of Formula (IIB3)

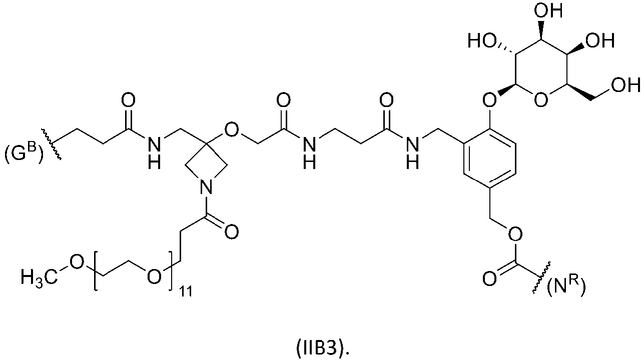

In embodiments there is provided a compound of Formula (II) or a salt thereof, wherein J^{B} is a group of Formula (IIB4)

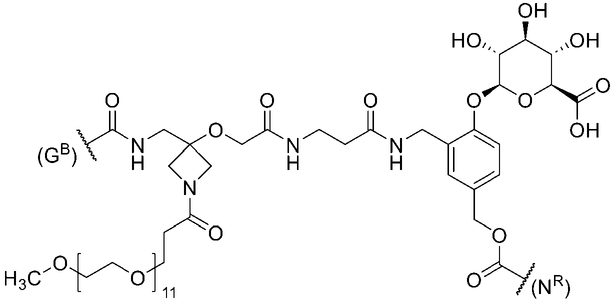

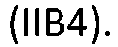

In embodiments there is provided a compound of Formula (IIM) or a salt thereof, wherein J^{B} is a group of Formula (IIMB)

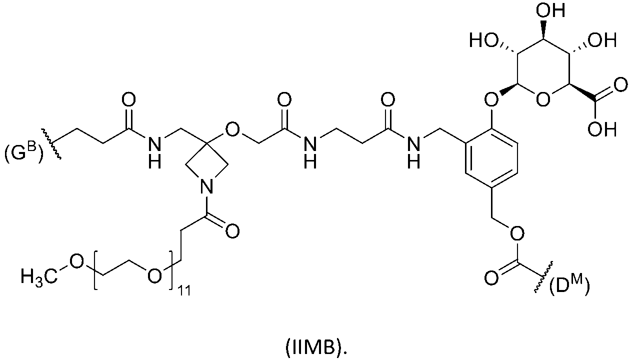

In embodiments there is provided a compound of Formula (IIM) or a salt thereof, wherein J^{B} is a group of Formula (IIMB1)

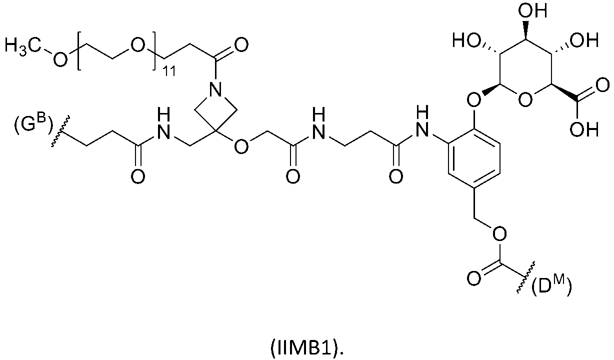

In embodiments there is provided a compound of Formula (IIM) or a salt thereof, wherein J^{B} is a group of Formula (IIMB2)

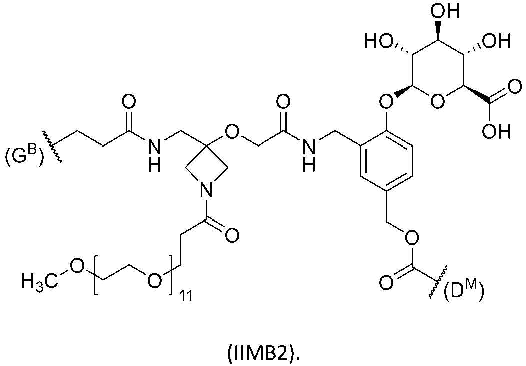

In embodiments there is provided a compound of Formula (IIM) or a salt thereof, wherein J^{B} is a group of Formula (IIMB3)

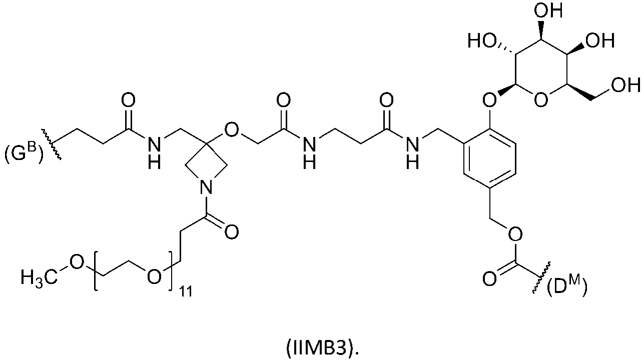

In embodiments there is provided a compound of Formula (IIM), or a salt thereof, wherein J^{B} is a group of Formula (IIMB4)

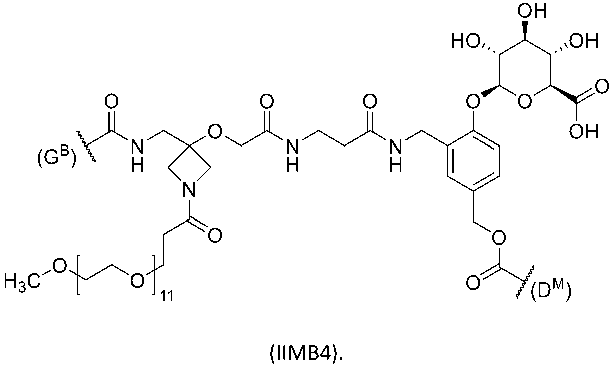

In embodiments there is provided a compound of Formula (IIC) or a salt thereof, wherein J^{B} is a group of Formula (IICB)

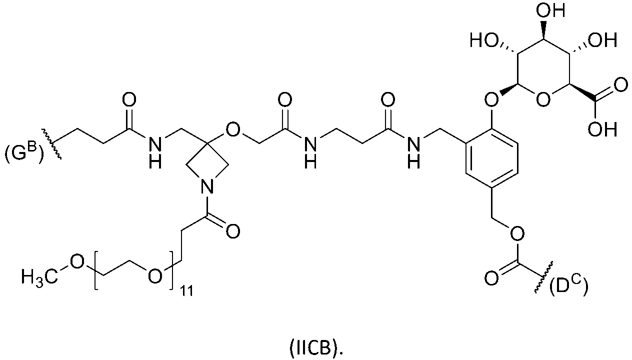

In embodiments there is provided a compound of Formula (IIC) or a salt thereof, wherein J^{B} is a group of Formula (IICB1)

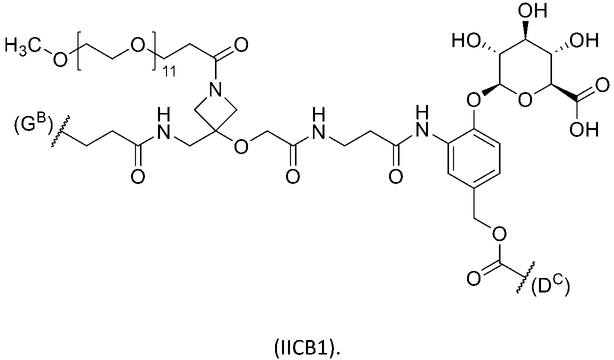

In embodiments there is provided a compound of Formula (IIC) or a salt thereof, wherein J^{B} is a group of Formula (IICB2)

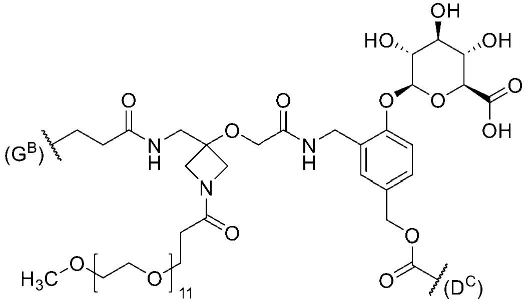

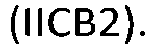

In embodiments there is provided a compound of Formula (IIC) or a salt thereof, wherein J^{B} is a group of Formula (IICB3)

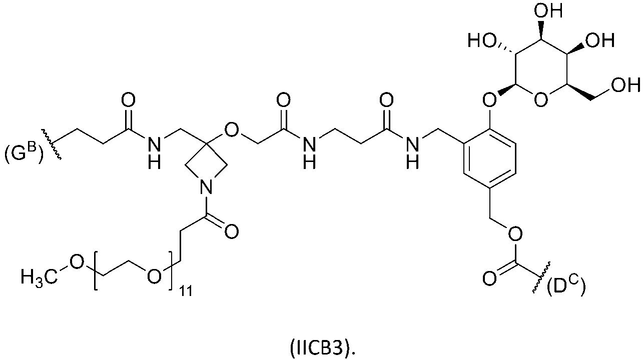

In embodiments there is provided a compound of Formula (IIC), or a salt thereof, wherein J^{B} is a group of Formula (IICB4)

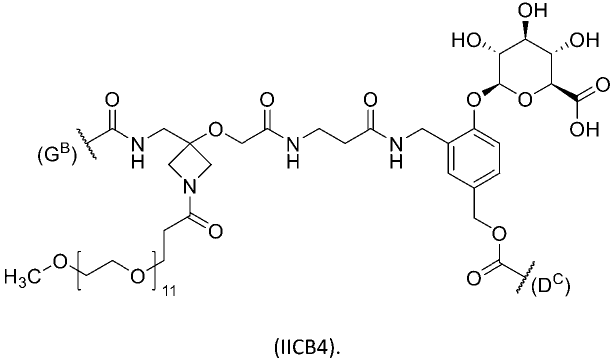

In embodiments there is provided a compound of Formula (IIM) or a salt thereof, that is; 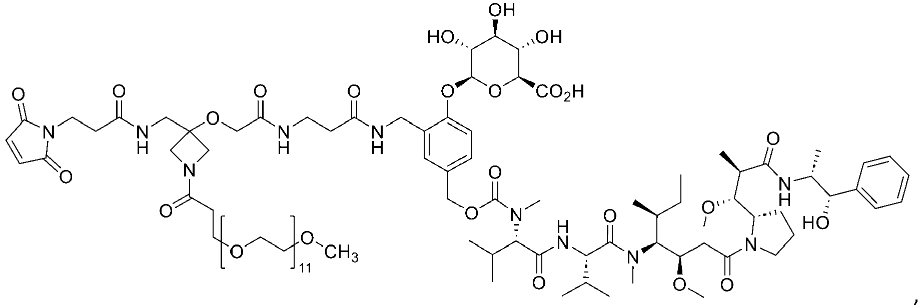 (2S,3S,4S,5R,6S)-6-(4-((5S,8S,11S,12R)-11-((S)-sec-butyl)-12-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-5,8-diisopropyl-4,10-dimethyl-3,6,9-trioxo-2,13-dioxa-4,7,10-triazatetradecyl)-2-((3-(2-((3-((3-(2,5-dioxo-2,5-dihydro-1H-pyrrol-1-yl)propanamido)methyl)-1-(2,5,8,11,14,17,20,23,26,29,32,35-dodecaoxaoctatriacontan-38-oyl)azetidin-3-yl)oxy)acetamido)propanamido)methyl)phenoxy)-3,4,5-trihydroxytetrahydro-2H-pyran-2-carboxylic acid, or a salt thereof.

In embodiments there is provided a compound of Formula (IIM) or a salt thereof, that is; 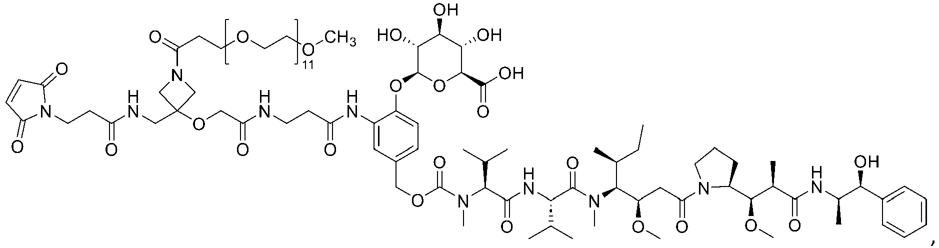 (2S,3S,4S,5R,6S)-6-(4-((5S,8S,11S,12R)-11-((S)-sec-butyl)-12-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-5,8-diisopropyl-4,10-dimethyl-3,6,9-trioxo-2,13-dioxa-4,7,10-triazatetradecyl)-2-(3-(2-((3-((3-(2,5-dioxo-2,5-dihydro-1H-pyrrol-1-yl)propanamido)methyl)-1-(2,5,8,11,14,17,20,23,26,29,32,35-dodecaoxaoctatriacontan-38-oyl)azetidin-3-yl)oxy)acetamido)propanamido)phenoxy)-3,4,5-trihydroxytetrahydro-2H-pyran-2-carboxylic acid, or a salt thereof.

In embodiments there is provided a compound of Formula (IIM) or a salt thereof, that is; 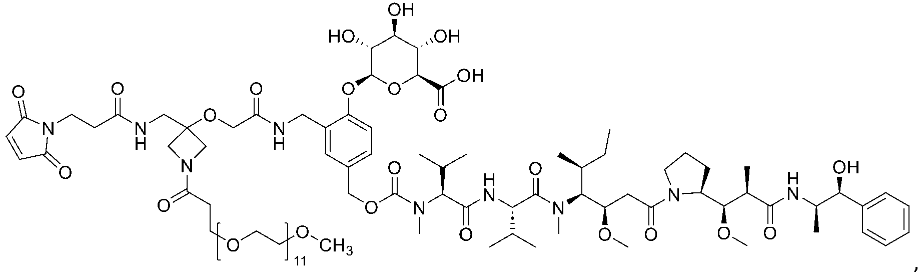 (2S,3S,4S,5R,6S)-6-(4-((5S,8S,11S,12R)-11-((S)-sec-butyl)-12-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-5,8-diisopropyl-4,10-dimethyl-3,6,9-trioxo-2,13-dioxa-4,7,10-triazatetradecyl)-2-((2-((3-((3-(2,5-dioxo-2,5-dihydro-1H-pyrrol-1-yl)propanamido)methyl)-1-(2,5,8,11,14,17,20,23,26,29,32,35-dodecaoxaoctatriacontan-38-oyl)azetidin-3-yl)oxy)acetamido)methyl)phenoxy)-3,4,5-trihydroxytetrahydro-2H-pyran-2-carboxylic acid, or a salt thereof.

In embodiments there is provided a compound of Formula (IIM) or a salt thereof, that is; 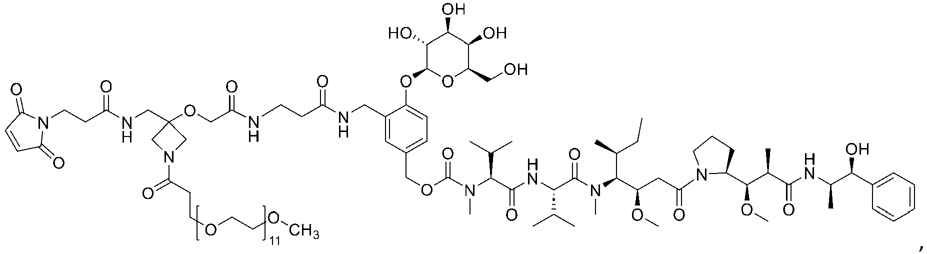 3-((3-(2-((3-((3-(2,5-dioxo-2,5-dihydro-1H-pyrrol-1-yl)propanamido)methyl)-1-(2,5,8,11,14,17,20,23,26,29,32,35-dodecaoxaoctatriacontan-38-oyl)azetidin-3-yl)oxy)acetamido)propanamido)methyl)-4-(((2S,3R,4S,SR,6R)-3,4,5-trihydroxy-6-(hydroxymethyl)tetrahydro-2H-pyran-2-yl)oxy)benzyl ((S)-1-(((S)-1-(((3R,45,55)-1-((S)-2-((1R,2R)-3-(((15,2R)-1-hydroxy-1-phenylpropan-2-yl)amino)-1-methoxy-2-methyl-3-oxopropyl)pyrrolidin-1-yl)-3-methoxy-5-methyl-1-oxoheptan-4-yl)(methyl)amino)-3-methyl-1-oxobutan-2-yl)amino)-3-methyl-1-oxobutan-2-yl)(methyl)carbamate, or a salt thereof.

In embodiments there is provided a compound of Formula (IIM) or a salt thereof, that is; 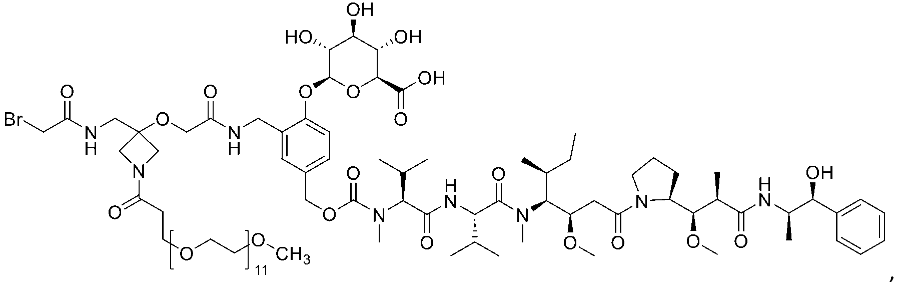 (2S,3S,4S,5R,6S)-6-(2-((3-(2-((3-((2-bromoacetamido)methyl)-1-(2,5,8,11,14,17,20,23,26,29,32,35-dodecaoxaoctatriacontan-38-oyl)azetidin-3-yl)oxy)acetamido)propanamido)methyl)-4-((5S,8S,11S,12R)-11-((S)-sec-butyl)-12-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-5,8-diisopropyl-4,10-dimethyl-3,6,9-trioxo-2,13-dioxa-4,7,10-triazatetradecyl)phenoxy)-3,4,5-trihydroxytetrahydro-2H-pyran-2-carboxylic acid, or a salt thereof.

In embodiments there is provided a compound of Formula (IIC) or a salt thereof, that is; 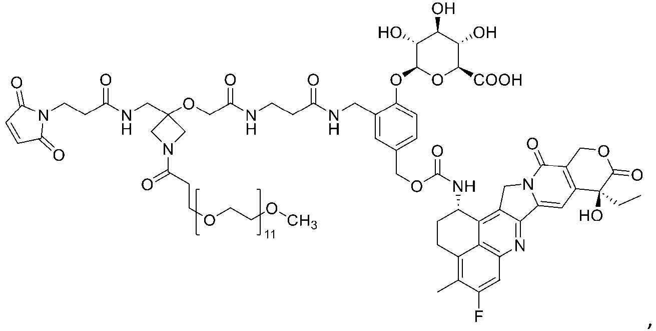 (2S,3S,4S,5R,6S)-6-(2-((3-(2-((3-((3-(2,5-dioxo-2,5-dihydro-1H-pyrrol-1-yl)propanamido)methyl)-1-(2,5,8,11,14,17,20,23,26,29,32,35-dodecaoxaoctatriacontan-38-oyl)azetidin-3-yl)oxy)acetamido)propanamido)methyl)-4-(((((1S,9S)-9-ethyl-5-fluoro-9-hydroxy-4-methyl-10,13-dioxo-2,3,9,10,13,15-hexahydro-1H,12H-benzo[de]pyrano[3',4':6,7]indolizino[1,2-b]quinolin-1-yl)carbamoyl)oxy)methyl)phenoxy)-3,4,5-trihydroxytetrahydro-2H-pyran-2-carboxylic acid, or a salt thereof.

In a further aspect there is provided a compound of Formula (III) 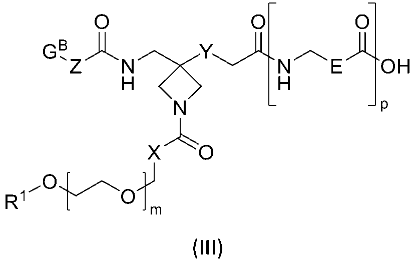 or a salt thereof, wherein G^{B} is a conjugation group for conjugation to an antibody or antigen-binding fragment thereof, and E, R¹, X, Y, Z, m and p are as defined in any embodiment of a conjugate of Formula (I).

In embodiments there is provided a compound of Formula (III), or a salt thereof, that is 3-(2-((3-((3-(2,5-dioxo-2,5-dihydro-1H-pyrrol-1-yl)propanamido)methyl)-1-(2,5,8,11,14,17,20,23,26,29,32,35-dodecaoxaoctatriacontan-38-oyl)azetidin-3-yl)oxy)acetamido)propanoic acid, or a salt thereof.

In embodiments there is provided a compound of Formula (III), or a salt thereof, that is 2-((3-((3-(2,5-dioxo-2,5-dihydro-1H-pyrrol-1-yl)propanamido)methyl)-1-(2,5,8,11,14,17,20,23,26,29,32,35-dodecaoxaoctatriacontan-38-oyl)azetidin-3-yl)oxy)acetic acid, or a salt thereof.

In a further aspect there is provided a compound of Formula (IV)

J^{IV}-D^{R} (IV)

or a salt thereof, wherein D^{R} is a drug comprising a nitrogen atom N^{R},
J^{IV} is a group of Formula (IVA) 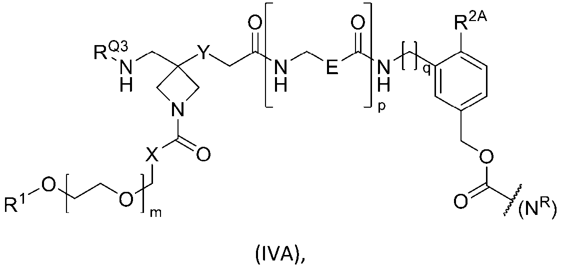
wherein E, R¹, X, Y, m, p and q are as defined in any of the embodiments of a conjugate of Formula (I) disclosed herein, R^{2A} is 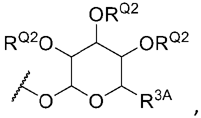 wherein R^{3A} is CO₂R^{Q1} or CH₂OR^{Q2}, R^{Q1} is H or R^{P1}, each R^{Q2} is independently is H or R^{P2}, and R^{Q3} is H or R^{P3}, wherein R^{P1} is a carboxylic acid protecting group, each R^{P2} is independently an alcohol protecting group and R^{P3} is an amine protecting group, and (N^{R}) indicates the point of attachment to the nitrogen atom N^{R}.

In a further aspect there is provided a compound of Formula (IVM)

J^{IVM}-D^{M} (IVM)

or a salt thereof, wherein J^{IVM} is a group of Formula (IVA) 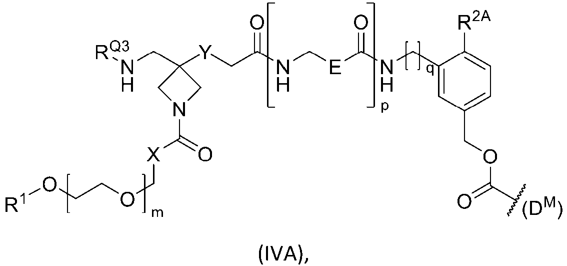
wherein D^{M}, E, R¹, X, Y, m, p and q are as defined in any of the embodiments of a conjugate of Formula (IM) disclosed herein, R^{2A} is 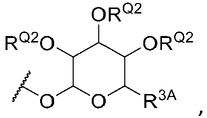 wherein R^{3A} is CO₂R^{Q1} or CH₂OR^{Q2}, R^{Q1} is H or R^{P1}, each R^{Q2} is independently H or R^{P2} and R^{Q3} is H or R^{P3}, wherein R^{P1} is a carboxylic acid protecting group, each R^{P2} is independently an alcohol protecting group and R^{P3} is an amine protecting group, and (N^{R}) indicates the point of attachment to the nitrogen atom N^{R}.

In embodiments there is provided a compound of Formula (IV) or (IVM), or a salt thereof, wherein R^{2A} is 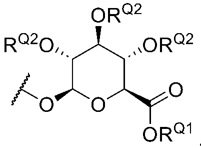 In further embodiments, R^{2A} is

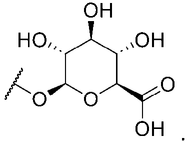

In embodiments there is provided a compound of Formula (IV) or (IVM), or a salt thereof, wherein R^{2A} is 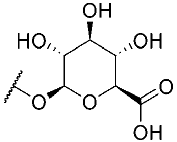 and R^{Q3} is H.

In embodiments there is provided a compound of Formula (IV) or (IVM), or a salt thereof, wherein R^{2A} is 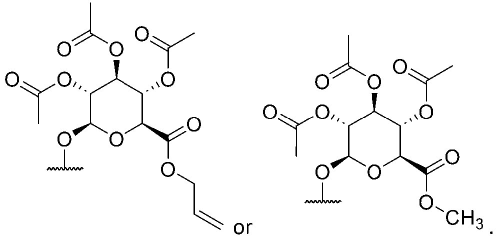 In further embodiments, R^{Q3} is C(O)OCH₂CH=CH₂.

In embodiments there is provided a compound of Formula (IVM), or a salt thereof, that is 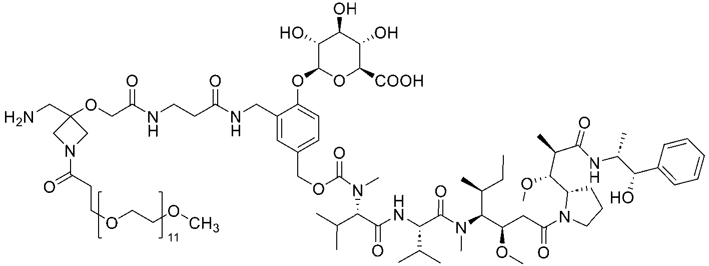 (2S,3S,4S,5R,6S)-6-(2-((3-(2-((3-(aminomethyl)-1-(2,5,8,11,14,17,20,23,26,29,32,35-dodecaoxaoctatriacontan-38-oyl)azetidin-3-yl)oxy)acetamido)propanamido)methyl)-4-((5S,8S,11S,12R)-11-((S)-sec-butyl)-12-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-5,8-diisopropyl-4,10-dimethyl-3,6,9-trioxo-2,13-dioxa-4,7,10-triazatetradecyl)phenoxy)-3,4,5-trihydroxytetrahydro-2H-pyran-2-carboxylic acid, or a salt thereof.

In embodiments there is provided a compound of Formula (IVM), or a salt thereof, that is 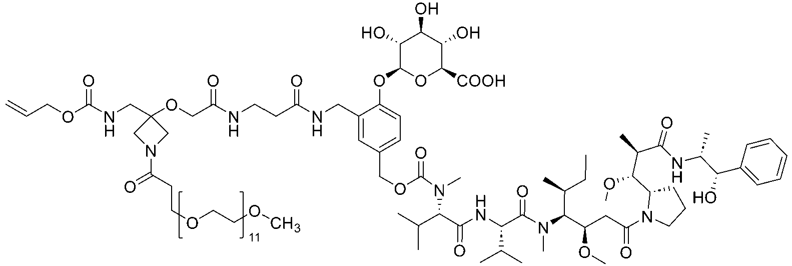 (2*S*,3*S*,4*S*,5*R*,6*S*)-6-[2-[[3-[[2-[3-[(allyloxycarbonylamino)methyl]-1-[3-[2-[2-[2-[2-[2-[2-[2-[2-[2-[2-(2-methoxyethoxy)ethoxy]ethoxy]ethoxy]ethoxy]ethoxy]ethoxy]ethoxy]ethoxy]ethoxy]ethoxy]propano yl]azetidin-3-yl]oxyacetyl]amino]propanoylamino]methyl]- 4-[[[(1*S)*-1-[[(1*S*)-1-[[(1*S*,2*R*)-4-[(2*S*)-2-[(1*R*,2*R*)-3-[[(1*R*,2*S*)-2-hydroxy-1-methyl-2-phenyl-ethyl]amino]-1-methoxy-2-methyl-3-oxopropyl]pyrrolidin-1-yl]-2-methoxy-1-[(1*S*)-1-methylpropyl]-4-oxo-butyl]-methyl-carbamoyl]-2-methyl-propyl]carbamoyl]-2-methyl-propyl]-methyl-carbamoyl]oxymethyl]phenoxy]-3,4,5-trihydroxy-tetrahydropyran-2-carboxylic acid, or a salt thereof.

In embodiments there is provided a compound of Formula (IVM), or a salt thereof, that is 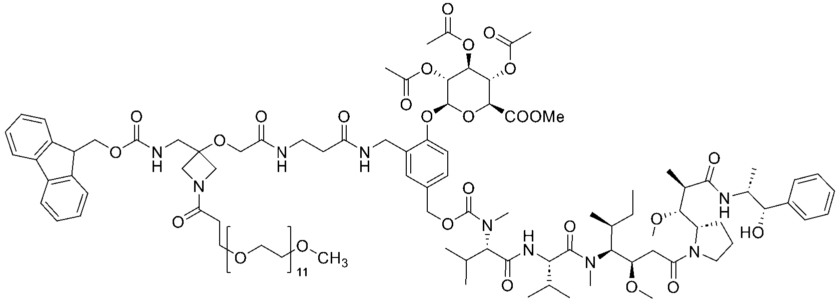 (2S,3R,4S,5S,6S)-2-(2-((3-(2-((3-(((((9H-fluoren-9-yl)methoxy)carbonyl)amino)methyl)-1-(2,5,8,11,14,17,20,23,26,29,32,35-dodecaoxaoctatriacontan-38-oyl)azetidin-3-yl)oxy)acetamido)propanamido)methyl)-4-((5S,8S,11S,12R)-11-((S)-sec-butyl)-12-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-5,8-diisopropyl-4,10-dimethyl-3,6,9-trioxo-2,13-dioxa-4,7,10-triazatetradecyl)phenoxy)-6-(methoxycarbonyl)tetrahydro-2H-pyran-3,4,5-triyl triacetate, or a salt thereof.

In embodiments there is provided a compound of Formula (IVM), or a salt thereof, that is 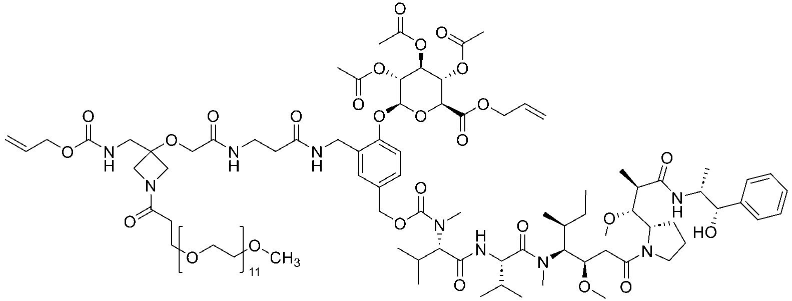 allyl (2*S*,3*S*,4*S*,5*R*,6*S*)-3,4,5-triacetoxy-6-[2-[[3-[[2-[3-[(allyloxycarbonylamino)methyl]-1-[3-[2-[2-[2-[2-[2-[2-[2-[2-[2-[2-(2-methoxyethoxy)ethoxy]ethoxy]ethoxy]ethoxy]ethoxy]ethoxy]ethoxy]ethoxy]ethoxy]ethoxy]propano yl]azetidin-3-yl]oxyacetyl]amino]propanoylamino]methyl]-4-[[[(15)-1-[[(15)-1-[[(15,2R)-4-[(25)-2-[(1*R*,2*R*)-3-[[(1*R*,2*S*)-2-hydroxy-1-methyl-2-phenyl-ethyl]amino]-1-methoxy-2-methyl-3-oxopropyl]pyrrolidin-1-yl]-2-methoxy-l-[(1*S*)-1-methylpropyl]-4-oxo-butyl]-methyl-carbamoyl]-2-methyl-propyl]carbamoyl]-2-methyl-propyl]-methylcarbamoyl]oxymethyl]phenoxy]tetrahydropyran-2-carboxylate, or a salt thereof.

In a further aspect there is provided a compound of Formula (V) 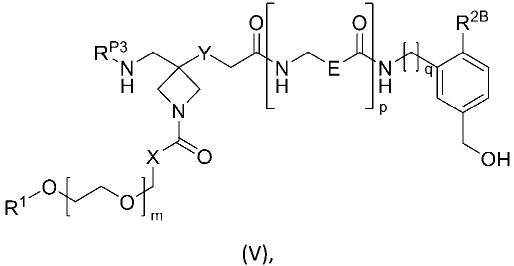 or a salt thereof, wherein E, R¹, X, Y, m, p and q are as defined in any of the embodiments of a conjugate of Formula (I) disclosed herein, R^{2B} is 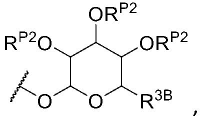 wherein R^{3B} is CO₂R^{P1} or CH₂OR^{P2}, R^{P1} is a carboxylic acid protecting group, each R^{P2} is independently an alcohol protecting group and R^{P3} is an amine protecting group.

In embodiments there is provided a compound of Formula (V), or a salt thereof, that is 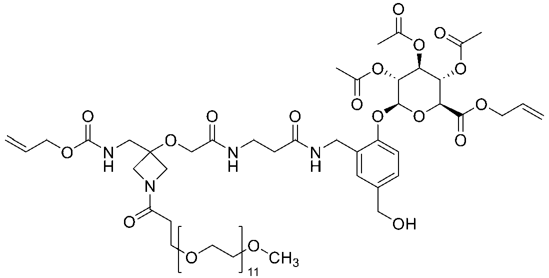 allyl (2*S*,3*S*,4*S*,5*R*,6*S*)-3,4,5-triacetoxy-6-[2-[[3-[[2-[3-[(allyloxycarbonylamino)methyl]-1-[3-[2-[2-[2-[2-[2-[2-[2-[2-[2-[2-(2-methoxyethoxy)ethoxy]ethoxy]ethoxy]ethoxy]ethoxy]ethoxy]ethoxy]ethoxy]ethoxy]ethoxy]propano yl]azetidin-3-yl]oxyacetyl]amino]propanoylamino]methyl]-4-(hydroxymethyl)phenoxy]tetrahydropyran-2-carboxylate, or a salt thereof.

In embodiments there is provided a compound of Formula (V), or a salt thereof, that is 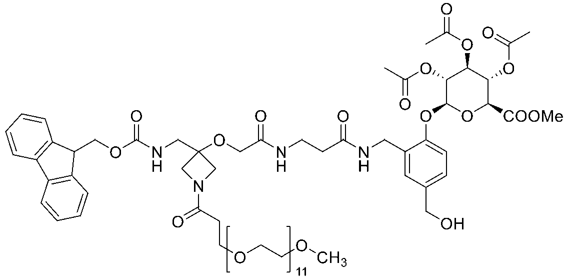 (2S,3R,4S,5S,6S)-2-(2-((3-(2-((3-(((((9H-fluoren-9-yl)methoxy)carbonyl)amino)methyl)-1-(2,5,8,11,14,17,20,23,26,29,32,35-dodecaoxaoctatriacontan-38-oyl)azetidin-3-yl)oxy)acetamido)propanamido)methyl)-4-(hydroxymethyl)phenoxy)-6-(methoxycarbonyl)tetrahydro-2H-pyran-3,4,5-triyl triacetate, or a salt thereof.

In embodiments there is provided a compound of Formula (IV), (IVM) or (V), or a salt thereof, wherein R^{P1} is selected from benzyl, allyl and C₁₋₄ alkyl. In further embodiments R^{P1} is allyl. In further embodiments R^{P1} is methyl.

In embodiments there is provided a compound of Formula (IV), (IVM) or (V), or a salt thereof, wherein each R^{P2} is independently selected from benzyl, acetyl, and CH₂CH=CH₂. In further embodiments each R^{P2} is acetyl.

In embodiments there is provided a compound of Formula (IV), (IVM) or (V), or a salt thereof, wherein R^{P3} is selected from C(O)OCH₂CH=CH₂, trifluoroacetyl, tert-butyloxycarbonyl (Boc), benzyloxycarbonyl (Cbz), benzyl, 2,4-dimethoxybenzyl and fluorenylmethoxycarbonyl (Fmoc). In further embodiments R^{P3} is C(O)OCH₂CH=CH₂. In further embodiments R^{P3} is Fmoc.

The present specification is intended to include all isotopes of atoms occurring in the present compounds and conjugates. Isotopes will be understood to include those atoms having the same atomic number but different mass numbers. For example, isotopes of hydrogen include tritium and deuterium. Isotopes of carbon include ¹³C and ¹⁴C. Isotopes of nitrogen include ¹⁵N.

The compounds disclosed herein may contain one or more chiral centers. Accordingly, if desired, such compounds can be prepared or isolated as pure stereoisomers, i.e. as individual enantiomers, diastereoisomers, or as a stereoisomerically enriched mixture. All such stereoisomer (and enriched) mixtures are included within the scope of the embodiments, unless otherwise stated. Pure stereoisomers (or enriched mixtures) may be prepared using, for example, optically active starting materials or stereoselective reagents well-known in the art. Alternatively, racemic mixtures of such compounds can be separated using, for example, chiral column chromatography, chiral resolving agents and the like.

Unless stereochemistry is explicitly indicated in a chemical structure or chemical name, the chemical structure or chemical name is intended to embrace all possible stereoisomers, diastereoisomers, conformers, rotamers and tautomers of the compound depicted. For example, a compound containing a chiral carbon atom is intended to embrace both the (R) enantiomer and the (S) enantiomer, as well as mixtures of the enantiomers, including racemic mixtures; and a compound containing two chiral carbons is intended to embrace all enantiomers and diastereoisomers including (R,R), (S,S), (R,S) and (S,R).

A suitable pharmaceutically acceptable salt of a conjugate of Formula (I), (IM) or (IC) is, for example, an acid addition salt. An acid addition salt of a conjugate of Formula (I), (IM) or (IC), may be formed by bringing the compound into contact with a suitable inorganic or organic acid under conditions known to the skilled person. An acid addition salt may for example be formed using an inorganic acid selected from hydrochloric acid, hydrobromic acid, sulphuric acid and phosphoric acid. An acid addition salt may also be formed using an organic acid selected from trifluoroacetic acid, citric acid, maleic acid, oxalic acid, acetic acid, formic acid, benzoic acid, fumaric acid, succinic acid, tartaric acid, lactic acid, pyruvic acid, methanesulfonic acid, benzenesulfonic acid and para-toluenesulfonic acid.

A suitable pharmaceutically acceptable salt of a conjugate of Formula (I), (IM) or (IC) is, for example, a base addition salt. A base addition salt of a conjugate of Formula (I), (IM) or (IC) may be formed by bringing the compound into contact with a suitable inorganic or organic base under conditions known to the skilled person. A base addition salt may for example be an alkali metal salt (such as a sodium, potassium, or lithium salt) or an alkaline earth metal salt (such as a calcium salt), which may be formed using an alkali metal or alkaline earth metal hydroxide or alkoxide (e.g., an ethoxide or methoxide). A base addition salt may also be formed using a suitably basic organic amine (e.g., a choline or meglumine salt).

A further suitable pharmaceutically acceptable salt of a conjugate of Formula (I), (IM) or (IC) is, for example, a salt formed within a patient's body after administration of a conjugate of Formula (I), (IM) or (IC) to the patient.

In a further aspect there is provided a pharmaceutical composition comprising a conjugate of Formula (I), (IM) or (IC), or a pharmaceutically acceptable salt thereof and a pharmaceutically acceptable excipient.

The term "pharmaceutical composition" refers to a preparation which is in such form as to permit the biological activity of the active ingredient, and which contains no additional components which are unacceptably toxic to a patient to which the composition would be administered. Such compositions can be sterile. A pharmaceutical composition according to the present specification will comprise a conjugate of Formula (I), (IM) or (IC), or a pharmaceutically acceptable salt thereof, or a pharmaceutically acceptable salt thereof, and a pharmaceutically acceptable excipient.

In embodiments there is provided a pharmaceutical composition comprising a conjugate of Formula (I), (IM) or (IC), or a pharmaceutically acceptable salt thereof, and a pharmaceutically acceptable excipient, carrier, buffer or stabiliser. Such materials should be non-toxic and should not interfere with the efficacy of the active ingredient. The precise nature of the carrier or other material will depend on the route of administration, which may be oral, or by injection, e.g. cutaneous, subcutaneous, or intravenous.

In embodiments there is provided a pharmaceutical composition comprising a conjugate of Formula (I), (IM) or (IC), or a pharmaceutically acceptable salt thereof, and a pharmaceutically acceptable, non-toxic, sterile carrier. In further embodiments the carrier is a physiological saline, non-toxic buffer, or preservative. Suitable formulations for use in the therapeutic methods disclosed herein are described in Remington's Pharmaceutical Sciences, 22nd ed., Ed. Lloyd V. Allen, Jr. (2012), the contents of which are incorporated by reference.

Examples of suitable excipients include one or more of water, saline, phosphate buffered saline, dextrose, glycerol and ethanol, as well as any combination thereof. In embodiments there is provided a pharmaceutical composition comprising a conjugate of Formula (I), (IM) or (IC), or a pharmaceutically acceptable salt thereof, and one or more isotonic agents. In further embodiments the one or more isotonic agents are selected from a sugar, a polyalcohol and sodium chloride.

In embodiments there is provided a pharmaceutical composition comprising a conjugate of Formula (I), (IM) or (IC), or a pharmaceutically acceptable salt thereof, contained within one or more formulations selected from a capsule, a tablet, an aqueous suspension, a solution, a nasal aerosol, and a lyophilised powder which can be reconstituted to make a suspension or solution before use.

In embodiments there is provided a pharmaceutical composition comprising a conjugate of Formula (I), (IM) or (IC), or a pharmaceutically acceptable salt thereof, a buffer, a surfactant and/or a stabiliser agent. In further embodiments the buffer is an acetate, phosphate or citrate buffer. In further embodiments the surfactant is polysorbate. In further embodiments the stabiliser agent is human albumin.

The pharmaceutical composition comprising a conjugate of Formula (I), (IM) or (IC), or a pharmaceutically acceptable salt thereof, can be administered to a patient by any appropriate systemic or local route of administration. For example, administration may be oral, buccal, sublingual, ophthalmic, intranasal, intratracheal, pulmonary, topical, transdermal, urogenital, rectal, subcutaneous, intravenous, intra-arterial, intraperitoneal, intramuscular, intracranial, intrathecal, epidural, intraventricular or intratumoural.

The pharmaceutical composition comprising a conjugate of Formula (I), (IM) or (IC), or a pharmaceutically acceptable salt thereof, can be formulated for administration by any appropriate means, for example by epidermal or transdermal patches, ointments, lotions, creams, or gels; by nebulisers, vaporisers, or inhalers; by injection or infusion; or in the form of capsules, tablets, liquid solutions or suspensions in water or non-aqueous media, drops, suppositories, enemas, sprays, or powders. The most suitable route for administration in any given case will depend on the physical and mental condition of the subject, the nature and severity of the disease, and the desired properties of the formulation.

Pharmaceutical compositions comprising a conjugate of Formula (I), (IM) or (IC), or a pharmaceutically acceptable salt thereof, for oral administration may be in tablet, capsule, powder or liquid form. A tablet may comprise a solid carrier or an adjuvant. Liquid pharmaceutical compositions generally comprise a liquid carrier such as water, petroleum, animal or vegetable oils, mineral oil or synthetic oil. Physiological saline solution, dextrose or other saccharide solution or glycols such as ethylene glycol, propylene glycol or polyethylene glycol may be included. A capsule may comprise a solid carrier such a gelatin.

For intravenous, cutaneous or subcutaneous injection, or injection at the site of affliction, the conjugate of Formula (I), (IM) or (IC), or a pharmaceutically acceptable salt thereof, will be in the form of a parenterally acceptable aqueous solution which is pyrogen-free and has suitable pH, isotonicity and stability. Those of relevant skill in the art are well able to prepare suitable solutions using, for example, isotonic vehicles such as Sodium Chloride Injection, Ringer's Injection, Lactated Ringer's Injection. Preservatives, stabilisers, buffers, antioxidants and/or other additives may be included, as required.

In one aspect there is provided a conjugate of Formula (I), (IM) or (IC), or a pharmaceutically acceptable salt thereof, for use in therapy.

In one aspect there is provided a conjugate of Formula (I), (IM), or (IC) or a pharmaceutically acceptable salt thereof, for use in the treatment of cancer.

Where "cancer" is mentioned, this includes both non-metastatic cancer and also metastatic cancer, such that treating cancer involves treatment of both primary tumours and also tumour metastases.

The term "therapy" is intended to have its normal meaning of dealing with a disease in order to entirely or partially relieve one, some or all of its symptoms, or to correct or compensate for the underlying pathology. The term "therapy" also includes "prophylaxis" unless there are specific indications to the contrary. The terms "therapeutic" and "therapeutically" should be interpreted in a corresponding manner.

The term "prophylaxis" is intended to have its normal meaning and includes primary prophylaxis to prevent the development of the disease and secondary prophylaxis whereby the disease has already developed and the patient is temporarily or permanently protected against exacerbation or worsening of the disease or the development of new symptoms associated with the disease.

The term "treatment" is used synonymously with "therapy". Similarly the term "treat" can be regarded as "applying therapy" where "therapy" is as defined herein.

In embodiments there is provided a conjugate of Formula (I), (IM) or (IC), or a pharmaceutically acceptable salt thereof, for use in the treatment of HER2 positive cancer.

In one aspect there is provided the use of a conjugate of Formula (I), (IM) or (IC), or a pharmaceutically acceptable salt thereof, as described herein, in the manufacture of a medicament, such as a medicament for the treatment of cancer.

In one aspect there is provided a method of treating cancer in a patient comprising administering to the patient an effective amount of a conjugate of Formula (I), (IM) or (IC), or a pharmaceutically acceptable salt thereof.

Terms such as "treating" or "treatment" refer to both (1) therapeutic measures that cure, slow down, lessen symptoms of, and/or halt progression of a diagnosed pathologic condition or disorder and (2) prophylactic or preventative measures that prevent and/or slow the development of a targeted pathologic condition or disorder. Thus, those in need of treatment include those already with the disorder; those prone to have the disorder; and those in whom the disorder is to be prevented. In certain aspects, a patient is successfully "treated" for cancer according to the methods of the present disclosure if the patient shows, e.g., total, partial, or transient remission of a certain type of cancer.

The term "effective amount" means an amount of an active ingredient which is sufficient enough to significantly and positively modify the symptoms and/or conditions to be treated (e.g., provide a positive clinical response). The effective amount of an active ingredient for use in a pharmaceutical composition will vary with the particular condition being treated, the severity of the condition, the duration of the treatment, the nature of concurrent therapy, the particular active ingredient(s) being employed, the particular pharmaceutically-acceptable excipient(s)/carrier(s) utilized, and like factors within the knowledge and expertise of the attending physician.

The term "patient" refers to any animal (e.g., a mammal), including, but not limited to humans, non-human primates, rodents, and the like, which is to be the recipient of a particular treatment. In embodiments the term "patient" refers to a human subject.

In embodiments there is provided a method of treating cancer in a patient comprising administering to the patient an effective amount of a conjugate of Formula (I), (IM) or (IC), or a pharmaceutically acceptable salt thereof, wherein the cancer is a HER2 positive cancer.

In embodiments there is provided a conjugate of Formula (I), (IM) or (IC), or a pharmaceutically acceptable salt thereof, and an additional anti-tumour substance for the conjoint treatment of cancer.

In embodiments there is provided a combination for use in the treatment of cancer comprising a conjugate of the Formula (I), (IM) or (IC), or a pharmaceutically acceptable salt thereof and an additional anti-tumour agent.

In embodiments there is provided a conjugate of the Formula (I), (IM) or (IC), or a pharmaceutically acceptable salt thereof, in combination with an additional anti-tumour agent.

Herein, where the term "conjoint treatment" is used in reference to a combination treatment, it is to be understood that this may refer to simultaneous, separate or sequential administration. In one aspect, "conjoint treatment" refers to simultaneous administration. In another aspect, "conjoint treatment" refers to separate administration. In a further aspect, "conjoint treatment" refers to sequential administration.

In embodiments there is provided a method of treating cancer in a patient comprising administering to the patient an effective amount of a conjugate of Formula (I), (IM) or (IC), or a pharmaceutically acceptable salt thereof, and simultaneously, separately or sequentially administering at least one additional anti-tumour substance to said patient, where the amounts of the conjugate of Formula (I), (IM) or (IC) or a pharmaceutically acceptable salt thereof, and the additional anti-tumour substance are jointly effective in producing an anti-cancer effect.

### Conjugation

Examples of G^{A} and G^{B} include, but are not limited to, the following, wherein X¹ is CH or N, h is 0 or 1,
R^{K} is H or CH₃, Hal is Cl, Br or I, R^{L} is C₁₋₆ alkyl, and 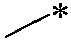 indicates the point of attachment to the antibody, or antigen-binding fragment thereof.

| **G^{A}** | **G^{B}** |
|---|---|
| 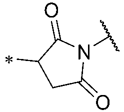 | 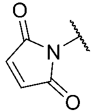 |
| 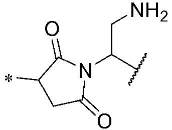 | 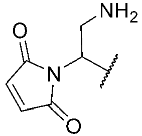 |
| 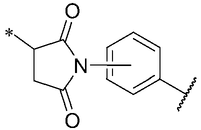 | 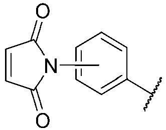 |
| 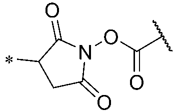 | 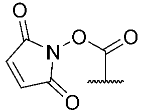 |
| 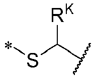 | 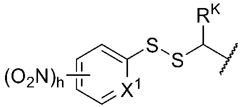 |

| | |
|---|---|
| 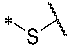 | 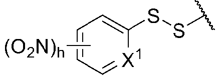 |
| 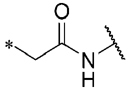 | 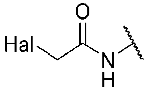 |
| 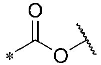 | 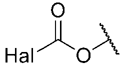 |
| 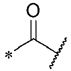 | 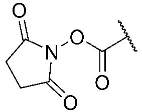 |
| 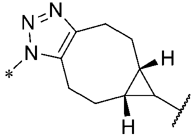 | 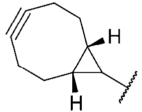 |
| 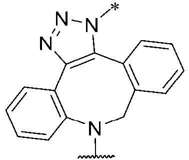 | 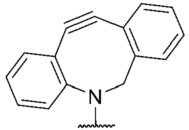 |
| 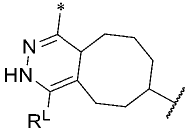 | 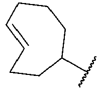 |
| 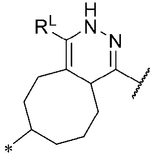 | 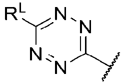 |
| 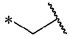 | 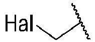 |
| 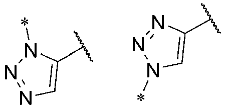 | 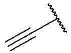 |
| 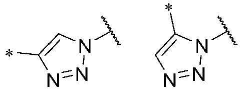 | 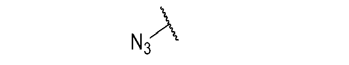 |

| | |
|---|---|
| 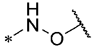 | 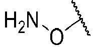 |
| 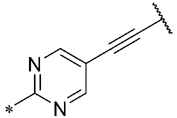 | 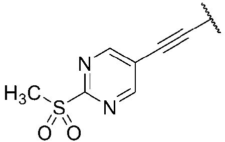 |

### Antibody or antigen-binding fragment thereof

As used herein, the term "antibody" refers to an immunoglobulin molecule that specifically binds to, or is immunologically reactive with, a particular antigen.

In embodiments the antibody is isolated or recombinant. "Isolated", when used herein refers to a polypeptide, e.g., an antibody, that has been identified and separated and/or recovered from a cell or cell culture from which it was expressed. Ordinarily, an isolated antibody will be prepared by at least one purification step. Thus, an "isolated antibody" refers to an antibody which is substantially free of other antibodies having different antigenic specificities.

In embodiments the antibody comprises at least two "light chains" (LC) and two "heavy chains" (HC). The light chains and heavy chains of such antibodies are polypeptides consisting of several domains. Each heavy chain comprises a heavy chain variable region (abbreviated herein as "VH") and a heavy chain constant region (abbreviated herein as "CH"). The heavy chain constant region comprises the heavy chain constant domains CH1, CH2 and CH3 (antibody classes IgA, IgD, and IgG) and optionally the heavy chain constant domain CH4 (antibody classes IgE and IgM). Each light chain comprises a light chain variable domain (abbreviated herein as "VL") and a light chain constant domain (abbreviated herein as "CL").

In embodiments the antibody is a full-length antibody. An "intact" or "full-length" antibody, as used herein, refers to an antibody having two heavy (H) chain polypeptides and two light (L) chain polypeptides interconnected by disulfide bonds.

A "variable region" of an antibody refers to the variable region of the antibody light chain or the variable region of the antibody heavy chain, either alone or in combination. The variable regions VH and VL can be further subdivided into regions of hypervariability, termed complementarity determining regions (CDRs) (also known as hypervariable regions), interspersed with regions that are more conserved, termed framework regions (FRs). In embodiments each VH and VL is composed of three CDRs and four FRs, arranged from amino-terminus to carboxy-terminus in the following order: FR1, CDR1, FR2, CDR2, FR3, CDR3, FR4. The VH or VL chain of the antibody can further include all or part of a heavy or light chain constant region.

Binding between an antibody and its target antigen or epitope is mediated by the CDRs. The term "epitope" refers to a target protein region (e.g. polypeptide) capable of binding to (e.g. being bound by) an antibody or antigen-binding fragment of the disclosure. The CDRs are the main determinants of antigen specificity. There are at least two techniques for determining CDRs: (1) an approach based on cross-species sequence variability (i.e., Kabat et al. Sequences of Proteins of Immunological Interest, (5th ed., 1991, National Institutes of Health, Bethesda Md.)); and (2) an approach based on crystallographic studies of antigen-antibody complexes (Al-lazikani et al. (1997) J. Molec. Biol. 273:927-948)). In addition, combinations of these two approaches are sometimes used in the art to determine CDRs.

The "constant domains" (or "constant regions") of the heavy chain and of the light chain are not involved directly in binding of an antibody to a target, but exhibit various effector functions. The constant regions of the antibodies can mediate the binding of the immunoglobulin to host tissues or factors, including various cells of the immune system (e.g., effector cells) and the first component (Clq) of the classical complement system.

There are five major classes of heavy chain constant region, classified as IgA, IgG, IgD, IgE and IgM, each with characteristic effector functions designated by isotype. Ig molecules interact with multiple classes of cellular receptors. For example, IgG molecules interact with three classes of Fcy receptors (FcyR) specific for the IgG class of antibody, namely FcyRI, FcγRII, and FcyRIll. Binding of antibody to Fc receptors on cell surfaces triggers a number of important and diverse biological responses including engulfment and destruction of antibody-coated particles, clearance of immune complexes, lysis of antibody-coated target cells by killer cells (called antibody-dependent cell-mediated cytotoxicity, or ADCC), release of inflammatory mediators, placental transfer and control of immunoglobulin production. The important sequences for the binding of IgG to the FcyR receptors have been reported to be located in the CH2 and CH3 domains.

In embodiments the antibody or antigen-binding fragment thereof is an IgG isotype. The antibody or antigen-binding fragment thereof can be any IgG subclass, for example IgG1, IgG2, IgG3, or IgG4 isotype. In embodiments the antibody or antigen-binding fragment thereof is based on an IgG1 isotype.

The terms "Fc region", "Fc part" and "Fc" are used interchangeably herein and refer to the portion of a native immunoglobulin that is formed by two Fc chains. Each "Fc chain" comprises a constant domain CH2 and a constant domain CH3. Each Fc chain may also comprise a hinge region. A native Fc region is homodimeric. In embodiments the Fc region may be heterodimeric because it may contain modifications to enforce Fc heterodimerisation. The Fc region contains the carbohydrate moiety and binding sites for complement and Fc receptors (including the FcRn receptor), and has no antigen binding activity. Fc can refer to this region in isolation, or this region in the context of an antibody, antibody fragment, or Fc fusion protein. Polymorphisms have been found in a number of Fc domain sites, including but not limited to EU positions 270, 272, 312, 315, 356, and 358, resulting in minor variations between the sequences described in the instant application and sequences known in the art. As a result, every naturally occurring IgG Fc region is referred to as a "wild type IgG Fc domain" or "WT IgG Fc domain" (i.e., any allele). Human IgG1, IgG2, IgG3, and IgG4 heavy chain sequences can be obtained in a variety of sequence databases, including the UniProt database (www.uniprot.org) under accession numbers P01857 (IGHG1_HUMAN), P01859 (IGHG2_HUMAN), P01860 (IGHG3_HUMAN), and P01861 (IGHG4_HUMAN) respectively.

In embodiments the antibody of the disclosure is a monoclonal antibody. A "monoclonal antibody" (mAb) refers to a homogeneous antibody population involved in the highly specific recognition and binding of a single antigenic determinant, or epitope. This is in contrast to polyclonal antibodies that typically include different antibodies directed against different antigenic determinants. The term "monoclonal antibody" can encompass both full-length monoclonal antibodies as well as antibody fragments (such as Fab, Fab', F(ab')2, Fv), single chain (scFv) mutants, fusion proteins comprising an antibody portion, and any other modified immunoglobulin molecule comprising an antigen recognition site. Furthermore, "monoclonal antibody" refers to such antibodies made in any number of ways including, but not limited to, hybridoma, phage selection, recombinant expression, and transgenic animals. In embodiments the antibody of the disclosure is an isolated monoclonal antibody. In further embodiments the antibody is a fully human monoclonal antibody.

In embodiments the antibody of the disclosure is a full-length antibody described above. Alternatively, the antibody can be an antigen-binding fragment. The term "antigen-binding fragment" as used herein incudes any naturally-occurring or artificially-constructed configuration of an antigen-binding polypeptide comprising one, two or three light chain CDRs, and/or one, two or three heavy chain CDRs, wherein the polypeptide is capable of binding to the antigen.

In embodiments the antigen-binding fragment of the disclosure is a Fab fragment. The antibody according to the disclosure can also be a Fab', an Fv, an scFv, an Fd, a V NAR domain, an IgNAR, an intrabody, an IgG CH2, a minibody, a single-domain antibody, an Fcab, an scFv-Fc, F(ab')2, a di-scFv, a bi-specific T-cell engager (BITE), a F(ab')3, a tetrabody, a triabody, a diabody, a DVD-Ig, an (scFv)2, a mAb2 or a DARPin.

The terms "Fab fragment" and "Fab" are used interchangeably herein and contain a single light chain (*e.g.* a constant domain CL and a VL) and a single heavy chain (*e.g.* a constant domain CH1 and a VH). The heavy chain of a Fab fragment is not capable of forming a disulfide bond with another heavy chain.

A "Fab' fragment" contains a single light chain and a single heavy chain but in addition to the CH1 and the VH, a "Fab' fragment" contains the region of the heavy chain between the CH1 and CH2 domains that is required for the formation of an inter-chain disulfide bond. Thus, two "Fab' fragments" can associate via the formation of a disulfide bond to form a F(ab')2 molecule.

A "F(ab')2 fragment" contains two light chains and two heavy chains. Each chain includes a portion of the constant region necessary for the formation of an inter-chain disulfide bond between two heavy chains.

An "Fv fragment" contains only the variable regions of the heavy and light chain. It contains no constant regions.

A "single-domain antibody" is an antibody fragment containing a single antibody domain unit (*e*.*g*., VH or VL).

A "single-chain Fv" ("scFv") is antibody fragment containing the VH and VL domain of an antibody, linked together to form a single chain. A polypeptide linker is commonly used to connect the VH and VL domains of the scFv.

A "tandem scFv", also known as a TandAb, is a single-chain Fv molecule formed by covalent bonding of two scFvs in a tandem orientation with a flexible peptide linker.

A "bi-specific T cell engager" (BiTE) is a fusion protein consisting of two single-chain variable fragments (scFvs) on a single peptide chain. One of the scFvs binds to T cells via the CD3 receptor, and the other to a tumour cell antigen.

A "diabody" is a small bivalent and bispecific antibody fragment comprising a heavy chain variable domain (VH) connected to a light chain variable domain (VL) on the same polypeptide chain (VH-VL) connected by a peptide linker that is too short to allow pairing between the two domains on the same chain (Kipriyanov, Int. J. Cancer 77 (1998), 763-772). This forces pairing with the complementary domains of another chain and promotes the assembly of a dimeric molecule with two functional antigen binding sites.

A "DARPin" is a bispecific ankyrin repeat molecule. DARPins are derived from natural ankyrin proteins, which can be found in the human genome and are one of the most abundant types of binding proteins. A DARPin library module is defined by natural ankyrin repeat protein sequences, using 229 ankyrin repeats for the initial design and another 2200 for subsequent refinement. The modules serve as building blocks for the DARPin libraries. The library modules resemble human genome sequences. A DARPin is composed of 4 to 6 modules. Because each module is approx. 3.5 kDa, the size of an average DARPin is 16-21 kDa. Selection of binders is done by ribosome display, which is completely cell-free and is described in He M. and Taussig MJ., Biochem Soc Trans. 2007, Nov;35(Pt 5):962-5.

In embodiments the antibody or antigen-binding fragment thereof can be further modified to contain additional chemical moieties not normally part of the protein. Those derivatised moieties can improve the solubility, the biological half-life or absorption of the protein. The moieties can also reduce or eliminate any desirable side effects of the proteins and the like. An overview for those moieties can be found in Remington's Pharmaceutical Sciences, 22nd ed., Ed. Lloyd V. Allen, Jr. (2012).

### Drug (D^{R})

As used herein, D^{R} is independently a drug comprising a nitrogen atom N^{R}. For a conjugate of Formula (I) or a pharmaceutically acceptable salt thereof, nitrogen atom N^{R} is covalently attached to J^{A}. For a compound of Formula (II) or a salt thereof, nitrogen atom N^{R} is covalently attached to J^{B}.

A conjugate of Formula (I) or a pharmaceutically acceptable salt thereof, may undergo cleavage to release the drug in its free drug form. D^{R} may be represented as N(R^{m})(Rⁿ), wherein N(R^{m})(Rⁿ) is collectively the drug and the nitrogen atom shown is N^{R}. In this representation, the free drug form is HN(R^{m})(Rⁿ).

For a conjugate of Formula (I) or a pharmaceutically acceptable salt thereof, or a compound of Formula (II) or a salt thereof, the nitrogen atom N^{R} may be the nitrogen atom of a secondary of tertiary carbamate. In other words, the nitrogen atom N^{R} may, in the free drug form, be the nitrogen atom of a primary or secondary amine.

In embodiments there is provided a conjugate of Formula (I) or a pharmaceutically acceptable salt thereof, or a compound of Formula (II) or a salt thereof, wherein the nitrogen atom N^{R} in the nitrogen atom of an aliphatic secondary of tertiary carbamate. In other words, the nitrogen atom N^{R}, in the free drug form, is the nitrogen atom of an aliphatic primary or secondary amine.

In embodiments the drug is a therapeutic agent. In further embodiments the drug is a cytotoxic drug. In further embodiments the drug is a DNA damage response inhibitor, antineoplastic agent and tubulin disrupting agent. In further embodiments the drug is a microtubule inhibitor (MTI).

In embodiments the drug is a diagnostic agent or an imaging agent.

In embodiments D^{R} is

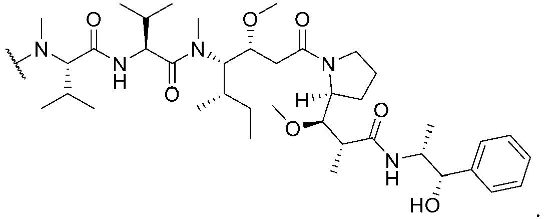

In embodiments D^{R} is

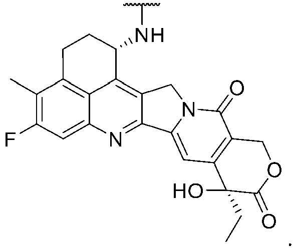

In embodiments the free drug form of the drug is MMAE ((S)-N-((3R,4S,5S)-1-((S)-2-((1R,2R)-3-(((15,2R)-1-hydroxy-1-phenylpropan-2-yl)amino)-1-methoxy-2-methyl-3-oxopropyl)pyrrolidin-1-yl)-3-methoxy-5-methyl-1-oxoheptan-4-yl)-N,3-dimethyl-2-((S)-3-methyl-2-(methylamino)butanamido)butanamide).

In embodiments the free drug form of the drug is exatecan ((15,95)-1-amino-9-ethyl-5-fluoro-9-hydroxy-4-methyl-1,2,3,9,12,15-hexahydro-10H,13H-benzo[de]pyrano[3',4':6,7]indolizino[1,2-b]quinoline-10,13-dione).

The released drug may, following release from the conjugate in the biological system, undergo a chemical modification, such as an enzyme-mediated chemical reaction and/or metabolism. As such, in embodiments the drug may be a pro-drug.

### Examples

The specification will now be illustrated by the following non-limiting Examples.

### General Information

Reagents and solvents (all anhydrous HPLC-grade) were obtained from commercial suppliers and used without any further purification unless otherwise stated. All reagents were weighed and handled in air unless otherwise stated. Brine refers to a saturated solution of NaCl. Concentration under reduced pressure refers to the use of a rotary evaporator. Pegylated reagents were obtained from PUREPEG. Mc-vc-PAB-MMAE (maleimidocaproyl-valine-citrulline-p-aminobenzoyloxycarbonyl-MMAE, CAS:646502-53-6) was obtained from Med Chem Express and will be referred to as **LP1.** In general Examples and Intermediate compounds are named using CHEMDRAW PROFESSIONAL version 22.2.0.3300 from PERKINELMER.

### List of abbreviations

ADC: Antibody Drug Conjugate; All: Allyl; Alloc: Allyloxycarbonyl; TFA: trifluoroacetic acid; FA: Formic acid; FC: flash chromatography; FMOC: fluorenylmethyloxycarbonyl; DCM: dichloromethane; DBA: Dibenzylideneacetone; DIPEA: Di isopropyl ethylamine; DMF: N,N-dimethylformamide; DMSO: dimethylsulfoxide; dppf: 1,1'-Bis(diphenylphosphino)ferrocene; ESI: Electrospray ionisation; HFIP: 1,1,1,3,3,3-Hexafluoro-2-propanol; HATU: 1-[Bis(dimethylamino)methylene]-1H-1,2,3-triazolo[4,5-b]pyridinium 3-oxid hexafluorophosphate; HOPO: 1-Hydroxy-2-pyridone; mAb: Monoclonal antibody; MS Molecular sieves; RT: Retention time; Tf: triflate; THF: Tetra hydro furan; UHPLC: Ultrahigh performance liquid chromatography; V: Volumes.

### Flash Column chromatography

### Method A

Flash chromatography was performed using a BIOTAGE ISOLERA and fractions checked for purity using thin-layer chromatography (TLC). TLC was performed using MERCK KIESELGEL 60 F254 silica gel, with fluorescent indicator on aluminium plates. Visualisation of TLC was achieved with UV light. chromatography solvents were bought and used without further purification from VWR U.K. This method was applied to **Intermediates 5-8** and **22-26,** unless stated differently.

### Method B

Chromatographic purification of products was accomplished with a CHEETAH MP200 system with integrated UV detection, using column chromatography on (A) silica gels (40-60 µm), eluted with petroleum ether and ethyl acetate or dichloromethane and methanol, (B) C18 spherical (20-35 µm), eluted with water and acetonitrile with formic acid (0.1%) or trifluoracetic acid (0.05%) or ammonium formate (10 mmol) modifier. This method was applied to all other not stated in FC method A, unless stated differently.

### LC/MS conditions

### Method A

Positive mode electrospray mass spectrometry was performed using a WATERS ACQUITY H-CLASS SQD2. Mobile phases used were solvent A (water with 0.1% formic acid) and solvent B (acetonitrile with 0.1% formic acid). Initial composition 5% B held over 25 seconds, then increased from 5% B to 100% B over a 1 minute 35 seconds' period. The composition was held for 50 seconds at 100% B, then returned to 5% B in 5 seconds and held there for 5 seconds. The total duration of the gradient run was 3.0 minutes. Flow rate was 0.8 mL/minute. Detection was at 254 nm. Columns: WATERS ACQUITY UPLC BEH SHIELD RP18 1.7µm 2.1 x 50 mm at 50 °C fitted with WATERS ACQUITY UPLC BEH SHIELD RP18 VANGUARD Pre-column, 130A, 1.7µm, 2.1 mm x 5 mm. This method was applied to Intermediates 20, 21, 24-26**,** and **37,** unless stated differently.

### Method B

Samples were analysed using a QEXACTIVE ORBITRAP (THERMO) coupled to a DIONEX ULTIMATE 3000 UPLC (THERMO) equipped with an ACQUITY UPLC CSH C18 RP column (2.1 mm x 50 mm, 1.7 µm particle (Waters)). Autosampler and column temperatures were set to 50 °C and 10 °C respectively. Five µL of 200uM sample dissolved in 1:1 v/v acetonitrile/water were injected on column and separated over 9 minutes (reduced samples) or 10 minutes (intact samples) using a gradient of 5-100% mobile phase B (0.1% FA in acetonitrile) in mobile phase A (0.1% TFA in water) at 0.5 mL/min. Settings for heated electrospray source were as follows: sheath gas flow rate 50, auxiliary gas flow rate 15 and spray voltage 3.5 kV. RF level was set to 50. Capillary temperature and auxiliary gas heater temperature were set to 350°C and 400°C respectively. UV trace was recorded at 223 nm and 330 nm. Full scan mass spectra were acquired in positive mode with resolution of 70000, AGC target set to 3E6, maximum injection time set to 200 ms and mass range of 150 - 2000 m/z. Fragmentation spectra were obtained using all ion fragmentation mode for 133-2000 m/z with stepped normalized collision energy of 15, 28 and 45. The acquired data was analysed with THERMO XCALIBUR QUAL BROWSER (version 3.1.66.10). This method was used for **Intermediates 5-8** and **22-23** and **LP2-LP9,** unless stated differently.

### Method C

Samples were analysed using a SHIMADZU LCMS-2020 with electrospray ionization in positive ion detection mode with 20ADXR pump, SIL-20ACXR autosampler, CTO-20AC column oven, M20A PDA Detector and LCMS 2020 MS detector. LC was run in two set ups: 1) Halo C18 column (2.0 µm 3.0x30 mm) in combination with a gradient (5-100% B in 1.2 minutes) of water and FA (0.1%) (A) and CH₃CN and FA (0.1%) (B)at a flow rate of 1.5 mL/min; 2) POROSHELL HPH C18 column (2.7 µm 3.0x50 mm) in combination with a gradient (5-95% B in 2 minutes) of aqueous 46 mM ammonium carbonate/ammonia buffer at pH 10 (A) and MeCN (B) at a flow rate of 1.2 mL/min ; 3) Halo C18 column (2.0 µm 3.0x30 mm) in combination with a gradient (5-95% B in 2 minutes) of water and TFA (0.05%) (A) and CH₃CN and TFA (0.05%) at a flow rate of 1.5 mL/min (B).The Column Oven (CTO-20AC) temperature was 40.0°C.The injection volume was 1 µl. PDA (SPD-M20A) detection was in the range 190-400 nm. The MS detector, which was configured with electrospray ionization as ionizable source; Acquisition mode: Scan; Nebulizing Gas Flow:1.5 L/min; Drying Gas Flow:15 L/min; Detector Voltage: Tuning Voltage ± 0.2 kv; DL Temperature: 250 °C; Heat Block Temperature: 250 °C; Scan Range: 90.00 - 900.00 m/z. This method was applied to all other intermediates not stated in LCMS method A or B, unless stated differently.

### HPLC conditions

### Method A

Reverse-phase ultra-fast high-performance liquid chromatography (UFLC) was carried out on a SHIMADZU PROMINENCE machine using a PHENOMENEX GEMINI NX 5µ C18 column (at 50 °C) dimensions: 150 x 21.2 mm. Eluents used were solvent A (H₂O with 0.1% formic acid) and solvent B (CH₃CN with 0.1% formic acid) or solvent A (H₂O with 0.1% trifluoroacetic acid acid) and solvent B (CH₃CN with 0.1% trifluoroacetic acid), used for intermediates **35, 36** and **LP2-LP5,** unless stated differently.

### Method B

Preparative HPLC was performed with a WATERS MASSLYNX system with integrated MS detection and equipped with Prep C18 OBD 5µm 30 x 150 mm columns from XBRIDGE or XSELECT CSH. Alternatively, GILSON GX-281 with integrated UV detection was used, equipped with either XBRIDGE or SUNFIRE C18 10µm, 19x150 ID or 19x250mm. As eluent (acidic) gradients of water/MeCN/FA acid (95/5/0.1) or water/0.05% TFA (A) and MeCN/ MeOH (B) or 0.05% ammonia in water /10 mmol NH₄HCO₃ (A) and MeCN/ MeOH (B) were applied. This method was use for intermediate **20.**

### Synthesis of Intermediate 8

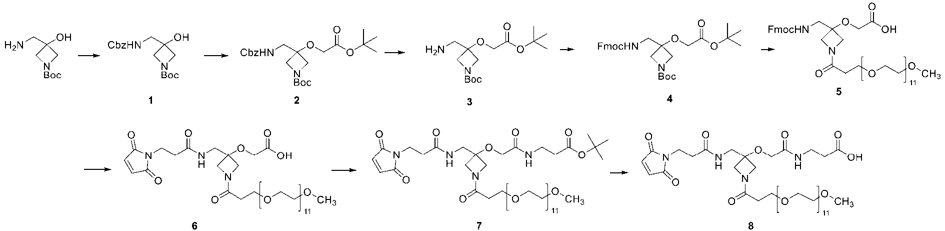

### Step 1

tert-butyl 3-(aminomethyl)-3-hydroxyazetidine-1-carboxylate (10 g, 49.44 mmol) was added to N-ethyl-N-isopropylpropan-2-amine (19.17 g, 148.33 mmol) and N-(benzyloxycarbonyloxy)succinimide (14.79 g, 59.33 mmol) in THF (150 mL) under nitrogen. The resulting mixture was stirred at 25 °C for 3 hours. The solvent was removed under reduced pressure. The crude product was purified by flash silica chromatography, elution gradient 0 to 10% MeOH in DCM. Pure fractions were evaporated to dryness to afford tert-butyl 3-((((benzyloxy)carbonyl)amino)methyl)-3-hydroxyazetidine-1-carboxylate (**Intermediate 1,** 13.00 g, 78 %) as a yellow oil. m/z (ES+), [M+H]+ = 337; TFA, HPLC tR = 0.903 min.

### Step 2

**Intermediate 1** (13 g, 38.65 mmol) was added to tert-butyl 2-bromoacetate (9.05 g, 46.38 mmol) and sodium hydride (1.855 g, 77.29 mmol) in THF (150 mL) under nitrogen at 0 °C. The resulting mixture was stirred at 25 °C for 2 hours. The reaction mixture was quenched with water (50 mL), extracted with EtOAc (3 x 150 mL), the top layer was dried over Na₂SO₄, filtered and evaporated to afford yellow oil. The crude product was purified by flash silica chromatography, elution gradient 0 to 50% EtOAc in petroleum ether. Pure fractions were evaporated to dryness to afford tert-butyl 3-((((benzyloxy)carbonyl)amino)methyl)-3-(2-(tert-butoxy)-2-oxoethoxy)azetidine-1-carboxylate **(Intermediate 2,** 9.00 g, 51.7 %) as a colourless oil. m/z (ES+), [M+H]+ = 451; TFA, HPLC tR = 1.125 min.

### Step 3

**Intermediate 2** (**2,** 9 g, 19.98 mmol) was added to ROYER^{™} Palladium Catalyst Powder (CAS 7440-05-3, 6.07 g, 19.98 mmol) in THF (120 mL) under hydrogen. The resulting mixture was stirred at 25 °C for 6 hours. The reaction mixture was filtered through silica and washed with THF (3 x 50 mL). The solvent was removed under reduced pressure to afford tert-butyl 3-(aminomethyl)-3-(2-(tert-butoxy)-2-oxoethoxy)azetidine-1-carboxylate (**Intermediate 3,** 6.00 g, 85 %) as a yellow oil. m/z (ES+), [M+H]+ = 317; TFA, HPLC tR = 0.882 min.

### Step 4

**Intermediate 3** (5 g, 15.80 mmol) was added to 9-fluorenylmethyl chloroformate (6.13 g, 23.70 mmol) and N,N-diisopropylethylamine (6.13 g, 47.41 mmol) in THF (50 mL) under nitrogen. The resulting mixture was stirred at 25 °C for 3 hours. The solvent was removed under reduced pressure. The crude product was purified by flash silica chromatography, elution gradient 0 to 50% EtOAc in petroleum ether. Pure fractions were evaporated to dryness to afford tert-butyl 3-(((((9H-fluoren-9-yl)methoxy)carbonyl)amino)methyl)-3-(2-(tert-butoxy)-2-oxoethoxy)azetidine-1-carboxylate (4, 3.66 g, 43.0 %) as a white solid. m/z (ES+), [M+H]+ = 539; TFA, HPLC tR = 1.230 min.

### Step 5

To **Intermediate 4** (615 mg, 1.14 mmol) in DCM (4 mL) was added TFA (4 mL). The reaction mixture was stirred at room temperature for 1.5 h. The reaction mixture was concentrated under reduced pressure to give a crude product which was used without further purification. A solution of 1-[(38-oxo-2,5,8,11,14,17,20,23,26,29,32,35-dodecaoxaoctatriacontan-38-yl)oxy]-2,5-pyrrolidinedione (708 mg, 1.03 mmol) and DIPEA (0.397 mL, 2.28 mmol) in DMF (5 mL) was added to crude 2-((3-(((((9H-fluoren-9-yl)methoxy)carbonyl)amino)methyl)azetidin-3-yl)oxy)acetic acid (436 mg, 1.14 mmol). The reaction mixture was stirred at room temperature for 1.5 h. The reaction mixture was purified by reverse phase column chromatography (5-60% MeCN in water +0.1% FA) to give 2-((3-(((((9H-fluoren-9-yl)methoxy)carbonyl)amino)methyl)-1-(2,5,8,11,14,17,20,23,26,29,32,35-dodecaoxaoctatriacontan-38-oyl)azetidin-3-yl)oxy)acetic acid (**Intermediate 5,** 654 mg, 66.5 %) as a colourless oil. LCMS (15 min): 6.43 min; 954.7 [M+H]+

### Step 6

To **Intermediate 5** (615 mg, 1.14 mmol) in DCM (4 mL) was added diethylamine (4 mL). The reaction mixture was stirred at room temperature for 1.5 h. The reaction mixture was concentrated under reduced pressure to give a crude product which was used without further purification. To crude 2-((3-(aminomethyl)-1-(2,5,8,11,14,17,20,23,26,29,32,35-dodecaoxaoctatriacontan-38-oyl)azetidin-3-yl)oxy)acetic acid (504 mg, 0.69 mmol) was added 2,5-dioxopyrrolidin-1-yl 3-(2,5-dioxo-2,5-dihydro-1H-pyrrol-1-yl)propanoate (367 mg, 1.38 mmol) and N-ethyl-N-isopropylpropan-2-amine (0.240 mL, 1.38 mmol). The reaction mixture was stirred at room temperature for 2 h. The reaction mixture was purified by reverse phase chromatography (5-50% MeCN in water +0.1% FA) to give 2-((3-((3-(2,5-dioxo-2,5-dihydro-1H-pyrrol-1-yl)propanamido)methyl)-1-(2,5,8,11,14,17,20,23,26,29,32,35-dodecaoxaoctatriacontan-38-oyl)azetidin-3-yl)oxy)acetic acid (**Intermediate 6,** 280 mg, 46.0 %) as a colourless oil. LCMS (15 min): 4.66 min; 881. [M+H]+

### Step 7

To the stirred solution of **Intermediate 6** (1g, 1.13 mmol) in DCM (20 mL) was added tert-butyl 3-aminopropanoate hydrochloride (410 mg, 2.3 mmol) to the flask. Then added HATU (860 mg, 2.3mmol), DIEA (590 mg, 4.6mmol.) and DMF (5 ml) to the flask and stirred at room temperature for 2 hours. The reaction mixture was diluted with EtOAc (20 mL) and then washed with saturated sodium bicarbonate aqueous solution (10 mL), aqueous citric acid (1M, 10 mL), brine (10 mL). After dried over sodium sulfate, and filtered, the solution was concentrated to dryness to afford tert-butyl 3-(2-((3-((3-(2,5-dioxo-2,5-dihydro-1H-pyrrol-1-yl) propanamido) methyl)-1-(2,5,8,11,14,17,20,23,26,29,32,35-dodecaoxaoctatriacontan-38-oyl) azetidin-3-yl) oxy) acetamido)propanoate was obtained as an oil (**Intermediate 7,** 500 mg, 43.7%). MS ESI: [M+H]⁺ m/z 1009.4.

### Step 8

To the solution of **Intermediate 7** (550 mg, 0.5 mmol) in the mixed solvent of DCM (11 mL) was added TFA (5.5 mL) at 20 °C. The resulting solution was stirred at room temperature for 2 hours. After the starting material was consumed completely, the reaction solution was concentrated to dryness under vacuum. The crude residue was purified by silica gel column with 0-15% MeOH in DCM to afford 3-(2-((3-((3-(2,5-dioxo-2,5-dihydro-1H-pyrrol-1-yl)propanamido)methyl)-1-(2,5,8,11,14,17,20,23,26,29,32,35-dodecaoxaoctatriacontan-38-oyl)azetidin-3-yl)oxy)acetamido)propanoic acid as an oil (**Intermediate 8,** 200 mg, 38.5%). MS ESI: [M+H]⁺ m/z 953.4. 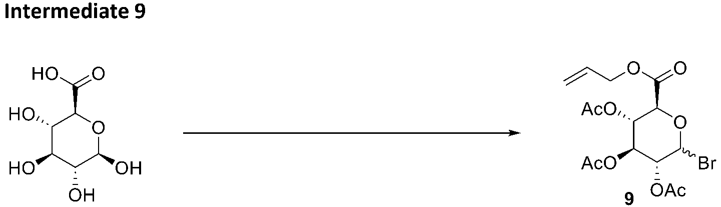 2,3,4,6,7,8,9,10-octahydropyrimido[1,2-a]azepine (26.5 ml, 177.35 mmol) was added dropwise to a 1-L round bottom flask containing (2S,3S,4S,5R,6R)-3,4,5,6-tetrahydroxytetrahydro-2H-pyran-2-carboxylic acid (31.3 g, 161.22 mmol) in DMF (100 ml) at 21 °C. Next, 3-bromoprop-1-ene (16.72 ml, 193.47 mmol) was added to the reaction mixture dropwise over 10 minutes and the reaction was stirred at 21 °C for 24 hours. Reaction mixture was cooled to 0 °C and treated with pyridine (104 mL, 1289.60 mmol). Acetic anhydride (244 mL, 2579.20 mmol) was next added to the reaction mixture. The reaction was warmed up to room temperature and run for 2 hours at 21 °C. Reaction mixture concentrated under reduced vacuum and the remaining pyridine was azeotropically removed with toluene (1 x 100 mL). Crude material was diluted with DCM (65 mL) and cooled to 0 °C. 30% hydrobromic acid in acetic acid (175 mL, 3226.03 mmol) was next added to the reaction mixture at 0 °C. The reaction was warmed up to room temperature and run for 2 hours 30 minutes at 21 °C. Solvent was evaporated then the compound was purified by normal phase flash column chromatography to afford (2*S*,3*S*,4*S*,5*R*,6*R*)-2-((allyloxy)carbonyl)-6-bromotetrahydro-2*H*-pyran-3,4,5-triyl triacetate (**Intermediate 9,** 33 g, 48% yield) as a beige translucent material. LCMS (ESI) m/z 445.0 (M + Na)+.

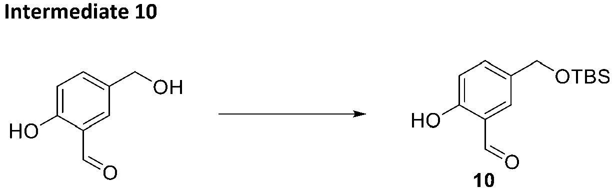

TBS-Cl (20.80 g, 138.02 mmol) in DCM (25 mL) was added dropwise to 1H-imidazole (17.90 g, 262.90 mmol) and 2-hydroxy-5-(hydroxymethyl)benzaldehyde (20 g, 131.45 mmol) in DCM (500 mL) at 0°C over a period of 2 hours under nitrogen. The resulting mixture was stirred at 0 °C for 2 hours. The reaction mixture was quenched with water (500 mL), extracted with DCM (2 x 300 mL) and the organic layer was dried over Na₂SO₄, filtered and evaporated to afford 5-(((tertbutyldimethylsilyl)oxy)methyl)-2-hydroxybenzaldehyde (**Intermediate 10,** 35.0 g, 100 %) as a colourless material. m/z (ES+), [M+Na]⁺ = 289; NH₄HCO₃, HPLC tR = 1.505 min.

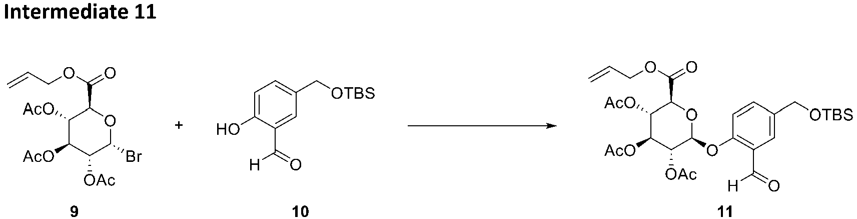

To a vacuum-dried 500 mL round-bottom flask was added molecular sieves (4 Å beads, 5.0 g), silver oxide (29.2 g, 125.8 mmol) and acetonitrile (150 mL), producing a black slurry. To this slurry was added a solution of **Intermediate 9** (10.7 g, 25.2 mmol) in acetonitrile (50 mL) over 20 min followed by the addition of **Intermediate 10** (13.6 g, 51.1 mmol) in acetonitrile (50 mL) in one portion. The resulting mixture was stirred vigorously at 20 °C for 16 h. After 16 h, the reaction mixture was filtered through a 5-cm pad of Celite and rinsed with dichloromethane (3 x 25 mL). Solvent was evaporated then the compound was purified by normal phase flash column chromatography to afford (25,35,45,5R,65)-2-((allyloxy)carbonyl)-6-(4-(((tert-butyldimethylsilyl)oxy)methyl)-2-formylphenoxy)tetrahydro-2H-pyran-3,4,5-triyl triacetate as a white material (**Intermediate 11,** 5.2 g, 34% yield). LCMS (ESI) m/z 626.3 (M + NH₄)+.

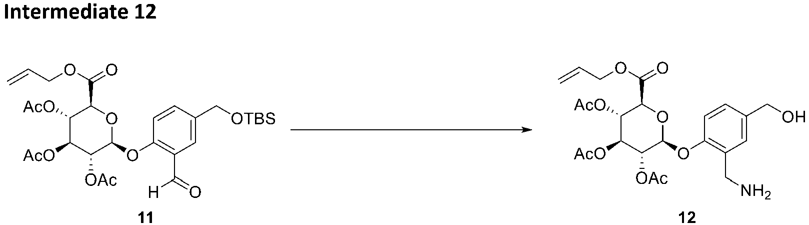

To a solution of **Intermediate 11** (5.2 g, 8.6 mmol) in acetonitrile (40 mL) was added tert-butyl carbamate (3.8 g, 32.3 mmol), trifluoroacetic acid (2.0 mL, 25.9 mmol), and triethylsilane (4.1 mL, 25.8 mmol). Stirred for 2 h at 20 °C then solvent was evaporated. To the resulting colorless oil was added 1,4-dioxane (8 mL) and HCl (4.0 M in 1,4-dioxane, 50 mL, 200 mmol). The mixture was stirred at 20 °C for 30 min the solvent was evaporated. The resulting white powder was dissolved in DMSO (3 mL) then passed through cation-exchange resin pre-treated with methanol (WATERS PORAPAK CX). The desired compound was eluted off the resin with methanol to afford (25,35,45,5R,65)-2-((allyloxy)carbonyl)-6-(2-(aminomethyl)-4-(hydroxymethyl)phenoxy)tetrahydro-2H-pyran-3,4,5-triyl triacetate as a white material (**Intermediate 12,** 2.5 g, 80% over 2 steps). LCMS (ESI) m/z 496.5 (M + H)+.

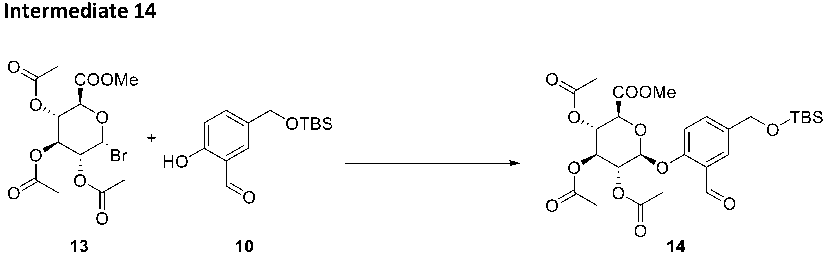

Silver(I) oxide p.a. (52.2 g, 225.22 mmol) was added to 4Å Molecular Sieves (30 g), (2R,3R,4S,5S,6S)-2-bromo-6-(methoxycarbonyl)tetrahydro-2H-pyran-3,4,5-triyl triacetate (**Intermediate 13**, 44.7 g, 112.61 mmol) , **Intermediate 10** (15 g, 56.30 mmol) in MeCN (500 mL) at 0°C over a period of 2 hours under nitrogen. The resulting mixture was stirred at 25 °C for 16 hours. The mixture was filtered through a Celite pad. The crude product was purified by flash silica chromatography, elution gradient 0 to 30% EtOAc in petroleum ether. Pure fractions were evaporated to dryness to afford (2S,3R,4S,5S,6S)-2-(4-(((tert-butyldimethylsilyl)oxy)methyl)-2-formylphenoxy)-6-(methoxycarbonyl)tetrahydro-2H-pyran-3,4,5-triyl triacetate (**Intermediate 14**) as a pale yellow material. m/z (ES+), [M+Na]+ = 605; TFA, HPLC tR = 1.071 min.

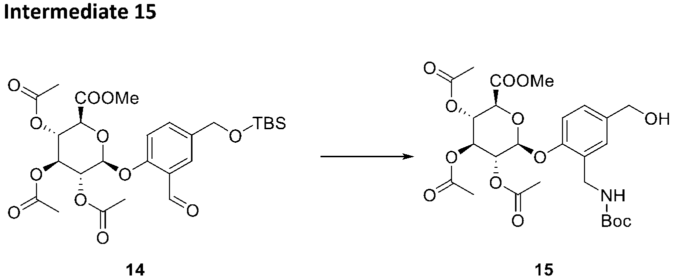

TFA (8 mL) was added to Et₃SiH (32 mL), tert-butyl carbamate (15.28 g, 130.43 mmol) and **Intermediate 14** (20 g, 34.32 mmol) in MeCN (200 mL) at 0°C under nitrogen. The resulting mixture was stirred at 0°C for 16 hours. The reaction mixture was quenched with saturated NaHCO₃ (200 mL), extracted with EtOAc (2 x 300 mL), and the organic layer was dried over Na₂SO₄, filtered and evaporated to afford a colourless residue. The crude product was purified by flash silica chromatography, elution gradient 0 to 50% THF in petroleum ether. Pure fractions were evaporated to dryness to afford (2S,3R,4S,5S,6S)-2-(2-(((tert-butoxycarbonyl)amino)methyl)-4-(hydroxymethyl)phenoxy)-6-(methoxycarbonyl)tetrahydro-2H-pyran-3,4,5-triyl triacetate **(Intermediate 15,** 18.80 g, 96 %) as a pale yellow material. m/z (ES+), [M+Na]⁺ = 592; TFA, HPLC tR = 1.088 min.

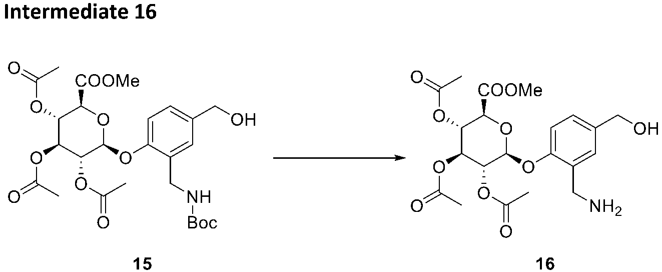

**Intermediate 15** (10 g, 17.56 mmol) in HCl/1,4-dioxane (40 mL)/1,4-dioxane (40 mL) at 25°C under nitrogen. The resulting mixture was stirred at 25 °C for 1 hour. The solvent was removed under reduced pressure to afford (25,3R,45,55,65)-2-(2-(aminomethyl)-4-(hydroxymethyl)phenoxy)-6-(methoxycarbonyl)tetrahydro-2H-pyran-3,4,5-triyl triacetate hydrochloride (**Intermediate 16**) as a white material. m/z (ES+), [M+H]+ = 470; TFA, HPLC tR = 0.587 min.

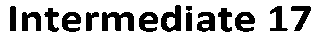

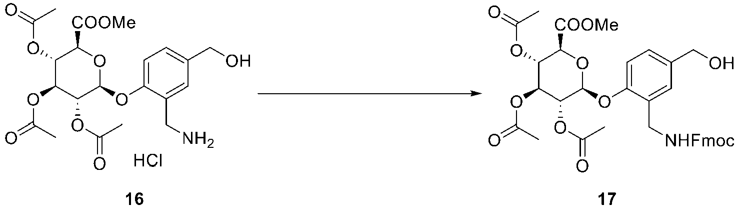

**Intermediate 16** (1 g, 1.98 mmol), (9H-fluoren-9-yl)methyl carbonochloridate (0.767 g, 2.97 mmol) and DIPEA (1.036 mL, 5.93 mmol) in DCM (10 mL) was stirred at RT for 3 hours. The reaction mixture was quenched with water (50 mL), extracted with DCM (2 x 50 mL), the combined organic layer was dried over Na₂SO₄, filtered and evaporated to afford a residue. The crude product was purified by flash silica chromatography, elution gradient 0 to 80% petroleum ether in EtOAc. Pure fractions were evaporated to dryness to afford (2S,3R,4S,5S,6S)-2-(2-(((((9H-fluoren-9-yl)methoxy)carbonyl)amino)methyl)-4-(hydroxymethyl)phenoxy)-6-(methoxycarbonyl)tetrahydro-2H-pyran-3,4,5-triyl triacetate (**Intermediate 17,** 0.952 g, 69.6 %) as a white material. m/z (ES+), [M+H]+ = 692; TFA, HPLC tR = 0.880 min.

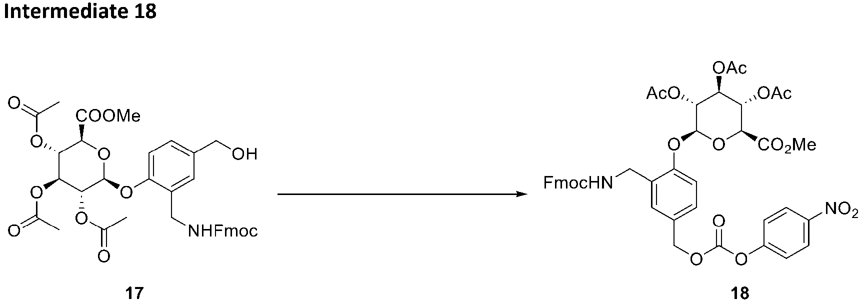

**Intermediate 17** (3.5 g, 5.06 mmol), bis(4-nitrophenyl) carbonate (1.693 g, 5.57 mmol) and DIEA (1.326 mL, 7.59 mmol) in DMF (35mL) was stirred at RT for 3 hours. The reaction mixture was quenched with water (50 mL), extracted with EtOAc (2 x 50 mL), and the combined organic layer was dried over Na₂SO₄, filtered and evaporated to afford residue. The crude product was purified by flash silica chromatography, elution gradient 0 to 100% EtOAc in petroleum ether. Pure fractions were evaporated to dryness to afford (2S,3R,4S,5S,6S)-2-(2-(((((9H-fluoren-9-yl)methoxy)carbonyl)amino)methyl)-4-((((4-nitrophenoxy)carbonyl)oxy)methyl)phenoxy)-6-(methoxycarbonyl)tetrahydro-2H-pyran-3,4,5-triyl triacetate (**Intermediate 18,** 3.68 g, 85 %) as a white material. m/z (ES+), [M+H]+ = 857; fa, HPLC tR = 1.007 min.

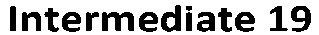

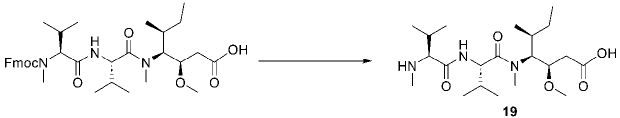

(55,85,115,12R)-11-((S)-sec-butyl)-1-(9H-fluoren-9-yl)-5,8-diisopropyl-12-methoxy-4,10-dimethyl-3,6,9-trioxo-2-oxa-4,7,10-triazatetradecan-14-oic acid (12 g, 18.81 mmol) in trimethylamine (80 mL) was stirred at 70 °C for 3 hours. The solvent was removed under reduced pressure. The precipitate was collected by filtration, washed with petroleum ether (100 mL) and dried under vacuum to afford (3R,45,55)-4-((S)-N,3-dimethyl-2-((S)-3-methyl-2-(methylamino)butanamido)butanamido)-3-methoxy-5-methylheptanoic acid (**Intermediate 19,** 5.80 g, 74.2 %) as a white solid. LCMS (3 mins): No PDA; 416 [M+H]+.

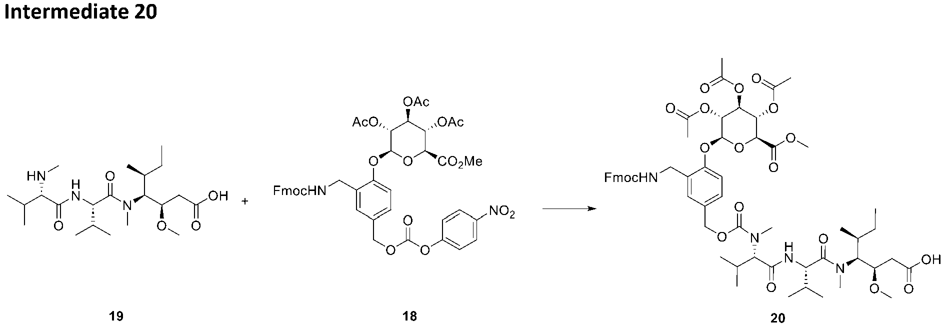

**Intermediate 18** (1.5 g, 1.75 mmol), **Intermediate 19** (0.728 g, 1.75 mmol), DIPEA (0.917 mL, 5.25 mmol) and 2-Hydroxypyridine-N-oxide (0.195 g, 1.75 mmol) in DMA (200mL) were stirred at RT for 18 hours. The reaction mixture was extracted with ethyl acetate (3 x 50 mL), the organic layer was dried over Na₂SO₄, filtered and evaporated to afford yellow material. The crude product was purified by preparative HPLC (Column: XBRIDGE Prepl phenyl OBD Colum, 30*150nm 5um; Mobile Phase A: Water(10mmol/L NH₄HCO₃), Mobile Phase B: ACN; Flow rate: 60 mL/min; Gradient: isocratic 54-59; Wave Length: 220/254 nm; RT1(min): 15. Fractions containing the desired compound were evaporated to dryness to afford (5S,8S,11S,12R)-1-(3-(((((9H-fluoren-9-yl)methoxy)carbonyl)amino)methyl)-4-(((2S,3R,4S,5S,6S)-3,4,5-triacetoxy-6-(methoxycarbonyl)tetrahydro-2H-pyran-2-yl)oxy)phenyl)-11-((S)-sec-butyl)-5,8-diisopropyl-12-methoxy-4,10-dimethyl-3,6,9-trioxo-2-oxa-4,7,10-triazatetradecan-14-oic acid (**Intermediate 20,** 1.400 g, 70.6 %) as a material. m/z (ES+), [M+H]+ = 1134; FA, HPLC tR = 1.197 min.

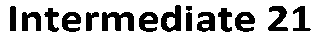

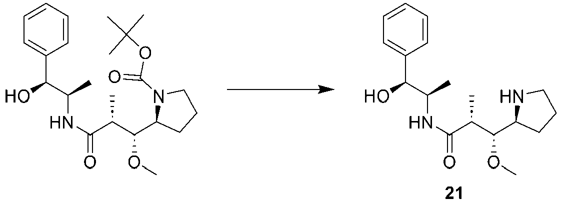

To tert-butyl (S)-2-((1R,2R)-3-(((1S,2R)-1-hydroxy-1-phenylpropan-2-yl)amino)-1-methoxy-2-methyl-3-oxopropyl)pyrrolidine-1-carboxylate (256 mg, 0.61 mmol) in DCM (2 mL) was added TFA (2 mL). The reaction mixture was stirred at room temperature for 2 h. The reaction mixture was concentrated under reduced pressure to give a crude (2R,3R)-N-((1S,2R)-1-hydroxy-1-phenylpropan-2-yl)-3-methoxy-2-methyl-3-((S)-pyrrolidin-2-yl)propanamide (**Intermediate 21**) which was used without purification. LCMS (3 min): 0.55 min; 322.0 [M+H]+

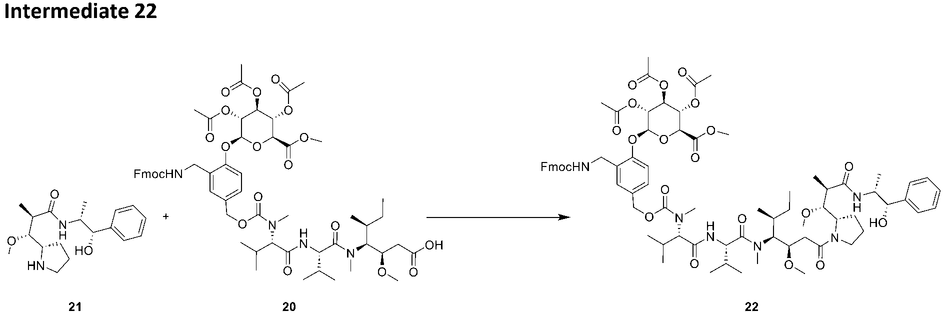

To crude **Intermediate 21** (234 mg, 0.73 mmol) in DMF (5 mL) was added **Intermediate 20** (486 mg, 0.429 mmol), HATU (269 mg, 0.71 mmol) and DIPEA (0.360 mL, 2.07 mmol). The reaction mixture was stirred at room temperature for 30 mins. The reaction mixture was concentrated under reduced pressure to give a crude. Purification by column chromatography (0-6% MeOH in DCM) gave (2S,3R,4S,5S,6S)-2-(2-(((((9H-fluoren-9-yl)methoxy)carbonyl)amino)methyl)-4-((5S,8S,11S,12R)-11-((S)-sec-butyl)-12-(2-((S)-2-((1R,2R)-3-(((15,2R)-1-hydroxy-1-phenylpropan-2-yl)amino)-1-methoxy-2-methyl-3-oxopropyl)pyrrolidin-1-yl)-2-oxoethyl)-5,8-diisopropyl-4,10-dimethyl-3,6,9-trioxo-2,13-dioxa-4,7,10-triazatetradecyl)phenoxy)-6-(methoxycarbonyl)tetrahydro-2H-pyran-3,4,5-triyl triacetate (**Intermediate 22,** 552 mg, 90 %) as a colourless material. LCMS (15 min): 8.99 min; 1436.9 [M+H]+

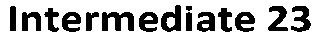

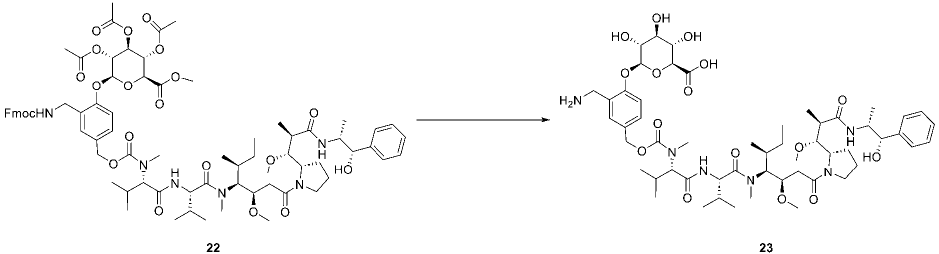

To **Intermediate 22** (552 mg, 0.38 mmol) in MeOH (8 mL) and water (2 mL) was added potassium carbonate (531 mg, 3.84 mmol). The reaction mixture was stirred at room temperature for 3 h. Acetic acid (1.1 mL) was added and the reaction mixture purified by reverse phase chromatography (C18, 120 g, 5-40% MeCN in water +0.1% FA, 25 CV) to give (2S,3S,4S,5R,6S)-6-(2-(aminomethyl)-4-((5S,8S,11S,12R)-11-((S)-sec-butyl)-12-(2-((S)-2-((1R,2R)-3-(((15,2R)-1-hydroxy-1-phenylpropan-2-yl)amino)-1-methoxy-2-methyl-3-oxopropyl)pyrrolidin-1-yl)-2-oxoethyl)-5,8-diisopropyl-4,10-dimethyl-3,6,9-trioxo-2,13-dioxa-4,7,10-triazatetradecyl)phenoxy)-3,4,5-trihydroxytetrahydro-2H-pyran-2-carboxylic acid (**Intermediate 23,** 259 mg, 62.8 %) as a white material. LCMS (15 min): 5.57 min; 1074.9 [M+H]⁺.

### Synthesis of LP2

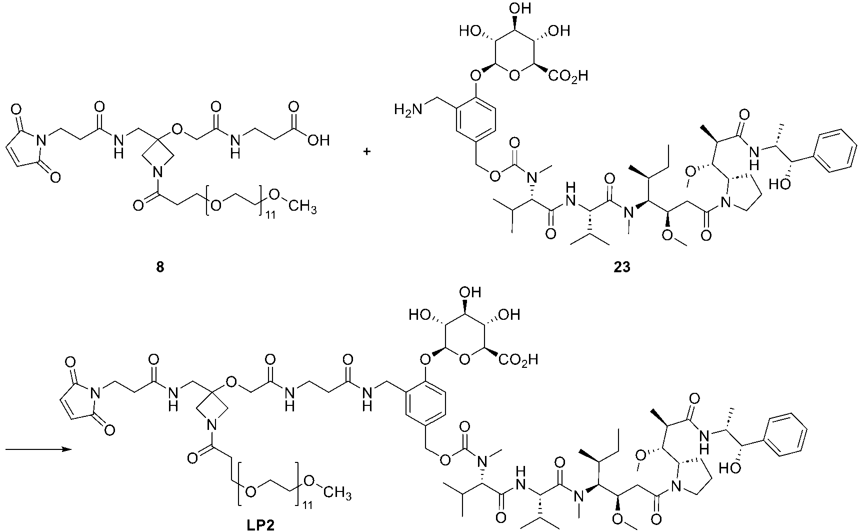

To the solution of **Intermediate 8** (460mg, 0.5 mmol) in DMF (4.6 ml) was added the solution of **Intermediate 23** (520 mg, 0.48 mmol), HBTU (275 mg, 0.49 mmol) and DIEA (187 mg, 0.48 mmol) at - 15 to -10 °C. After stirring for 10mins, the crude residue was quenched with HCl in 1,4-dioxane. The crude product was purified by prep-HPLC (column : C18 reverse column ; gradient : MeCN in water (0.1 A% FA) : 25-40%)) to afford (2S,3S,4S,5R,6S)-6-(4-((5S,8S,11S,12R)-11-((S)-sec-butyl)-12-(2-((S)-2-((1R,2R)-3-(((15,2R)-1-hydroxy-1-phenylpropan-2-yl)amino)-1-methoxy-2-methyl-3-oxopropyl)pyrrolidin-1-yl)-2-oxoethyl)-5,8-diisopropyl-4,10-dimethyl-3,6,9-trioxo-2,13-dioxa-4,7,10-triazatetradecyl)-2-((3-(2-((3-((3-(2,5-dioxo-2,5-dihydro-1H-pyrrol-1-yl)propanamido)methyl)-1-(2,5,8,11,14,17,20,23,26,29,32,35-dodecaoxaoctatriacontan-38-oyl)azetidin-3-yl)oxy)acetamido)propanamido)methyl)phenoxy)-3,4,5-trihydroxytetrahydro-2H-pyran-2-carboxylic acid as a light yellow solid. (**LP2,** 190 mg, yield: 20%). MS ESI: [M/2+H]+ m/z 1004.4.

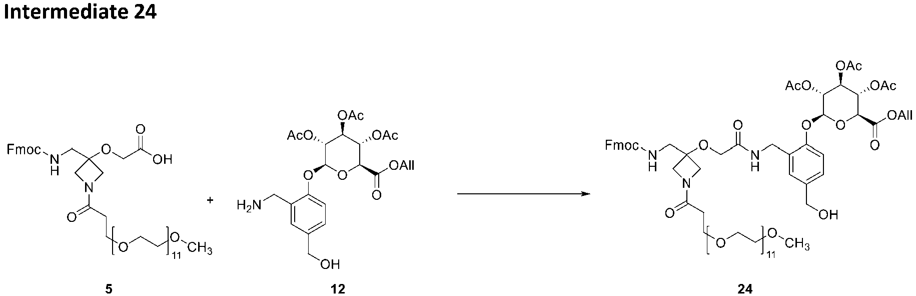

**Intermediate 5** (200 mg, 0.21 mmol) and Intermediate 12 (109 mg, 0.22 mmol) were stirred in DMF (5 mL) with 2-(3H-[1,2,3]triazolo[4,5-b]pyridin-3-yl)-1,1,3,3-tetramethylisouronium hexafluorophosphate(V) (88 mg, 0.23 mmol) for 1h at room temperature. The crude reaction was concentrated under reduced pressure and purified by column chromatography (dichloromethane : methanol from to 0 to 10%) to afford (2S,3R,4S,5S,6S)-2-(2-((2-((3-(((((9H-fluoren-9-yl)methoxy)carbonyl)amino)methyl)-1-(2,5,8,11,14,17,20,23,26,29,32,35-dodecaoxaoctatriacontan-38-oyl)azetidin-3-yl)oxy)acetamido)methyl)-4-(hydroxymethyl)phenoxy)-6-((allyloxy)carbonyl)tetrahydro-2H-pyran-3,4,5-triyl triacetate (**Intermediate 24,** 200 mg, 66% yield). LCMS (3 min): 1.88 min; 1431.5 [M+H]+, 1453.3 [M+Na]+.

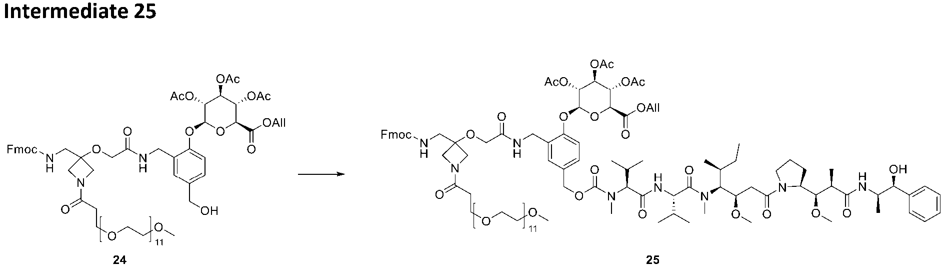

**Intermediate 24** (200 mg, 0.14 mmol) was dissolved in DMF (5 mL). Bis(4-nitrophenyl) carbonate (85 mg, 0.28 mmol) was added in one portion followed by addition of N-ethyl-N-isopropylpropan-2-amine (0.073 mL, 0.42 mmol) and stirred overnight at 25 °C. An additional 500 mg of bis(4-nitrophenyl) carbonate was added and the reaction was left stirring at 25 °C for 3h. The crude reaction was concentrated under reduced pressure and purified by column chromatography (dichloromethane : methanol from 0% to 5%). The isolated intermediate was dissolved in DMF (5 mL) followed by addition of (S)-N-((3R,4S,5S)-1-((S)-2-((1R,2R)-3-(((1S,2R)-1-hydroxy-1-phenylpropan-2-yl)amino)-1-methoxy-2-methyl-3-oxopropyl)pyrrolidin-1-yl)-3-methoxy-5-methyl-1-oxoheptan-4-yl)-N,3-dimethyl-2-((S)-3-methyl-2-(methylamino)butanamido)butanamide (100 mg, 0.14 mmol), 2-hydroxypyridine 1-oxide (16.31 mg, 0.15 mmol) and N-ethyl-N-isopropylpropan-2-amine (0.073 mL, 0.42 mmol). The reaction was stirred overnight at 25 °C. The crude reaction was concentrated under reduced pressure then purified by column chromatography (dichloromethane : methanol from 0% to 5%) to obtain (2S,3R,4S,5S,6S)-2-(2-((2-((3-(((((9H-fluoren-9-yl)methoxy)carbonyl)amino)methyl)-1-(2,5,8,11,14,17,20,23,26,29,32,35-dodecaoxaoctatriacontan-38-oyl)azetidin-3-yl)oxy)acetamido)methyl)-4-((5S,8S,11S,12R)-11-((S)-sec-butyl)-12-(2-((S)-2-((1R,2R)-3-(((15,2R)-1-hydroxy-1-phenylpropan-2-yl)amino)-1-methoxy-2-methyl-3-oxopropyl)pyrrolidin-1-yl)-2-oxoethyl)-5,8-diisopropyl-4,10-dimethyl-3,6,9-trioxo-2,13-dioxa-4,7,10-triazatetradecyl)phenoxy)-6-((((E)-prop-1-en-1-yl)oxy)carbonyl)tetrahydro-2H-pyran-3,4,5-triyl triacetate (**Intermediate 25**, 200 mg, 65% yield). LCMS (3 min): 2.05 min; 1088.5 [M+2H]2+.

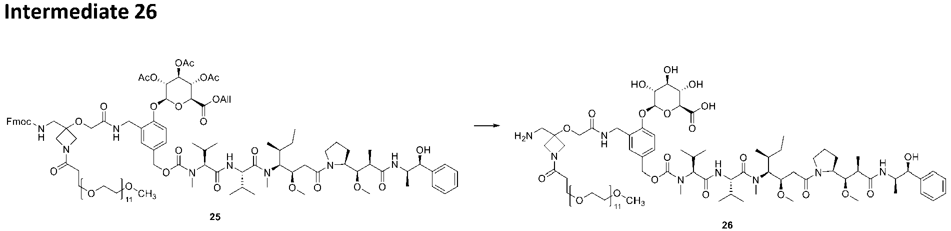

**Intermediate 25** (200 mg, 0.09 mmol) was dissolved in DCM (3 mL) followed by addition of formic acid (6.94 µl, 0.18 mmol), triethylamine (0.026 mL, 0.18 mmol) and tetrakis(triphenylphosphine)palladium(0) (15.94 mg, 0.01 mmol). The reaction was stirred at 25 °C for 30 minutes. The reaction was concentrated under reduced pressure and the residue was redissolved in 2 mL MeOH and 2 mL of THF. LiOH (50 mg) was added in 1mL of water. After stirring at 25 °C for 1h the reaction was concentrated and purified by reverse-phase chromatography (0.1% formic acid, water : acetonitrile from 15 to 40%) to afford (2S,3S,4S,5R,6S)-6-(2-((2-((3-(aminomethyl)-1-(2,5,8,11,14,17,20,23,26,29,32,35-dodecaoxaoctatriacontan-38-oyl)azetidin-3-yl)oxy)acetamido)methyl)-4-((5S,8S,11S,12R)-11-((S)-sec-butyl)-12-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-5,8-diisopropyl-4,10-dimethyl-3,6,9-trioxo-2,13-dioxa-4,7,10-triazatetradecyl)phenoxy)-3,4,5-trihydroxytetrahydro-2H-pyran-2-carboxylic acid **(Intermediate 26,** 100 mg, 61% yield). LCMS (3 min): 1.48 min; 1787.0 [M+H]+

### Synthesis of LP3

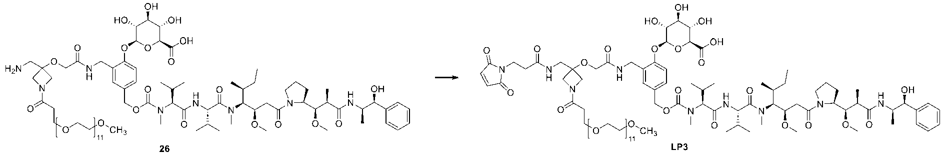

**Intermediate 26** (100 mg, 0.06 mmol) was dissolved in DMF (3 mL) followed by addition of 2,5-dioxopyrrolidin-1-yl 3-(2,5-dioxo-2,5-dihydro-1H-pyrrol-1-yl)propanoate (16.39 mg, 0.06 mmol) and N-ethyl-N-isopropylpropan-2-amine (0.029 mL, 0.17 mmol). The reaction was stirred at 25 °C for 10 minutes then concentrated under reduced pressured and purified by preparative HPLC (0.1% formic acid, water : acetonitrile from 15 to 60%) to afford (2S,3S,4S,5R,6S)-6-(4-((5S,8S,11S,12R)-11-((S)-sec-butyl)-12-(2-((S)-2-((1R,2R)-3-(((15,2R)-1-hydroxy-1-phenylpropan-2-yl)amino)-1-methoxy-2-methyl-3-oxopropyl)pyrrolidin-1-yl)-2-oxoethyl)-5,8-diisopropyl-4,10-dimethyl-3,6,9-trioxo-2,13-dioxa-4,7,10-triazatetradecyl)-2-((2-((3-((3-(2,5-dioxo-2,5-dihydro-1H-pyrrol-1-yl)propanamido)methyl)-1-(2,5,8,11,14,17,20,23,26,29,32,35-dodecaoxaoctatriacontan-38-oyl)azetidin-3-yl)oxy)acetamido)methyl)phenoxy)-3,4,5-trihydroxytetrahydro-2H-pyran-2-carboxylic acid (**LP3,** 71 mg, 65% yield). LCMS (15 min): 6.59 min; 1937.7 [M]+.

### Synthesis of LP4

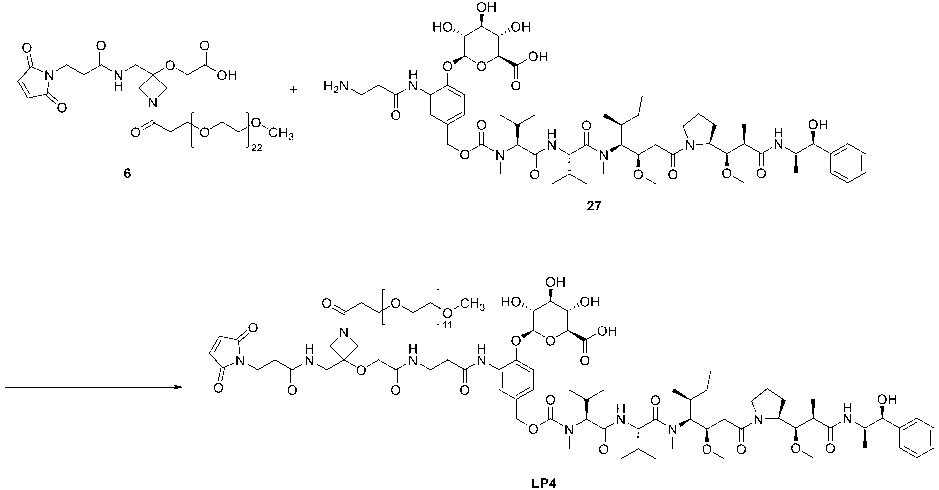

**Intermediate 27** was synthesised as previously described in WO2016/149535. To **Intermediate 6** (66.8 mg, 0.08 mmol) in DMF (2 mL) was added HATU (15.47 mg, 0.04 mmol) and DIPEA (28.4 µl, 0.16 mmol). The reaction mixture was stirred at room temperature for 10 mins. (2S,3S,4S,5R,6S)-6-(2-(3-aminopropanamido)-4-((55,85,115,12R)-11-((S)-sec-butyl)-12-(2-((S)-2-((1R,2R)-3-(((15,2R)-1-hydroxy-1-phenylpropan-2-yl)amino)-1-methoxy-2-methyl-3-oxopropyl)pyrrolidin-1-yl)-2-oxoethyl)-5,8-diisopropyl-4,10-dimethyl-3,6,9-trioxo-2,13-dioxa-4,7,10-triazatetradecyl)phenoxy)-3,4,5-trihydroxytetrahydro-2H-pyran-2-carboxylic acid (**Intermediate 27,** 46 mg, 0.04 mmol) in DMF (2 mL) was added and the reaction mixture stirred at room temperature for 30 mins. A further amount of DIPEA (28.4 µl, 0.16 mmol) was added and the reaction mixture stirred at 25 °C overnight. The reaction mixture was concentrated under reduced pressure to give a crude product which was purified by reverse phase preparative HPLC (C18, CSH, 0.1% formic acid, 15-30% MeCN in water) to give (2S,3S,4S,5R,6S)-6-(4-((5S,8S,11S,12R)-11-((S)-sec-butyl)-12-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-5,8-diisopropyl-4,10-dimethyl-3,6,9-trioxo-2,13-dioxa-4,7,10-triazatetradecyl)-2-(3-(2-((3-((3-(2,5-dioxo-2,5-dihydro-1H-pyrrol-1-yl)propanamido)methyl)-1-(2,5,8,11,14,17,20,23,26,29,32,35-dodecaoxaoctatriacontan-38-oyl)azetidin-3-yl)oxy)acetamido)propanamido)phenoxy)-3,4,5-trihydroxytetrahydro-2H-pyran-2-carboxylic acid (**LP4,** 41.0 mg, 50.5 %) as a colourless oil. LCMS (15 min): 6.56 min; 998.6 [M+2H]+, 1009.4 [M+H+Na]+, 666.2 [M+3H]+

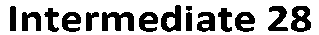

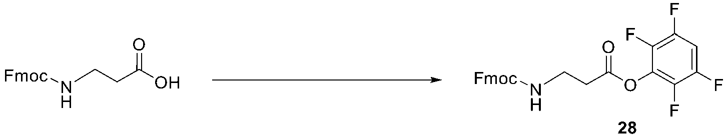

1-(3-Dimethylaminopropyl)-3-ethyl-carbodiimide (1.197 g, 7.71 mmol) was added to 2,3,5,6-tetrafluorophenol (1.280 g, 7.71 mmol) and 3-((((9H-fluoren-9-yl)methoxy)carbonyl)amino)propanoic acid (2g, 6.42 mmol) in DCM (20 mL) at 25°C. The resulting mixture was stirred at 25 °C for 2 hours. The reaction mixture was quenched with water (5 mL), extracted with DCM (3 x 5 mL), the organic layer was dried over Na₂SO₄, filtered and evaporated to afford 2,3,5,6-tetrafluorophenyl 3-((((9H-fluoren-9-yl)methoxy)carbonyl)amino)propanoate **(Intermediate 28,** 1.500 g, 50.8 %) as a white solid. LCMS (3 mins): 1.35 min; 460 [M+H]+.

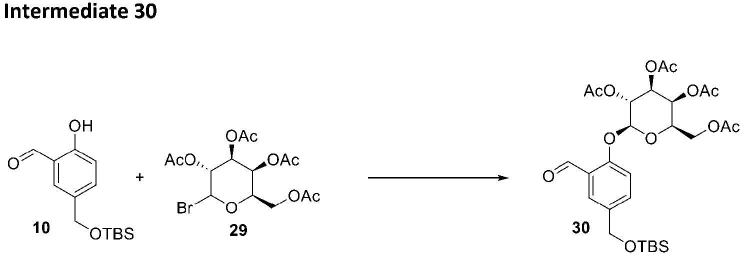

Silver(I) oxide p.a. (34.8 g, 150.14 mmol) was added to 4Å molecular sieves (40 g, 0.00 mmol), **Intermediate 29** (37.0 g, 90.09 mmol) and **Intermediate 10** (20 g, 75.07 mmol) in MeCN (500 mL) at 0°C under nitrogen. The resulting mixture was stirred at 25 °C for 16 hours. The mixture was filtered through a Celite pad. The crude product was purified by flash silica chromatography, elution gradient 0 to 50% THF in petroleum ether. Pure fractions were evaporated to dryness to afford (2R,35,45,5R,65)-2-(acetoxymethyl)-6-(4-(((tert-butyldimethylsilyl)oxy)methyl)-2-formylphenoxy)tetrahydro-2H-pyran-3,4,5-triyl triacetate (**Intermediate 30,** 36.0 g, 80 %) as a pale yellow solid. LCMS (3 mins): 1.06 min; 619 [M+Na]+.

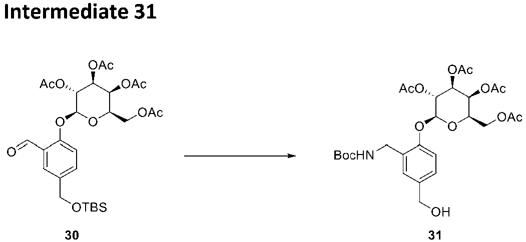

TFA (14 mL) was added to Et₃SiH (56 mL), tert-butyl carbamate (26.1 g, 222.89 mmol) and **Intermediate 30** (35 g, 58.66 mmol) in MeCN (350 mL) at 0°C under nitrogen. The resulting mixture was stirred at 0°C for 16 hours. The reaction mixture was quenched with saturated NaHCO₃ (200 mL), extracted with EtOAc (2 x 250 mL), and the organic layer was dried over Na₂SO₄, filtered and evaporated to afford a colourless residue. The crude product was purified by flash silica chromatography, elution gradient 0 to 100% THF in petroleum ether. Pure fractions were evaporated to dryness to afford (2R,3S,4S,5R,6S)-2-(acetoxymethyl)-6-(2-(((tert-butoxycarbonyl)amino)methyl)-4-(hydroxymethyl)phenoxy)tetrahydro-2H-pyran-3,4,5-triyl triacetate (**Intermediate 31,** 24.00 g, 70.1 %) as a pale yellow solid. LCMS (3 min): 0.79 min; 606 [M+Na]+.

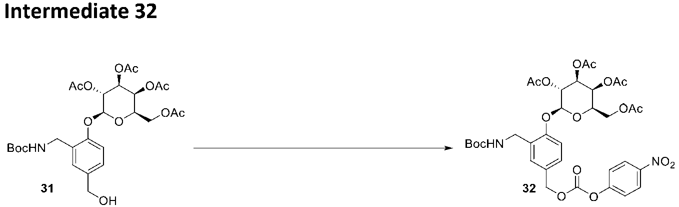

**Intermediate 31** (5 g, 8.57 mmol), bis(4-nitrophenyl) carbonate (3.13 g, 10.28 mmol) and DIEA (2.99 mL, 17.14 mmol) in DMF (60 mL) were stirred at 25°C for 3 hours. The reaction mixture was quenched with water (50 mL), extracted with EtOAc (2 x 50 mL), and the combined organic layer was dried over Na₂SO₄, filtered and evaporated to afford a residue. The crude product was purified by flash silica chromatography, elution gradient 0 to 100% EtOAc in petroleum ether. Pure fractions were evaporated to dryness to afford (2R,3S,4S,5R,6S)-2-(acetoxymethyl)-6-(2-(((tert-butoxycarbonyl)amino)methyl)-4-((((4-nitrophenoxy)carbonyl)oxy)methyl)phenoxy)tetrahydro-2H-pyran-3,4,5-triyl triacetate (**Intermediate 32,** 3.39 g, 52.8 %) as a white solid. LCMS (3 mins): 0.94 min; 749 [M+H]+.

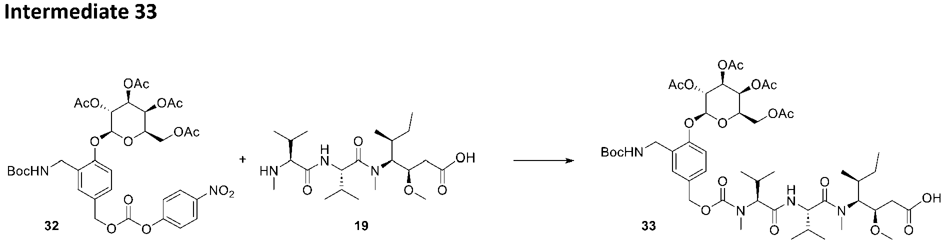

2-Hydroxypyridine-N-oxide (0.555 g, 5.00 mmol) was added to DIEA (1.586 mL, 9.08 mmol), **Intermediate 19** (1.887 g, 4.54 mmol) and **Intermediate 32** (3.4 g, 4.54 mmol) in DMA (50mL) at 25°C under nitrogen. The resulting mixture was stirred at 25 °C for 1 hour. The reaction mixture was quenched with water (50 mL) and 0.1M HCl (50 mL), extracted with EtOAc (2 x 50 mL), washed with 0.1 HCl, and the organic layer was dried over Na₂SO₄, filtered and evaporated to afford a residue. The crude product was purified by flash C18-flash chromatography, elution gradient 0 to 100% MeCN in water. Pure fractions were evaporated to dryness to afford (55,85,115,12R)-1-(3-(((tert-butoxycarbonyl)amino)methyl)-4-(((25,3R,45,55,6R)-3,4,5-triacetoxy-6-(acetoxymethyl)tetrahydro-2H-pyran-2-yl)oxy)phenyl)-11-((S)-sec-butyl)-5,8-diisopropyl-12-methoxy-4,10-dimethyl-3,6,9-trioxo-2-oxa-4,7,10-triazatetradecan-14-oic acid (**Intermediate 33,** 1.900 g, 40.8 %) as a white solid. LCMS (3 mins): 0.92 min; 1026 [M+H]+.

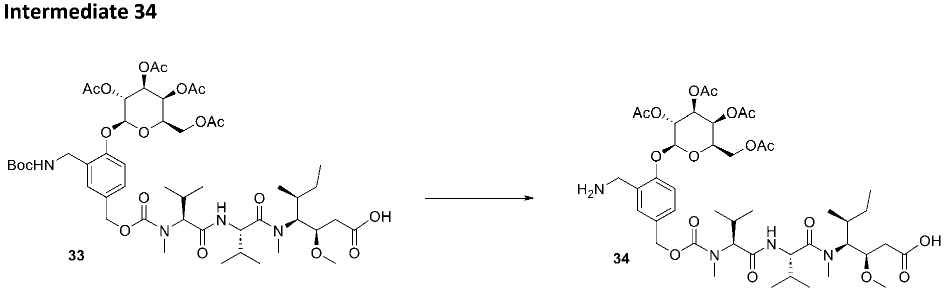

**Intermediate 33** (1.9 g, 1.85 mmol) was added to TFA (10 mL) and DCM (10.00 mL) at 25°C under nitrogen. The resulting mixture was stirred at 25 °C for 1 hour. The solvent was removed under reduced pressure to afford (5S,8S,11S,12R)-1-(3-(aminomethyl)-4-(((2S,3R,4S,5S,6R)-3,4,5-triacetoxy-6-(acetoxymethyl)tetrahydro-2H-pyran-2-yl)oxy)phenyl)-11-((S)-sec-butyl)-5,8-diisopropyl-12-methoxy-4,10-dimethyl-3,6,9-trioxo-2-oxa-4,7,10-triazatetradecan-14-oic acid (**Intermediate 34,** 1.500 g, 87 %) as a brown oil. LCMS (3 min): 0.64 min; 925 [M+H]+.

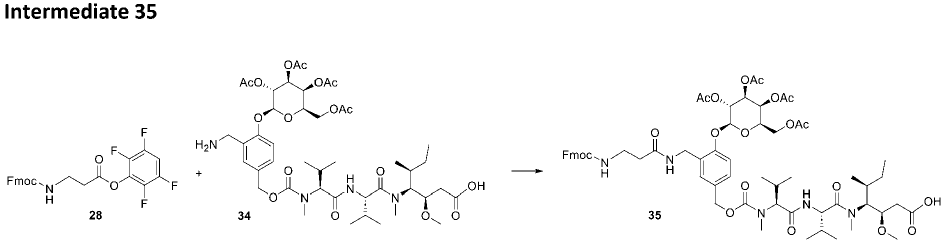

**Intermediate 34** (1.5 g, 1.47 mmol), **Intermediate 28** (0.741 g, 1.61 mmol) in THF (7 mL) and NaHCO₃ (7.00 mL) were stirred at 25°C for 3 hours. The solvent was removed under reduced pressure. The reaction mixture was acidified with 2M HCl. The crude product was purified by preparative HPLC Column: Xselect CSH Prep C18 OBD Colum, 30*150nm, 5µm; Mobile Phase A: Water(0.1%FA), Mobile Phase B: ACN; Flow rate: 60 mL/min mL/min; Gradient: 5% B to 5% B in 1.5 min, 5% B to 54% B in 2 min, 54% B to59% B in 10 min; Wave Length: 220nm nm; RT1(min): 9.6. Fractions containing the desired compound were evaporated to dryness to afford (55,85,115,12R)-1-(3-((3-((((9H-fluoren-9-yl)methoxy)carbonyl)amino)propanamido)methyl)-4-(((25,3R,45,55,6R)-3,4,5-triacetoxy-6-(acetoxymethyl)tetrahydro-2H-pyran-2-yl)oxy)phenyl)-11-((S)-sec-butyl)-5,8-diisopropyl-12-methoxy-4,10-dimethyl-3,6,9-trioxo-2-oxa-4,7,10-triazatetradecan-14-oic acid **(Intermediate** 35, 0.700 g, 39.2 %) as a white solid. LCMS (3 min): 1.31 min; 1218 [M+H]+.

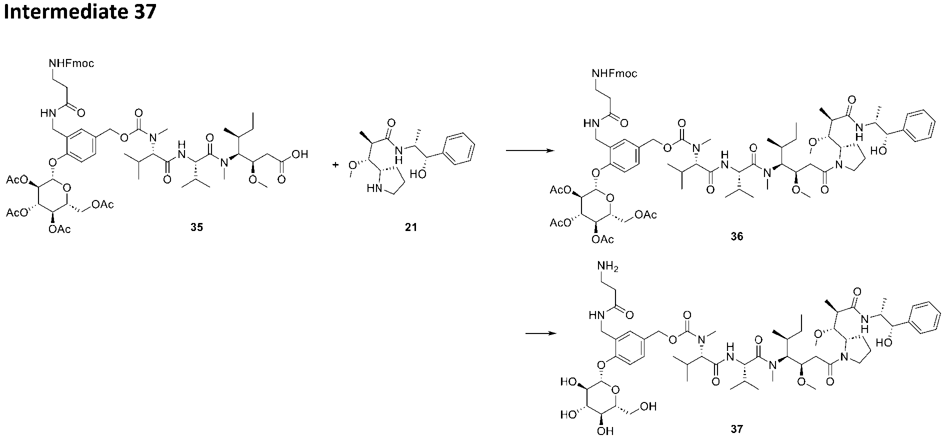

**Intermediate 21,** (250 mg, 0.78 mmol) was dissolved in dry DMF (2 mL) followed by addition of **Intermediate 35** (300 mg, 0.25 mmol), 2-(3H-[1,2,3]triazolo[4,5-b]pyridin-3-yl)-1,1,3,3-tetramethylisouronium hexafluorophosphate(V) (94 mg, 0.25 mmol) and N-ethyl-N-isopropylpropan-2-amine (214 uL, 1.23 mmol). The reaction mixture was stirred at 25°C for 10 mins followed by evaporation under reduced pressure. The crude product was purified by column chromatography (dichloromethane : methanol 10%, from 0 to 100%). The obtained **Intermediate 36** was dissolved in methanol (5 mL) followed by addition of potassium carbonate (100 mg). The reaction was stirred for 1h at 25°C followed by evaporation under reduced pressure. The crude reaction mixture was purified by reverse phase column chromatography (water : acetonitrile from 10 to 50%) to afford 3-((3-aminopropanamido)methyl)-4-(((2S,3R,4S,5S,6R)-3,4,5-trihydroxy-6-(hydroxymethyl)tetrahydro-2H-pyran-2-yl)oxy)benzyl ((S)-1-(((S)-1-(((3R,4S,5S)-1-((S)-2-((1R,2R)-3-(((1S,2R)-1-hydroxy-1-phenylpropan-2-yl)amino)-1-methoxy-2-methyl-3-oxopropyl)pyrrolidin-1-yl)-3-methoxy-5-methyl-1-oxoheptan-4-yl)(methyl)amino)-3-methyl-1-oxobutan-2-yl)amino)-3-methyl-1-oxobutan-2-yl)(methyl)carbamate (**Intermediate 37, 250** mg, 89% yield over two steps). LCMS (3 min): 1.36 min; 1131.8 [M+H]+, 1153.4 [M+Na]+.

### Synthesis of LP5

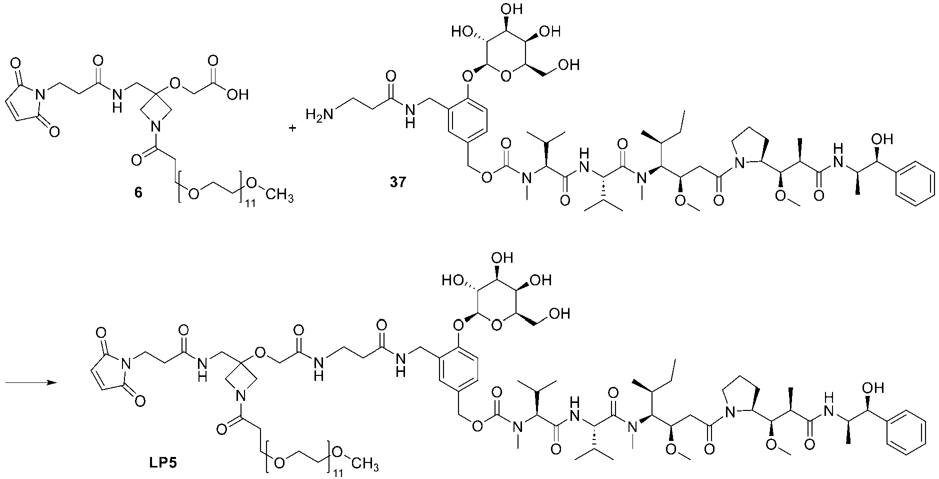

**Intermediate 37** (38.5 mg, 0.03 mmol) was dissolved in dry DMF (2 mL) followed by addition of **Intermediate 6** (15 mg, 0.02 mmol), 2-(3H-[1,2,3]triazolo[4,5-b]pyridin-3-yl)-1,1,3,3-tetramethylisouronium hexafluorophosphate(V) (9.7 mg, 0.03 mmol) and N-ethyl-N-isopropylpropan-2-amine (5.9 uL, 0.03 mmol). The reaction was stirred for 10 mins at 25°C and then concentrated. The residue was purified by HPLC preparative (0.1% formic acid H₂O : acetonitrile; from 10 to 50% over 10 mins) to afford 3-((3-(2-((3-((3-(2,5-dioxo-2,5-dihydro-1H-pyrrol-1-yl)propanamido)methyl)-1-(2,5,8,11,14,17,20,23,26,29,32,35-dodecaoxaoctatriacontan-38-oyl)azetidin-3-yl)oxy)acetamido)propanamido)methyl)-4-(((2S,3R,4S,5R,6R)-3,4,5-trihydroxy-6-(hydroxymethyl)tetrahydro-2H-pyran-2-yl)oxy)benzyl ((S)-1-(((S)-1-(((3R,4S,5S)-1-((S)-2-((1R,2R)-3-(((1S,2R)-1-hydroxy-1-phenylpropan-2-yl)amino)-1-methoxy-2-methyl-3-oxopropyl)pyrrolidin-1-yl)-3-methoxy-5-methyl-1-oxoheptan-4-yl)(methyl)amino)-3-methyl-1-oxobutan-2-yl)amino)-3-methyl-1-oxobutan-2-yl)(methyl)carbamate (**LP5**, 10.3 mg, 30% yield). LCMS (15 min): 6.40 min; 998.4 [M+2H]2+, 1993.8 [M+H]+. 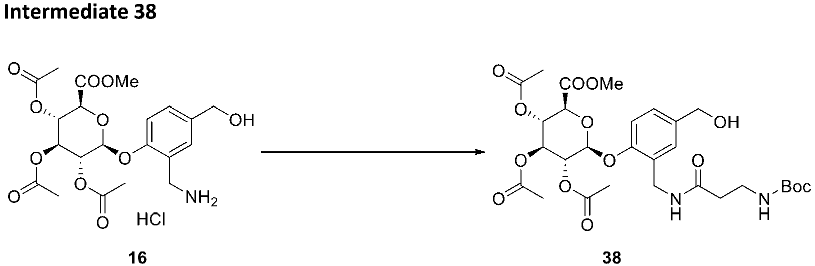 2,5-dioxopyrrolidin-1-yl 3-((tert-butoxycarbonyl)amino)propanoate (2.72 g, 9.49 mmol) was added to **Intermediate 16** (4 g, 7.91 mmol), DIEA (2.76 mL, 15.81 mmol) in DCM (40 mL) at 25°C under nitrogen. The resulting mixture was stirred at 25 °C for 4 hours. The reaction mixture was quenched with water (25 mL), extracted with EtOAc (3 x 50 mL), the organic layer was dried over Na2SO4, filtered and evaporated to afford pale yellow liquid. The crude product was purified by flash silica chromatography, elution gradient 0 to 40% THF in petroleum ether. Pure fractions were evaporated to dryness to afford (25,3R,45,55,65)-2-(2-((3-((tert-butoxycarbonyl)amino)propanamido)methyl)-4-(hydroxymethyl)phenoxy)-6-(methoxycarbonyl)tetrahydro-2H-pyran-3,4,5-triyl triacetate **(Intermediate 38,** 3.90 g, 77 %) as a pale yellow liquid. LCMS (3 min): 1.05 min; 641 [M+H]+.

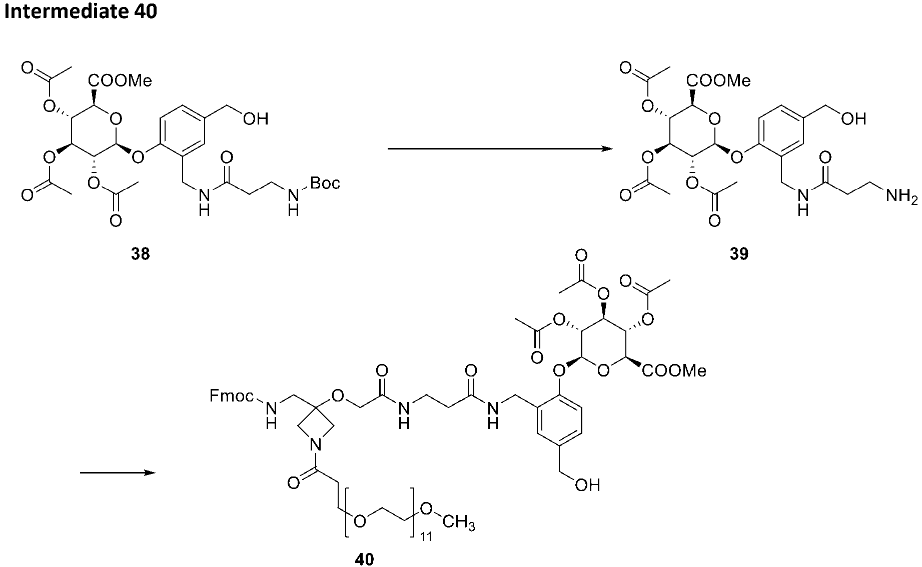

**Intermediate 38** (58 mg, 0.09 mmol) was stirred in TFA (1 mL) at 0 °C over a period of 2 hours to give (2S,3R,4S,5S,6S)-2-(2-((3-aminopropanamido)methyl)-4-(hydroxymethyl)phenoxy)-6-(methoxycarbonyl)tetrahydro-2H-pyran-3,4,5-triyl triacetate (**Intermediate 39**) that was used in next without further purification. To **Intermediate 5** (88 mg, 0.09 mmol) and HATU (52.8 mg, 0.14 mmol) in DMA (3 mL) was added DIEA (0.048 mL, 0.28 mmol) at 25°C over a period of 10 minutes. **Intermediate 39** (50 mg, 0.09 mmol) was added after the reaction had been stirred for 10 minutes The resulting mixture was stirred at 25 °C for 2 hours. The reaction solution was purified by flash C18-flash chromatography, elution gradient 0 to 100% MeCN in water(0.1% FA). Pure fractions were evaporated to dryness to afford (2S,3R,4S,5S,6S)-2-(2-((3-(2-((3-(((((9H-fluoren-9-yl)methoxy)carbonyl)amino)methyl)-1-(2,5,8,11,14,17,20,23,26,29,32,35-dodecaoxaoctatriacontan-38-oyl)azetidin-3-yl)oxy)acetamido)propanamido)methyl)-4-(hydroxymethyl)phenoxy)-6-(methoxycarbonyl)tetrahydro-2H-pyran-3,4,5-triyl triacetate (**Intermediate 40,** 1.600 g, 39.1 %) as a white solid.

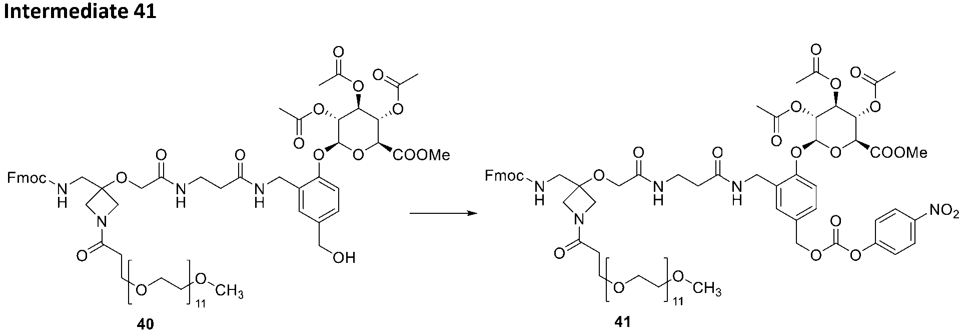

**Intermediate 40** (1.8 g, 1.22 mmol) and Bis(p-nitrophenyl) carbonate (1.484 g, 4.88 mmol) in MeCN (10 mL) was added DIEA (0.852 mL, 4.88 mmol). The resulting mixture was stirred at 25 °C for 2 hours. The crude product was purified by preparative HPLC (0.1%FA, from 60 to 90 % MeOH in water) as eluents. Fractions containing the desired compound were evaporated to dryness to afford (2S,3R,4S,5S,6S)-2-(2-((3-(2-((3-(((((9H-fluoren-9-yl)methoxy)carbonyl)amino)methyl)-1-(2,5,8,11,14,17,20,23,26,29,32,35-dodecaoxaoctatriacontan-38-oyl)azetidin-3-yl)oxy)acetamido)propanamido)methyl)-4-((((4-nitrophenoxy)carbonyl)oxy)methyl)phenoxy)-6-(methoxycarbonyl)tetrahydro-2H-pyran-3,4,5-triyl triacetate (**Intermediate 41,** 1.132 g, 56.6 %) as a yellow solid. LCMS (3 min): 1.42 min; 1640 [M+H]+

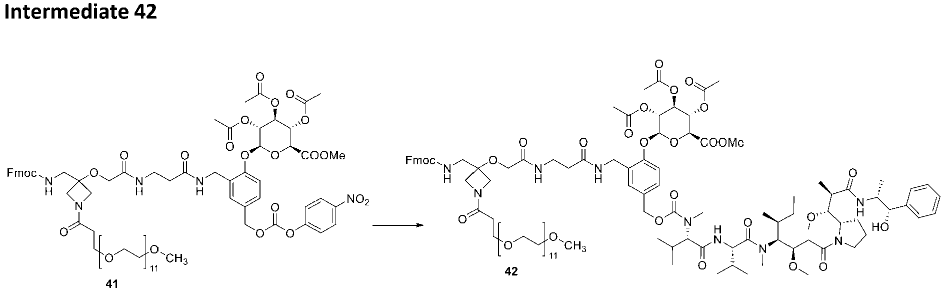

(S)-N-((3R,4S,5S)-1-((S)-2-((1R,2R)-3-(((1S,2R)-1-hydroxy-1-phenylpropan-2-yl)amino)-1-methoxy-2-methyl-3-oxopropyl)pyrrolidin-1-yl)-3-methoxy-5-methyl-1-oxoheptan-4-yl)-N,3-dimethyl-2-((S)-3-methyl-2-(methylamino)butanamido)butanamide (150 mg, 0.21 mmol), **Intermediate 41** (377 mg, 0.23 mmol) and 2-hydroxypyridine 1-oxide (25.5 mg, 0.23 mmol) were dissolved in DMF (5 mL) followed by addition of N-ethyl-N-isopropylpropan-2-amine (0.182 mL, 1.04 mmol). The reaction was left stirring at 25 °C for 4h and then is purified by reverse phase chromatography (0.1%FA, 30g, from 10 to 85% acetonitrile in 35CV to afford (2S,3R,4S,5S,6S)-2-(2-((3-(2-((3-(((((9H-fluoren-9-yl)methoxy)carbonyl)amino)methyl)-1-(2,5,8,11,14,17,20,23,26,29,32,35-dodecaoxaoctatriacontan-38-oyl)azetidin-3-yl)oxy)acetamido)propanamido)methyl)-4-((55,85,115,12R)-11-((S)-sec-butyl)-12-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-5,8-diisopropyl-4,10-dimethyl-3,6,9-trioxo-2,13-dioxa-4,7,10-triazatetradecyl)phenoxy)-6-(methoxycarbonyl)tetrahydro-2H-pyran-3,4,5-triyl triacetate **(Intermediate 42, 474** mg, quant.) LCMS (3 min): 1.98 min; 2219[M+H]+

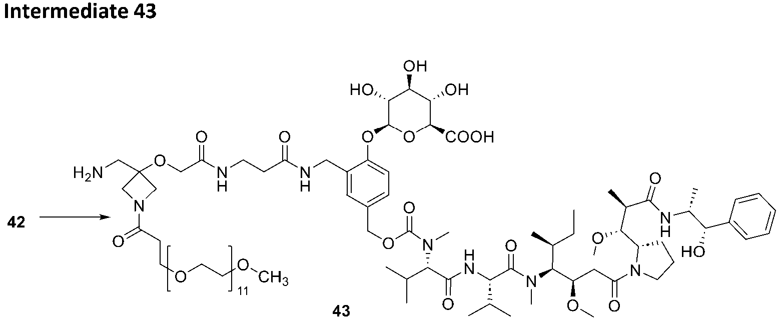

**Intermediate 42** (474 mg, 0.21 mmol) was dissolved in methanol (10 mL) followed by addition of a solution of potassium carbonate (0.243 mL, 4.27 mmol) in water (5 mL). After 3h stirring at 25 °C the reaction is purified by reverse phase chromatography (0.1%FA, 30g, from 5 to 35% in 35CV) to afford (2S,3S,4S,5R,6S)-6-(2-((3-(2-((3-(aminomethyl)-1-(2,5,8,11,14,17,20,23,26,29,32,35-dodecaoxaoctatriacontan-38-oyl)azetidin-3-yl)oxy)acetamido)propanamido)methyl)-4-((5S,8S,11S,12R)-11-((S)-sec-butyl)-12-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-5,8-diisopropyl-4,10-dimethyl-3,6,9-trioxo-2,13-dioxa-4,7,10-triazatetradecyl)phenoxy)-3,4,5-trihydroxytetrahydro-2H-pyran-2-carboxylic acid (**Intermediate 43,** 294 mg, 74 %)

### LP6

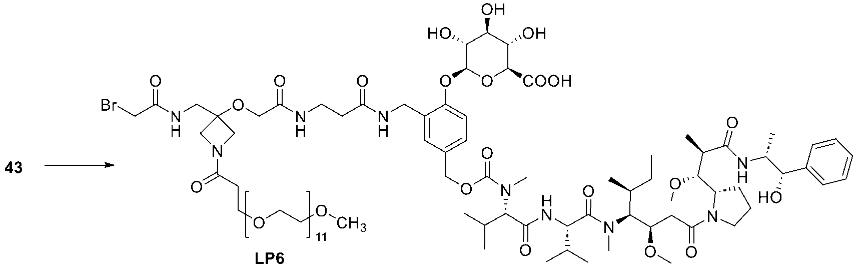

**Intermediate 43** (294 mg, 0.16 mmol) was dissolved in DMF (5 mL) followed by addition of 2,5-dioxopyrrolidin-1-yl 2-bromoacetate (44.8 mg, 0.19 mmol) and N-ethyl-N-isopropylpropan-2-amine (0.138 mL, 0.79 mmol). After 10 mins, the reaction was concentrated and purified by preparative HPLC (0.1%FA, from 15 to 60 in 15mins) to afford (2S,3S,4S,5R,6S)-6-(2-((3-(2-((3-((2-bromoacetamido)methyl)-1-(2,5,8,11,14,17,20,23,26,29,32,35-dodecaoxaoctatriacontan-38-oyl)azetidin-3-yl)oxy)acetamido)propanamido)methyl)-4-((55,85,115,12R)-11-((S)-sec-butyl)-12-(2-((S)-2-((1R,2R)-3-(((15,2R)-1-hydroxy-1-phenylpropan-2-yl)amino)-1-methoxy-2-methyl-3-oxopropyl)pyrrolidin-1-yl)-2-oxoethyl)-5,8-diisopropyl-4,10-dimethyl-3,6,9-trioxo-2,13-dioxa-4,7,10-triazatetradecyl)phenoxy)-3,4,5-trihydroxytetrahydro-2H-pyran-2-carboxylic acid (**LP6,** 150 mg, 80 %) LCMS (15 min): 6.71 min; 1978[M+2H]+

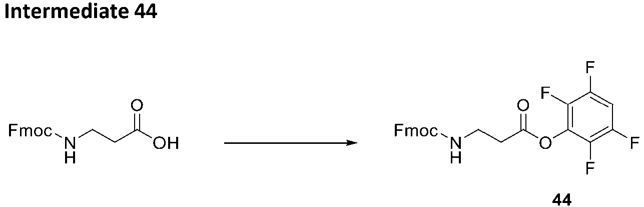

1-(3-Dimethylaminopropyl)-3-ethyl-carbodiimide (1.197 g, 7.71 mmol) was added to 2,3,5,6-tetrafluorophenol (1.280 g, 7.71 mmol) and 3-((((9H-fluoren-9-yl)methoxy)carbonyl)amino)propanoic acid (2g, 6.42 mmol) in DCM (20 mL) at 25°C. The resulting mixture was stirred at 25 °C for 2 hours. The reaction mixture was quenched with water (5 mL), extracted with DCM (3 x 5 mL), the organic layer was dried over Na₂SO₄, filtered and evaporated to afford 2,3,5,6-tetrafluorophenyl 3-((((9H-fluoren-9-yl)methoxy)carbonyl)amino)propanoate **(Intermediate 44,** 1.500 g, 50.8 %) as white solid. LCMS (3 mins): 1.35 min; 460 [M+H]+

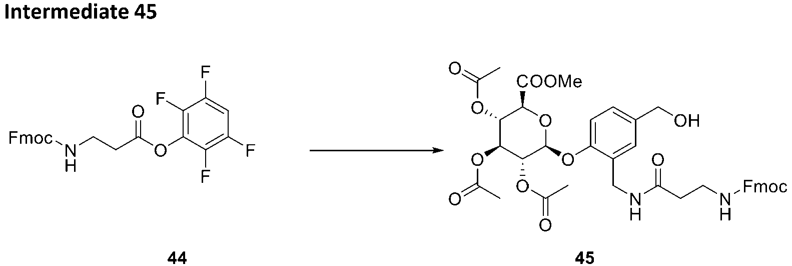

Sodium bicarbonate (1.097 g, 13.06 mmol) was added to **Intermediate 44** ( 2 g, 4.35 mmol) and **Intermediate 16** (1 g, 2.13 mmol) in THF (2 mL) and water (2 mL) at 25°C. The resulting mixture was stirred at 25 °C for 2 hours. The reaction mixture was acidified with 2M HCl. The solvent was removed under reduced pressure. The crude product was purified by flash C18-flash chromatography, elution gradient 0 to 50% MeCN in water. Pure fractions were evaporated to dryness to afford (2S,3R,4S,5S,6S)-2-(2-((3-((((9H-fluoren-9-yl)methoxy)carbonyl)amino)propanamido)methyl)-4-(hydroxymethyl)phenoxy)-6-(methoxycarbonyl)tetrahydro-2H-pyran-3,4,5-triyl triacetate (**Intermediate 45,** 2.20 g, 66.3 %) as a white solid. m/z (ES+), [M+H]+ = 763; base, HPLC tR = 1.167 min

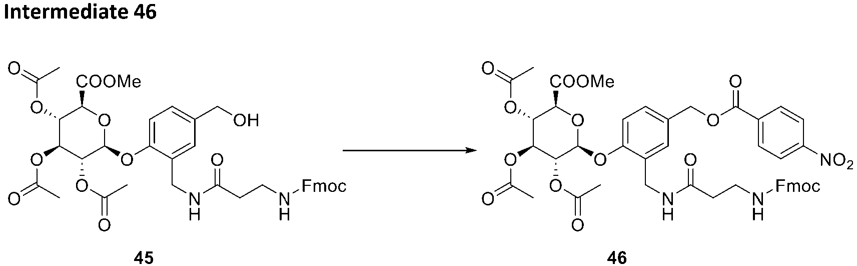

DIEA (0.412 mL, 2.36 mmol) was added to **Intermediate 45** (600mg, 0.79 mmol) and bis(4-nitrophenyl) carbonate (239 mg, 0.79 mmol) in THF (5 mL) at 25°C. The resulting mixture was stirred at 25 °C for 16 hours. The reaction mixture was quenched with water (5 mL), extracted with EtOAc (3 x 5 mL), the organic layer was dried over Na₂SO₄, filtered and evaporated to afford yellow solid. The crude product was purified by flash silica chromatography, elution gradient 0 to 70% EtOAc in petroleum ether. Pure fractions were evaporated to dryness to afford (2S,3R,4S,5S,6S)-2-(2-((3-((((9H-fluoren-9-yl)methoxy)carbonyl)amino)propanamido)methyl)-4-((((4-nitrophenoxy)carbonyl)oxy)methyl)phenoxy)-6-(methoxycarbonyl)tetrahydro-2H-pyran-3,4,5-triyl triacetate (**Intermediate 46, 590** mg, 81 %) as a white solid. m/z (ES+), [M+H]+ = 928; base, HPLC tR = 1.279 min

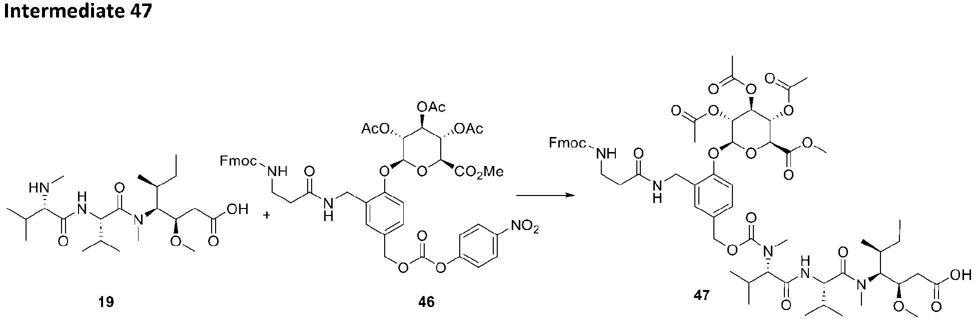

**Intermediate 46** (1.8 g, 1.94 mmol) was added to 2-hydroxypyridine-N-oxide (0.237 g, 2.13 mmol), DIEA (1.016 mL, 5.82 mmol) and **Intermediate 19** (0.887 g, 2.13 mmol) in DMA (20 mL) at 25°C under nitrogen. The resulting mixture was stirred at 25 °C for 1 hour. The solvent was removed under reduced pressure. The crude product was purified by preparative Column: Xselect CSH Prep C18 OBD Colum, 30*150nm, 5µm; Mobile Phase A: Water(0.1%FA), Mobile Phase B: ACN; Flow rate: 60 mL/min mL/min; Gradient: 5% B to 5% B in 2 min, 5% B to 55% B in 2.5 min, 55% B to 60% B in 10 min; Wave Length: 254nm/220nm nm; RT1(min): 11. Fractions containing the desired compound were evaporated to dryness to afford (55,85,115,12R)-1-(3-((3-((((9H-fluoren-9-yl)methoxy)carbonyl)amino)propanamido)methyl)-4-(((2S,3R,4S,5S,6S)-3,4,5-triacetoxy-6-(methoxycarbonyl)tetrahydro-2H-pyran-2-yl)oxy)phenyl)-11-((S)-sec-butyl)-5,8-diisopropyl-12-methoxy-4,10-dimethyl-3,6,9-trioxo-2-oxa-4,7,10-triazatetradecan-14-oic acid (**Intermediate 47,** 1.180 g, 50.5 %) as a white solid. m/z (ES+), [M+H]+ = 1204; acid, HPLC tR = 1.842 min

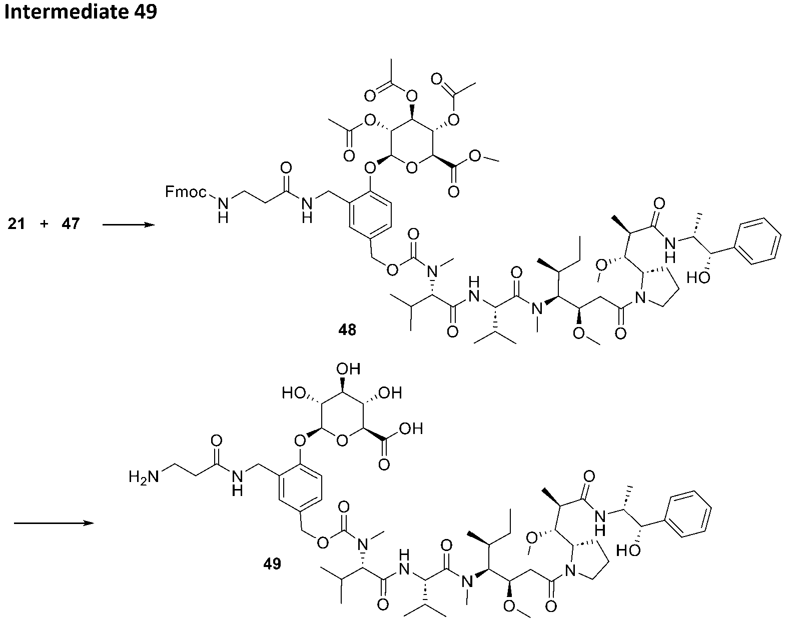

**Intermediate 21** (150 mg, 0.47 mmol) was dissolved in DMF (2 mL) and N-ethyl-N-isopropylpropan-2-amine (1 mL). A solution of **Intermediate 47** (175 mg, 0.15 mmol), 2-(3H-[1,2,3]triazolo[4,5-b]pyridin-3-yl)-1,1,3,3-tetramethylisouronium hexafluorophosphate(V) (148 mg, 0.39 mmol) and N-ethyl-N-isopropylpropan-2-amine (200 uL) in DMF (2 mL) was added to the previous reaction and was left stirring at 25 °C for 1h. The reaction was concentrated under reduced pressure and purified by column chromatography (dichloromethane : methanol, from 0 to 5%). **Intermediate 48** was dissolved in methanol (4 mL) followed by addition of potassium carbonate (150 mg) in water (1 mL). The reaction was left stirring at 25 °C overnight, then was concentrated under reduced pressure and redissolved in DMF (2 mL) and diethylamine (1 mL). After 10 mins, the reaction was purified by reverse-phase chromatography (0.1% formic acid, water:acetonitrile from 5 to 40%) to afford (2S,3S,4S,5R,6S)-6-(2-((3-aminopropanamido)methyl)-4-((5S,8S,11S,12R)-11-((S)-sec-butyl)-12-(2-((S)-2-((1R,2R)-3-(((15,2R)-1-hydroxy-1-phenylpropan-2-yl)amino)-1-methoxy-2-methyl-3-oxopropyl)pyrrolidin-1-yl)-2-oxoethyl)-5,8-diisopropyl-4,10-dimethyl-3,6,9-trioxo-2,13-dioxa-4,7,10-triazatetradecyl)phenoxy)-3,4,5-trihydroxytetrahydro-2H-pyran-2-carboxylic acid (**Intermediate 49,** 190 mg, 35% over 2 steps). LCMS (3 min): 1.43 min; 1145.4 [M+H]+

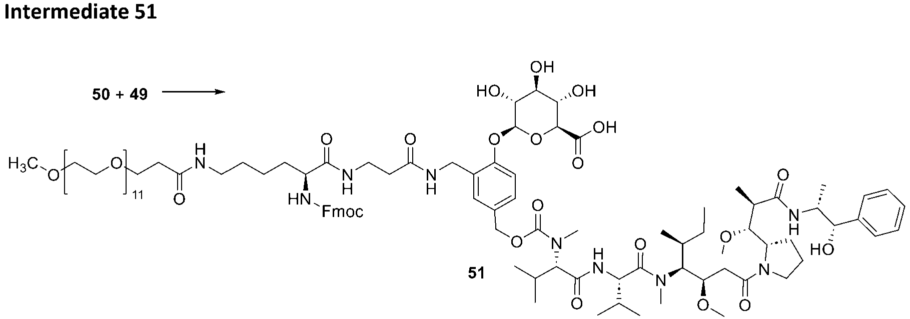

**Intermediate 50** was synthesized as previously described WO2022170002 A1. (S)-44-((((9H-fluoren-9-yl)methoxy)carbonyl)amino)-38-oxo-2,5,8,11,14,17,20,23,26,29,32,35-dodecaoxa-39-azapentatetracontan-45-oic acid (**50,** 234 mg, 0.25 mmol) was dissolved in DMF (2 mL) followed by addition of 2-(3H-[1,2,3]triazolo[4,5-b]pyridin-3-yl)-1,1,3,3-tetramethylisouronium hexafluorophosphate(V) (60 mg, 0.16 mmol) and N-ethyl-N-isopropylpropan-2-amine (87uL, 0.50 mmol). After 10 mins, **Intermediate 49** (190 mg, 0.17 mmol) was added and the reaction was stirred at 25 °C for 10 minutes. The crude reaction was purified by reverse-phase chromatography (0.1% formic acid, water : acetonitrile, from 5 to 75%) to afford (2S,3S,4S,5R,6S)-6-(2-((S)-44-((((9H-fluoren-9-yl)methoxy)carbonyl)amino)-38,45,49-trioxo-2,5,8,11,14,17,20,23,26,29,32,35-dodecaoxa-39,46,50-triazahenpentacontan-51-yl)-4-((5S,8S,11S,12R)-11-((S)-sec-butyl)-12-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-5,8-diisopropyl-4,10-dimethyl-3,6,9-trioxo-2,13-dioxa-4,7,10-triazatetradecyl)phenoxy)-3,4,5-trihydroxytetrahydro-2H-pyran-2-carboxylic acid (**Intermediate 51,** 110 mg, 32% yield). LCMS (3 min): 1.92 min; 2066.4 [M+H]+, 2088.8 [M+Na]+

### Synthesis LP7 (Reference)

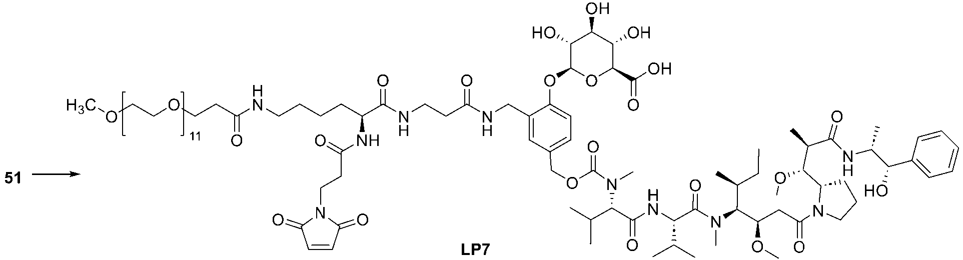

**Intermediate 51** (110 mg, 0.05 mmol) was dissolved in DMF (2 mL) and diethylamine (2 mL). After 10 mins, the reaction was concentrated under reduced pressured and thoroughly dried under high vacuum. The residue was dissolved in DMF (2 mL) followed by addition of 2,5-dioxopyrrolidin-1-yl 3-(2,5-dioxo-2,5-dihydro-1H-pyrrol-1-yl)propanoate (15 mg, 0.05 mmol). After 10 mins, the reaction was concentrated and purified by preparative HPLC (0.1% formic acid, water : acetonitrile from 10 to 40%) to afford (25,35,45,5R,65)-6-(4-((55,85,115,12R)-11-((S)-sec-butyl)-12-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-5,8-diisopropyl-4,10-dimethyl-3,6,9-trioxo-2,13-dioxa-4,7,10-triazatetradecyl)-2-((S)-44-(3-(2,5-dioxo-2,5-dihydro-1H-pyrrol-1-yl)propanamido)-38,45,49-trioxo-2,5,8,11,14,17,20,23,26,29,32,35-dodecaoxa-39,46,50-triazahenpentacontan-51-yl)phenoxy)-3,4,5-trihydroxytetrahydro-2H-pyran-2-carboxylic acid (**LP7,** 26.6 mg, 25% yield). LCMS (15 min): 6.70 min; 998.4 [M+2H]2+

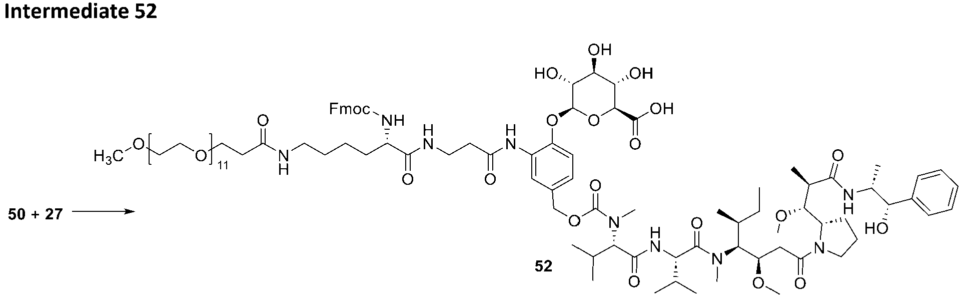

**Intermediate 50** (125 mg, 0.13 mmol) is dissolved in dry DMF (2 mL) followed by addition of 2-(3H-[1,2,3]triazolo[4,5-b]pyridin-3-yl)-1,1,3,3-tetramethylisouronium hexafluorophosphate(V) (47.9 mg, 0.13 mmol) and N-ethyl-N-isopropylpropan-2-amine (35 uL, 0.20 mmol). After 10 mins, **Intermediate 27** (75 mg, 0.07 mmol) was added. The reaction was stirred at room temperature for 10 mins then purified by reverse phase chromatography (0.1% formic acid, water: acetonitrile from 5 to 75%) to afford (2S,3S,4S,5R,6S)-6-(2-((S)-44-((((9H-fluoren-9-yl)methoxy)carbonyl)amino)-38,45-dioxo-2,5,8,11,14,17,20,23,26,29,32,35-dodecaoxa-39,46-diazanonatetracontan-49-amido)-4-((5S,8S,11S,12R)-11-((S)-sec-butyl)-12-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-5,8-diisopropyl-4,10-dimethyl-3,6,9-trioxo-2,13-dioxa-4,7,10-triazatetradecyl)phenoxy)-3,4,5-trihydroxytetrahydro-2H-pyran-2-carboxylic acid (**Intermediate 52, 20** mg, 11% yield). LCMS (15 min): 7.59 min; 2050.4 [M-H]-

### Synthesis LP8 (Reference)

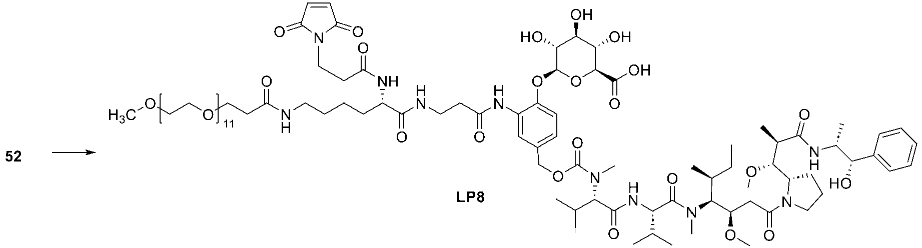

**Intermediate 52** (20 mg, 0.00975 mmol) was dissolved in DMF (2 mL) and diethylamine (2 mL). After 10 mins, the reaction was concentrated under reduced pressured and thoroughly dried under high vacuum. The crude was dissolved in dry DMF (5 mL) followed by addition of 2,5-dioxopyrrolidin-1-yl 3-(2,5-dioxo-2,5-dihydro-1H-pyrrol-1-yl)propanoate (3 mg, 0.010 mmol). After 10 mins, the reaction was concentrated and purified by preparative HPLC (0.1% formic acid, water : acetonitrile from 5 to 55%) to afford (25,35,45,5R,65)-6-(4-((55,85,115,12R)-11-((S)-sec-butyl)-12-(2-((S)-2-((1R,2R)-3-(((15,2R)-1-hydroxy-1-phenylpropan-2-yl)amino)-1-methoxy-2-methyl-3-oxopropyl)pyrrolidin-1-yl)-2-oxoethyl)-5,8-diisopropyl-4,10-dimethyl-3,6,9-trioxo-2,13-dioxa-4,7,10-triazatetradecyl)-2-((S)-44-(3-(2,5-dioxo-2,5-dihydro-1H-pyrrol-1-yl)propanamido)-38,45-dioxo-2,5,8,11,14,17,20,23,26,29,32,35-dodecaoxa-39,46-diazanonatetracontan-49-amido)phenoxy)-3,4,5-trihydroxytetrahydro-2H-pyran-2-carboxylic acid (**LP8**, 2.1 mg, 11% yield). LCMS (15 min): 6.68 min; 1980.7 [M]+

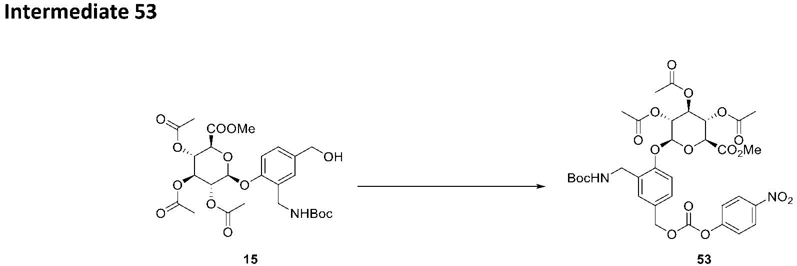

To **Intermediate 15** (300 mg, 0.53 mmol) in DMF (3 mL) was added bis(4-nitrophenyl) carbonate (176 mg, 0.58 mmol) and DIPEA (0.138 mL, 0.79 mmol). The reaction mixture was stirred at room temperature for 3 h. The reaction mixture was concentrated under reduced pressure to give a crude product. Purification by column chromatography (0-100% EtOAc in hexanes) gave (2S,3R,4S,5S,6S)-2-(2-(((tert-butoxycarbonyl)amino)methyl)-4-((((4-nitrophenoxy)carbonyl)oxy)methyl)phenoxy)-6-(methoxycarbonyl)tetrahydro-2H-pyran-3,4,5-triyl triacetate (**Intermediate 53, 266** mg, 68.7 %). LCMS (15 min): 1.99 min; 736.3 [M+H]+, 758.3 [M+Na]+

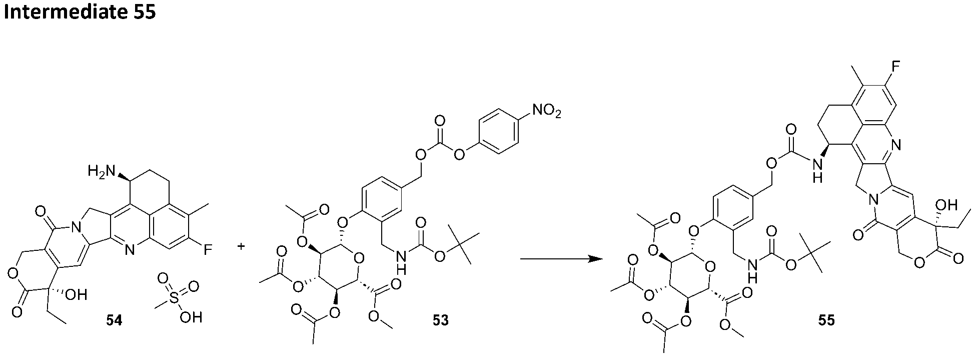

To **Intermediate 53** (266 mg, 0.36 mmol) in DMF (4 mL) was added (1S,9S)-1-amino-9-ethyl-5-fluoro-9-hydroxy-4-methyl-1,2,3,9,12,15-hexahydro-10H,13H-benzo[de]pyrano[3',4':6,7]indolizino[1,2-b]quinoline-10,13-dione methanesulfonate **(Intermediate 54, 183** mg, 0.34 mmol), DIPEA (0.189 mL, 1.09 mmol) and HOPO (0.036 mL, 0.36 mmol). The reaction mixture was stirred at room temperature overnight. The reaction mixture was purified by reverse phase chromatography (40-95% MeCN in water +0.1% FA). Fractions containing product were combined and concentratetd under reduced pressure. Water was added and extracted with EtOAc. The organic was washed with brine, dried (MgSO₄), filtered and concentrated under reduced pressure to give (2S,3R,4S,5S,6S)-2-(2-(((tert-butoxycarbonyl)amino)methyl)-4-(((((15,95)-9-ethyl-5-fluoro-9-hydroxy-4-methyl-10,13-dioxo-2,3,9,10,13,15-hexahydro-1H,12H-benzo[de]pyrano[3',4':6,7]indolizino[1,2-b]quinolin-1-yl)carbamoyl)oxy)methyl)phenoxy)-6-(methoxycarbonyl)tetrahydro-2H-pyran-3,4,5-triyl triacetate (**Intermediate 55, 302** mg, 81 %) as a yellow solid. LCMS (15 min): 7.84 min; 1032.5 [M+H]+

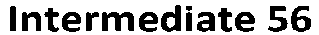

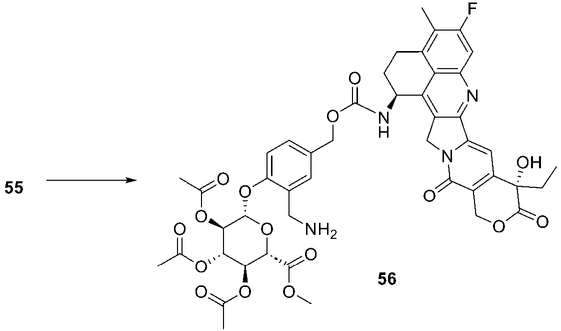

To **Intermediate 55** (302 mg, 0.29 mmol) in DCM (2 mL) was added TFA (2 mL). The reaction mixture was stirred at room temperature for 2 h. The reaction mixture was concentrated under reduced pressure to give a crude product which was used without further purification. LCMS (3 min): 1.41 min; 932.7 [M+H]+

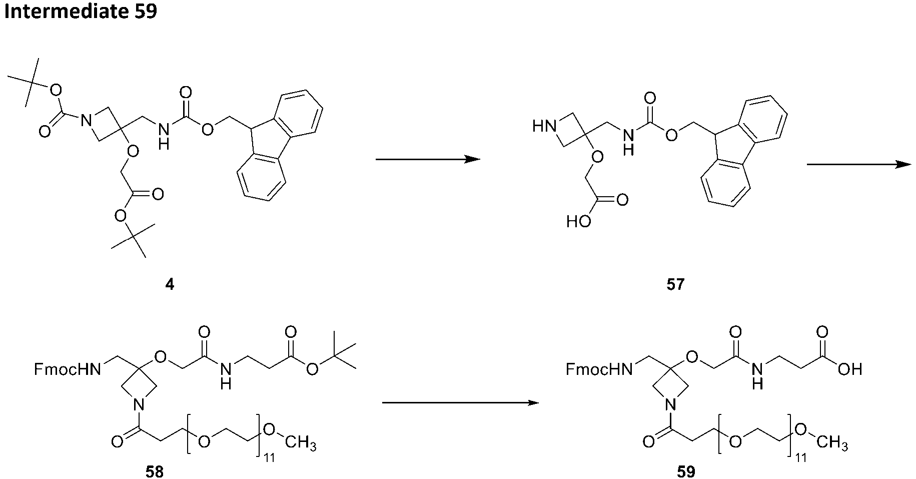

### Step 1

To **Intermediate** 4 (330 mg, 0.61 mmol) in DCM (3 mL) was added TFA (3 mL). The reaction mixture was stirred at room temperature for 2 h. The reaction mixture was concentrated under reduced pressure to give a crude product (intermediate 57) which was used without purification. LCMS (3 min): 1.28 min; 384.1 [M+H]+

### Step 2

To crude **Intermediate 57** (233 mg, .61 mmol) in DMF (6 mL) was added 1-[(38-oxo-2,5,8,11,14,17,20,23,26,29,32,35-dodecaoxaoctatriacontan-38-yl)oxy]-2,5-pyrrolidinedione (439 mg, 0.64 mmol) and DIPEA (0.638 mL, 3.66 mmol). The reaction mixture was stirred at room temperature for 1 h. tert-butyl 3-aminopropanoate hydrochloride (222 mg, 1.22 mmol) and HATU (464 mg, 1.22 mmol) were added and the reaction mixture stirred at room temperature for 30 min. The reaction mixture was purified by reverse phase chromatography (40-60% MeCN in water +0.1% FA) to give tert-butyl 3-(2-((3-(((((9H-fluoren-9-yl)methoxy)carbonyl)amino)methyl)-1-(2,5,8,11,14,17,20,23,26,29,32,35-dodecaoxaoctatriacontan-38-oyl)azetidin-3-yl)oxy)acetamido)propanoate (**Intermediate 58,** 545 mg, 83 %) as a colourless oil. LCMS (15 min): 7.19 min; 1082.0 [M+H]+, 1103.7 [M+Na]+

### Step 3

To **Intermediate 58** (545 mg, 0.50 mmol) in DCM (2 mL) was added TFA (2 mL). The reaction mixture was stirred at room temperature for 2 h. The reaction mixture was concentrated under reduced pressure to give a crude product (**Intermediate 59**) which was used without further purification. LCMS (3 min): 1.63 min; 1025.7 [M+H]+

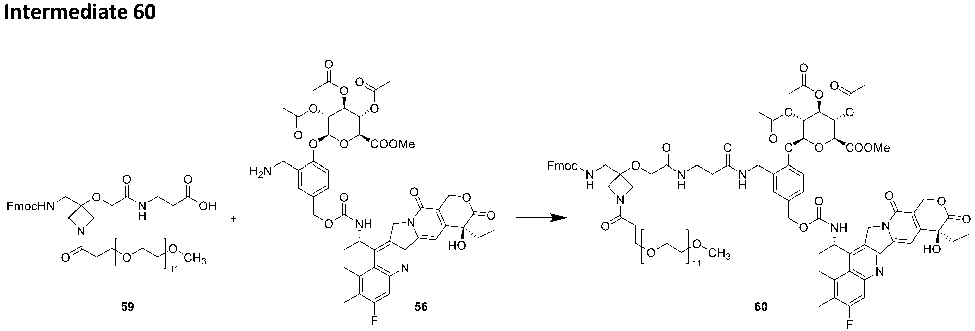

To **Intermediate 59** (327 mg, 0.32 mmol) and **Intermediate 56** (270 mg, .29 mmol) in DMF (3.2 mL) was added HATU (132 mg, 0.35 mmol) and DIPEA (0.303 mL, 1.74 mmol). The reaction mixture was stirred at room temperature for 2 h. The reaction mixture was purified by reverse phase chromatography ( from 15 to 50 % MeCN in water) to give (2S,3R,4S,5S,6S)-2-(2-((3-(2-((3-(((((9H-fluoren-9-yl)methoxy)carbonyl)amino)methyl)-1-(2,5,8,11,14,17,20,23,26,29,32,35-dodecaoxaoctatriacontan-38-oyl)azetidin-3-yl)oxy)acetamido)propanamido)methyl)-4-(((((1S,9S)-9-ethyl-5-fluoro-9-hydroxy-4-methyl-10,13-dioxo-2,3,9,10,13,15-hexahydro-1H,12H-benzo[de]pyrano[3',4':6,7]indolizino[1,2-b]quinolin-1-yl)carbamoyl)oxy)methyl)phenoxy)-6-(methoxycarbonyl)tetrahydro-2H-pyran-3,4,5-triyl triacetate (**Intermediate 60,** 334 mg, 59.5 %) as a white solid. LCMS (15 min): 7.56 min; 1938.1 [M+H]+

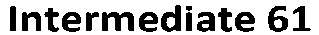

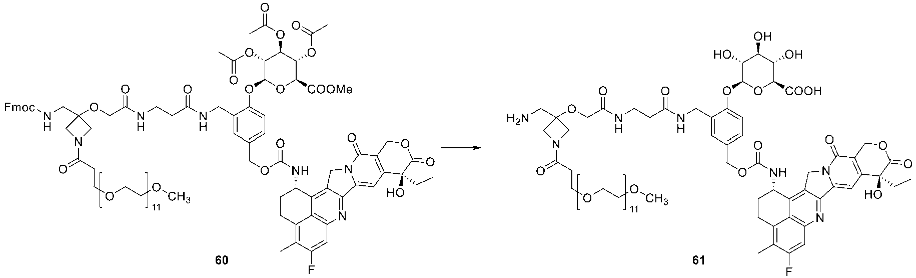

To **Intermediate 60** (334 mg, 0.17 mmol) in methanol (4 mL) and water (2 mL) was added potassium carbonate (98 µl, 1.72 mmol). The reaction mixture was stirred at room temperature for 4 h. acetic acid (200 µl, 3.49 mmol) was added and the reaction mixture purified by reverse phase chromatography to give (2S,3S,4S,5R,6S)-6-(2-((3-(2-((3-(aminomethyl)-1-(2,5,8,11,14,17,20,23,26,29,32,35-dodecaoxaoctatriacontan-38-oyl)azetidin-3-yl)oxy)acetamido)propanamido)methyl)-4-(((((1S,9S)-9-ethyl-5-fluoro-9-hydroxy-4-methyl-10,13-dioxo-2,3,9,10,13,15-hexahydro-1H,12H-benzo[de]pyrano[3',4':6,7]indolizino[1,2-b]quinolin-1-yl)carbamoyl)oxy)methyl)phenoxy)-3,4,5-trihydroxytetrahydro-2H-pyran-2-carboxylic acid (**Intermediate 61,** 120 mg, 44.2 %) as a white solid. LCMS (15 min): 5.06 min; 1576.1 [M+H]+

### LP9

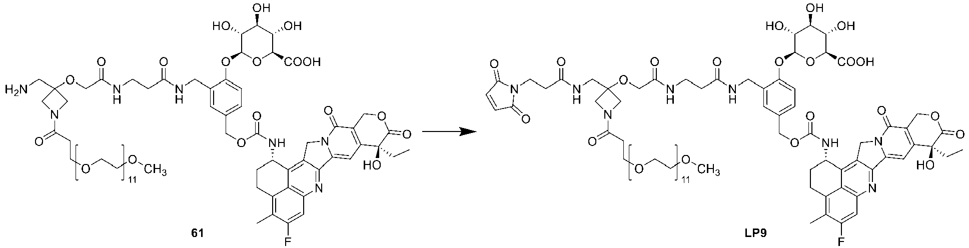

To **Intermediate 61** (120 mg, 0.08 mmol) in DMF (3 mL) was added 2,5-dioxopyrrolidin-1-yl 3-(2,5-dioxo-2,5-dihydro-1H-pyrrol-1-yl)propanoate (60.9 mg, 0.23 mmol) and DIPEA (0.080 mL, 0.46 mmol). The reaction mixture was stirred at room temperature for 2 h. The reaction mixture was purified by repeat reverse phase chromatography to give (2S,3S,4S,5R,6S)-6-(2-((3-(2-((3-((3-(2,5-dioxo-2,5-dihydro-1H-pyrrol-1-yl)propanamido)methyl)-1-(2,5,8,11,14,17,20,23,26,29,32,35-dodecaoxaoctatriacontan-38-oyl)azetidin-3-yl)oxy)acetamido)propanamido)methyl)-4-(((((1S,9S)-9-ethyl-5-fluoro-9-hydroxy-4-methyl-10,13-dioxo-2,3,9,10,13,15-hexahydro-1H,12H-benzo[de]pyrano[3',4':6,7]indolizino[1,2-b]quinolin-1-yl)carbamoyl)oxy)methyl)phenoxy)-3,4,5-trihydroxytetrahydro-2H-pyran-2-carboxylic acid (**LP9,** 39.0 mg, 29.7 %) as a white solid. LCMS (15 min): rt = 5.93 min; 1727.0 [M+H]+, 864.1 [M+2H]+, 576.4 [M+3H]+

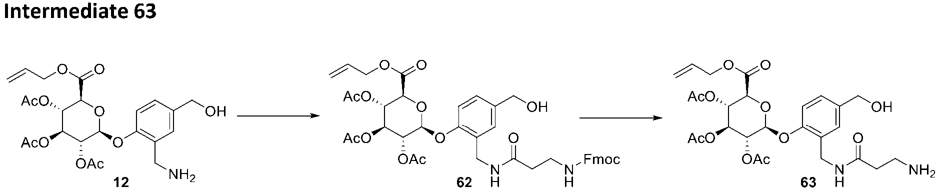

### Step 1

To a stirred reactor containing **Intermediate 12** (2.1 kg, 90.5% w/w, 3.57 mol) and acetonitrile (19 L) was added Fmoc-β-alanine (1.11 kg, 3.57 mol). The stirred mixture was cooled to 0°C. To this was added hexafluorophosphate azabenzotriazole tetramethyl uronium (1.36 kg, 3.57 mol) and N,N-diispropylethylamine (0.92 kg, 7.14 mol), and stirred for 4 hours, maintaining the temperature at 0°C. Water (19 L) and ethyl acetate (19 L) was added to the stirred mixture. The organic phase was separated and concentrated to ~19 L under vacuum. Ethyl acetate (28.5 L) was added to the concentrated solution and stirred at 20-25°C for 18 hours. The resulting suspension was filtered , the cake washed with ethyl acetate (3.87 L), and dried under vacuum to (2S,3R,4S,5S,6S)-2-(2-((3-((((9H-fluoren-9-yl)methoxy)carbonyl)amino)propanamido)methyl)-4-(hydroxymethyl)phenoxy)-6-((allyloxy)carbonyl)tetrahydro-2H-pyran-3,4,5-triyl triacetate (**Intermediate 62,** 1.6 kg, 99% w/w, 56%). LCMS m/z 789 [M+H]⁺

### Step 2

To a stirred reactor containing **Intermediate 62** (1 kg, 1.27 mol) and tetrahydrofuran (10 L) at -45°C under nitrogen was added 2,3,4,6,7,8,9,10-octahydropyrimido[1,2-a]azepine (385.98 g, 2.54 mol). The mixture was stirred at -45°C for four hours then diluted with acetonitrile (5 L) and quenched by the addition of hydrogen chloride in tert-butyl methyl ether solution (2.54 L, 2.0 M, 5.07 mol). The mixture was concentrated to ~5 L under vacuum, and diluted with n-heptane (5 L). The acetonitrile layer was collected containing (2S,3S,4S,5R,6S)-2-((allyloxy)carbonyl)-6-(2-((3-aminopropanamido)methyl)-4-(hydroxymethyl)phenoxy)tetrahydro-2H-pyran-3,4,5-triyl triacetate **(Intermediate 63,** 3.88 kg of MeCN solution, 89.97% area, assumed 100%). LCMS m/z 566.6 [M+H]⁺

### Alternative synthesis of LP2

### General Information for Alternative Synthesis of LP2

Flash chromatography was performed using a BIOTAGE ISOLERA and fractions checked for purity using thin-layer chromatography (TLC). TLC was performed using MERCK KIESELGEL 60 F254 silica gel, with fluorescent indicator on aluminium plates. Visualisation of TLC was achieved with UV light.

Extraction and chromatography solvents were bought and used without further purification from VWR U.K.

All fine chemicals were purchased from SIGMA-ALDRICH unless otherwise stated.

Pegylated reagents were obtained from QUANTA BIODESIGN US via STRATECH UK.

### LC/MS conditions for Alternative Synthesis of LP2

Positive mode electrospray mass spectrometry was performed using a WATERS ACQUITY H-CLASS SQD2 using one of the following methods.

The HPLC (WATERS ALLIANCE 2695) was run using a mobile phase of water (A) (TFA 0.03%) and acetonitrile (B) (TFA 0.03%).

LCMS 16.0 min: Initial composition 5% B increased to 25% B over a 3 minute period. The composition was then increased to 30% B over a 5 minute period, followed by increasing to 95% B over a 4 minute period. The composition was held for a minute at 95% B, then returned to 5% B in 10 seconds and held there for 2 minutes 50 seconds. The total duration of the gradient run was 16.0 minutes. Flow rate was 0.40 mL/minute. Wavelength detection range: 210nm. Columns: WATERS ACQUITY UPLC BEH C18 1.7um, 2.1*100mm at 40 °C.

LCMS 16.5 min: Initial composition 10% B held over 25 seconds, then increased from 5% B to 90% B over a 11 minute 35 seconds' period. The composition was held for 70 seconds at 90% B, then returned to 5% B in 10 seconds and held there for 3 minutes. The total duration of the gradient run was 16.5 minutes. Flow rate was 0.40 mL/minute. Wavelength detection range: 210 to 220 nm. Columns: WATERS ACQUITY UPLC BEH C18 1.7um, 2.1*100mm at 40 °C.

LCMS 26.0 min: Initial composition 10% B increased to 30% B over a 5 minute period, followed by increasing to 35% B over a 15 minute period. The composition was increased to 90% B over a 2 minute period, held for 1 minute at 90% B, then returned to 10% B in 10 seconds and held there for 2 minutes and 50 seconds. The total duration of the gradient run was 26.0 minutes. Flow rate was 0.40 mL/minute. Wavelength detection range: 210 nm. Columns: WATERS ACQUITY UPLC BEH C18 1.7um, 2.1*100mm at 40 °C.

LCMS 30.0 min: Initial composition 10% B increased from 45% B over a 5 minute period. The composition was increased to 47% B over 15 minutes, then increased to 95% B over 5 minutes. The composition was held at 95% B for 2 minutes, then returned to 10% B in 10 seconds and held there for 2 minutes 50 seconds. The total duration of the gradient run was 30 minutes. Flow rate was 0.40 mL/minute. Wavelength detection range: 210 nm. Columns: WATERS ACQUITY UPLC BEH C18 1.7um, 2.1*100mm at 55 °C.

LCMS 31.0 min: Initial composition 10% B increased from 45% B over a 5 minute period. The composition was increased to 50% B over 20 minutes, then increased to 90% B over 2 minutes. The composition was held at 90% B for 1 minute, then returned to 10% B in 10 seconds and held there for 2 minutes 50 seconds. The total duration of the gradient run was 31 minutes. Flow rate was 0.40 mL/minute. Wavelength detection range: 210 nm. Columns: WATERS ACQUITY UPLC BEH C18 1.7um, 2.1*100mm at 40 °C.

LCMS 31.0 min: Initial composition 10% B increased from 45% B over a 20 minute period. The composition was increased to 95% B over 5 minutes, held at 95% B for 2 minute, then decreased to 5% B in 10 seconds and held there for 2 minutes 50 seconds. The total duration of the gradient run was 31 minutes. Flow rate was 0.40 mL/minute. Wavelength detection range: 210 nm. Columns: WATERS ACQUITY UPLC CSH Phenyl-Hexyl 1.7um, 2.1*100mm at 40 °C.

### NMR Method for Alternative Synthesis of LP2

Proton NMR chemical shift values were measured on the delta scale at 300 MHz or 400 MHz using a BRUKER AV400. The following abbreviations have been used: s, singlet; d, doublet; t, triplet; q, quartet; quin, quintet; m, multiplet; br, broad. Coupling constants are reported in Hz.

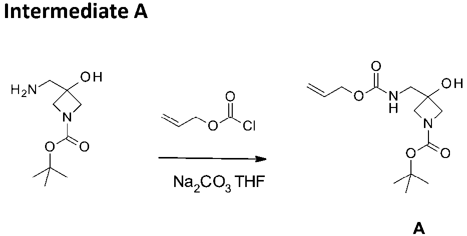

To a stirred reactor containing tert-butyl 3-(aminomethyl)-3-hydroxy-azetidine-1-carboxylate (290 g, 1.0eq, 1.48 mol) and allyl chloroformate (214 g, 1.2 eq, 1.78 mmol) in tetrahydrofuran (2.90 L) was added sodium carbonate (234 g, 1.5 eq, 2.22 mol). The contents of the reactor was stirred at 25°C for 2 hours. Water (2.90 L) was added to the reactor, and the resulting mixture was extracted twice with ethylacetate (2.90 L X 2). The combined organic layer was washed with brine ( 2.90 L) and then concentrated under reduced pressure down to a volume of 15 mL. MTBE (25 mL) was added and then the reaction was concentrated to 15 mL. Further MTBE (1.45 L) was added and the reaction was again concentrated down to 15 mL. MTBE (1.45 L) was added and the mixture stirred for 4 hours at 25°C. The solid formed was collected by filtration, washed with MTBE (580 mL) and dried under vacuum at 45°C to give tert-butyl 3-[(allyloxycarbonylamino)methyl]-3-hydroxy-azetidine-1-carboxylate, **Intermediate A** as a solid (301 g, 98.9 w/w %, 70.1% yield); ¹H NMR (500 MHz, DMSO-d6) δ ppm 1.37 (s, 9 H) 3.15 (d, J=6.10 Hz, 2 H) 3.50 - 3.65 (m, 2 H) 3.81 (br d, J=8.54 Hz, 2 H) 4.48 (br d, J=5.19 Hz, 2 H) 5.16 (dd, J=10.38, 1.53 Hz, 1 H) 5.27 (dd, J=17.24, 1.68 Hz, 1 H) 5.75 (s, 1 H) 5.90 (ddt, J=17.17, 10.60, 5.19, 5.19 Hz, 1 H) 7.40 (t, J=6.26 Hz, 1 H) LCMS (ESI) m/z 287 (M + H)+.

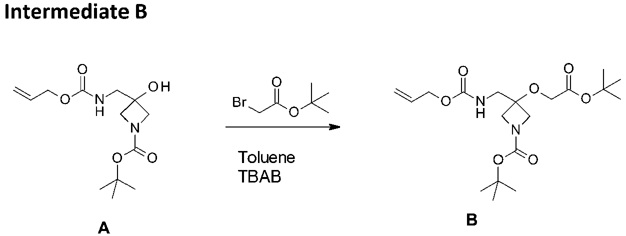

To a stirred reactor containing **Intermediate A** (100.0 g, 0.35 mol, 1.0 eq) added TBAB (20.0 g, 0.06 mol, 20 w/w%), and NaOH (15.37g, 0.38 mol, 1.1 eq) in toluene (1 L, 10 V) at 5°C was added *tert-*butyl bromoacetate (81.7 g, 0.42 mol, 1.2 eq.) dropwise. The reaction mixture was stirred at 25°C for 16 hours. Water (500 mL, 5 V) was added to the reactor and stirred for 5 min The layers were allowed to separate and the aqueous layer discarded. The organic layer was washed with brine twice ( 2 X 5 V, 10%) and then concentrated to dryness. MTBE (200 mL, 2 V) was added to the residue and stirred. N-heptane (300mL, 3V) was added and the reaction mixture was cooled to 5°C and stirred for 6 h. The resulting solid was filtered, washed with MTBE:heptane ( 80 mL:120 mL, 2:3, 2 V) and dried to a constant weight under vacuum at 45°C to give *tert*-butyl 3-[(allyloxycarbonylamino)methyl]-3-(2-tert-butoxy-2-oxo-ethoxy)azetidine-1-carboxylate, **Intermediate B** as a solid (100 g, 98.3 w/w%, 71.0% yield); ¹H NMR (500 MHz, CHLOROFORM-d) δ ppm 1.44 (s, 9 H) 1.47 - 1.50 (m, 9 H) 3.51 (br d, J=4.88 Hz, 2 H) 3.78 (br d, J=9.46 Hz, 2 H) 3.85 (d, J=9.77 Hz, 2 H) 3.98 (s, 2 H) 4.58 (br d, J=5.49 Hz, 2 H) 5.22 (br d, J=10.38 Hz, 1 H) 5.31 (dd, J=17.09, 1.53 Hz, 1 H) 5.74 (br s, 1 H) 5.93 (ddt, J=16.86, 10.91, 5.49, 5.49 Hz, 1 H); LCMS (ESI) m/z 401 (M + H)⁺.

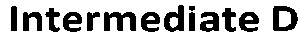

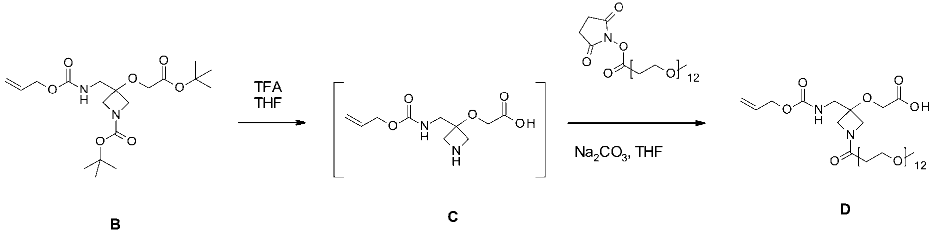

To a stirred solution of **Intermediate B** (211.3 g, 0.53 mol, 1.0 eq) in THF (1.69 L, 8 V) was added TFA (1.69L, 8.0 V). The reaction mixture was stirred at 50°C for 24 h. The reaction mixture was concentrated to dryness to give 2-[3-[(allyloxycarbonylamino)methyl]azetidin-3-yl]oxyacetic acid (215.1 g, 54.1 w/w %, 90.3% yield) which was used directly in the next step without purification.

To a stirred solution of 2-[3-[(allyloxycarbonylamino)methyl]azetidin-3-yl]oxyacetic acid (203.3 g, 54.1 w/w %) in THF (1.1 L, 10 V) at 5°C was added MPEG11-NHS (308.9 g, 0.45 mol, 1.0 eq) and Na₂CO₃ (143.2 g, 1.35 mol, 3.0eq). The reaction mixture was stirred for 2 h at 25°C. The solvent was removed under vacuum and water (1.1 L, 10 V) and MTBE (1.1 L, 10 V) were added to the residue. The organic layer was separated and discarded. The aqueous layer was washed twice with MTBE (1.1 L × 2, 10 V × 2); Note product in the aqueous phase. The pH of the aqueous phase was adjusted to 3-4 by the addition of HCl (1 M, 66 mL, ~0.6 V) The aqueous phase was extracted four times with EtOAc (1.1 L × 4, 10 V × 4); Note product now in organic phase. The combined organic layers were concentrated to dryness under vacuum to give 2-[3-[(allyloxycarbonylamino)methyl]-1-[3-[2-[2-[2-[2-[2-[2-[2-[2-[2-[2-(2-methoxyethoxy)ethoxy]ethoxy]ethoxy]ethoxy]ethoxy]ethoxy]ethoxy]ethoxy]ethoxy]ethoxy]propano yl]azetidin-3-yl]oxyacetic acid, **Intermediate D** as an oil (349.1 g, 80.0 w/w%, 76.1% yield); ¹H NMR (500 MHz, CHLOROFORM-d) δ ppm 2.29 - 2.41 (m, 2 H) 3.34 (s, 3 H) 3.41 (s, 1 H) 3.41 - 3.63 (m, 44 H) 3.64 - 3.77 (m, 4 H) 3.82 (br d, J=10.99 Hz, 1 H) 3.92 (br d, J=10.99 Hz, 1 H) 4.01 - 4.17 (m, 4 H) 4.52 (br d, J=5.49 Hz, 2 H) 5.17 (br d, J=10.38 Hz, 1 H) 5.22 - 5.29 (m, 1 H) 5.87 (ddt, J=16.78, 10.91, 5.53, 5.53 Hz, 1 H) 6.05 (br t, J=5.80 Hz, 1 H); LCMS (ESI) m/z 815.1 (M + H)⁺.

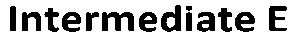

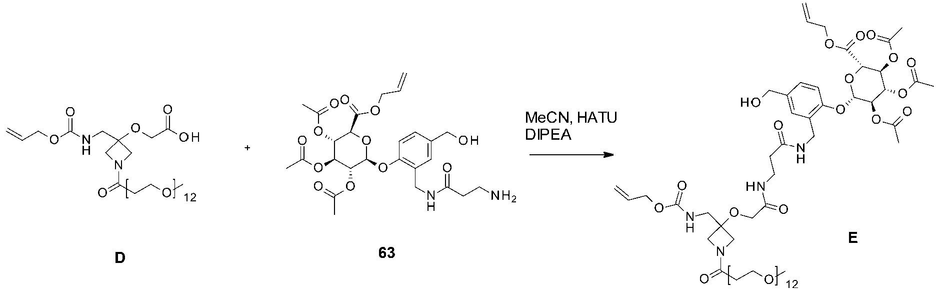

To a stirred solution of **Intermediate D** (62.5 g, 80.0 w/w%, 0.06 mol, 1.0 eq) and **Intermediate 63** (566.6 g, 0.06 mol, 1.05 eq) in MeCN (500 mL, 10 V) at 0°C was added HATU (34.2 g, 0.09 mol, 1.5 eq) and DIPEA (23.3 g, 0.18 mol, 3.0 eq). The reaction mixture was stirred at 0°C for 30 min, then water (500 mL, 10 V) was charged. The layers were separated and both retained. The aqueous phase was extracted twice with DCM (500 mL X 2, 10 V X 2). The combined organic layers were washed twice with brine (500 mL X 2, 10 V X 2) and then concentrated to dryness under vacuum at 30-40°C to give allyl (25,35,45,5R,65)-3,4,5-triacetoxy-6-[2-[[3-[[2-[3-[(allyloxycarbonylamino)methyl]-1-[3-[2-[2-[2-[2-[2-[2-[2-[2-[2-[2-(2-methoxyethoxy)ethoxy]ethoxy]ethoxy]ethoxy]ethoxy]ethoxy]ethoxy]ethoxy]ethoxy]ethoxy]propano yl]azetidin-3-yl]oxyacetyl]amino]propanoylamino]methyl]-4-(hydroxymethyl)phenoxy]tetrahydropyran-2-carboxylate, **Intermediate E** as an oil (56.3 g, 81.2 w/w%, 54.6% yield); ¹H NMR (500 MHz, DMSO-d6) 1.21 - 1.31 (m, 1 H) 1.99 (d, J=7.32 Hz, 6 H) 2.05 (s, 3 H) 2.29 (t, J=6.41 Hz, 2 H) 2.35 - 2.44 (m, 2 H) 3.24 (s, 3 H) 3.27 - 3.49 (m, 41 H) 3.56 - 3.66 (m, 2 H) 3.71 - 3.81 (m, 2 H) 3.87 - 4.00 (m, 3 H) 4.05 - 4.12 (m, 2 H) 4.18 - 4.25 (m, 1 H) 4.41 (d, J=4.88 Hz, 2 H) 4.47 - 4.56 (m, 3 H) 4.60 - 4.68 (m, 1 H) 4.76 (d, J=10.07 Hz, 1 H) 5.08 - 5.19 (m, 4 H) 5.23 - 5.35 (m, 3 H) 5.48 (t, J=9.61 Hz, 1 H) 5.56 (d, J=7.93 Hz, 1 H) 5.84 - 5.95 (m, 2 H) 7.01 (d, J=8.54 Hz, 1 H) 7.12 (s, 1 H) 7.17 (d, J=8.07 Hz, 1 H) 7.51 (br t, J=6.10 Hz, 1 H) 7.91 (br t, J=5.65 Hz, 1 H) 8.24 (t, J=5.95 Hz, 1 H); LCMS (ESI) m/z 682.1 (M + 2H)²⁺.

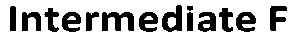

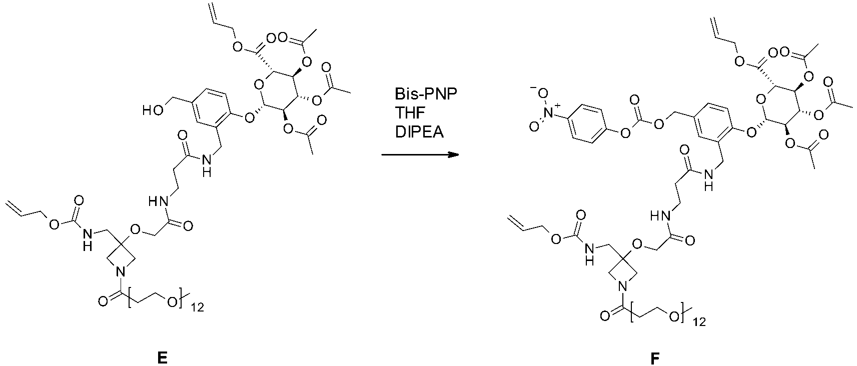

To a stirred solution of **Intermediate E** (40 g, 81.2 w/w%, 24 mmol, 1.0 eq) in THF (488 mL, 15 V) at 25°C was added Bis-PNP (25.6 g, 84 mmol, 3.5 eq) and DIPEA ( 7.1 g, 55 mmol, 2.3 eq)). The reaction mixture was stirred at 25°C for 16 h. The reaction was cooled to 10°C then NaCl (15% aq; 325 mL, 10 V) was charged. The pH of the reaction mixture was adjusted to pH 3-6 by the addition of HCl (1M, 19.5 mL, 0.6 V) at 10°C. The layers were allowed to separate and the aqueous layer discarded. The organic layer was concentrated to 10 V and then MTBE (1.63 L, 50 V) was added dropwise. The resulting oil was collected to give allyl ((25,35,45,5R,65)-3,4,5-triacetoxy-6-[2-[[3-[[2-[3-[(allyloxycarbonylamino)methyl]-1-[3-[2-[2-[2-[2-[2-[2-[2-[2-[2-[2-(2-methoxyethoxy)ethoxy]ethoxy]ethoxy]ethoxy]ethoxy]ethoxy]ethoxy]ethoxy]ethoxy]ethoxy]propano yl]azetidin-3-yl]oxyacetyl]amino]propanoylamino]methyl]-4-[(4-nitrophenoxy)carbonyloxymethyl]phenoxy]tetrahydropyran-2-carboxylate, **Intermediate F,** as an oil (40.8 g, 76.0 w/w%, 85.2% yield; ¹H NMR (300 MHz, DMSO-d6) δ ppm) 1.96 - 2.09 (m, 9 H) 2.21 - 2.35 (m, 2 H) 2.36 - 2.47 (m, 2 H) 3.24 (s, 3 H) 3.25 - 3.50 (m, 43 H) 3.55 - 4.00 (m, 8 H) 4.05 - 4.15 (m, 2 H) 4.16 - 4.29 (m, 1 H) 4.44 - 4.67 (m, 4 H) 4.80 (d, J=9.96 Hz, 1 H) 5.08 - 5.37 (m, 8 H) 5.44 - 5.57 (m, 1 H) 5.65 (d, J=7.81 Hz, 1 H) 5.80 - 5.98 (m, 2 H) 7.11 (d, J=8.44 Hz, 1 H) 7.28 (s, 1 H) 7.37 (br d, J=8.44 Hz, 1 H) 7.48 - 7.61 (m, 3 H) 7.92 (br t, J=5.56 Hz, 1 H) 8.26 - 8.37 (m, 3 H)); LCMS (ESI) m/z 764.6 (M + 2H)²⁺.

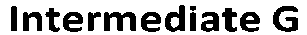

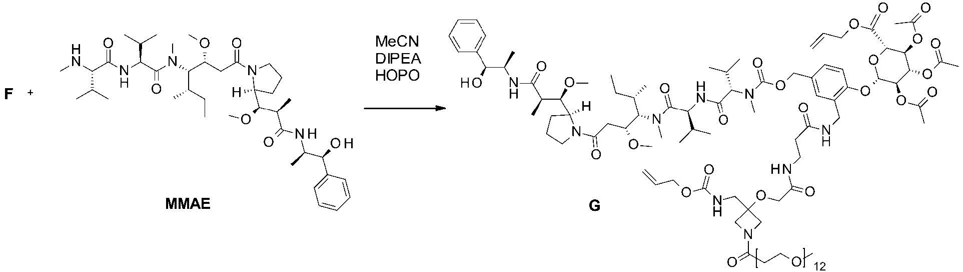

To a stirred mixture of **Intermediate F** (39.5 g, 76.0 w/w%, 20 mmol, 1.0eq), and MMAE (12.8 g, 24 mmol, 1.2 eq) in MeCN (20 V) at 25°C was added DIPEA (4.4g, 34 mmol, 1.7eq) dropwise, followed by HOPO (3.8 g, 34 mmol, 1.7 eq). The reaction mixture was stirred for 7 h at 25°C. The reaction was cooled to 10°C then NaCl (15% aq, 300 mL, 10 V) was charged. The pH of the reaction mixture was adjusted to pH 3-6 by the addition of HCl (1M, 9 mL, 0.6 V) at 10°C. The layers were allowed to separate and the aqueous layer discarded. The organic layer was concentrated to 3-5 V to give allyl (2*S*,3*S*,4*S*,5*R*,6*S*)-3,4,5-triacetoxy-6-[2-[[3-[[2-[3-[(allyloxycarbonylamino)methyl]-1-[3-[2-[2-[2-[2-[2-[2-[2-[2-[2-[2-(2-methoxyethoxy)ethoxy]ethoxy]ethoxy]ethoxy]ethoxy]ethoxy]ethoxy]ethoxy]ethoxy]ethoxy]propano yl]azetidin-3-yl]oxyacetyl]amino]propanoylamino]methyl]-4-[[[(15)-1-[[(15)-1-[[(15,2R)-4-[(25)-2-[(1R,2R)-3-[[(1R,25)-2-hydroxy-1-methyl-2-phenyl-ethyl]amino]-1-methoxy-2-methyl-3-oxopropyl]pyrrolidin-1-yl]-2-methoxy-1-[(1*S*)-1-methylpropyl]-4-oxo-butyl]-methyl-carbamoyl]-2-methyl-propyl]carbamoyl]-2-methyl-propyl]-methylcarbamoyl]oxymethyl]phenoxy]tetrahydropyran-2-carboxylate, **Intermediate G,** as an acetonitrile solution (112.3 g, 31.6 w/w%, 85.8% yield). A sample was concentrated to dryness to give a white solid for characterisation; ¹H NMR (500 MHz, DMSO-d6) δ ppm 0.72 - 0.94 (m, 18 H) 0.95 - 1.07 (m, 6 H) 1.30 (br s, 1 H) 1.44 - 1.60 (m, 2 H) 1.67 - 1.85 (m, 3 H) 1.95 - 2.02 (m, 6 H) 2.04 (s, 3 H) 2.05 - 2.14 (m, 2 H) 2.29 (br t, J=6.26 Hz, 3 H) 2.40 (br s, 3 H) 2.80 - 2.91 (m, 3 H) 2.97 (br s, 1 H) 3.00 - 3.09 (m, 1 H) 3.12 (br s, 2 H) 3.16 - 3.26 (m, 10 H) 3.28 - 3.36 (m, 3 H) 3.41 - 3.43 (m, 3 H) 3.50 (s, 43 H) 3.53 - 3.65 (m, 5 H) 3.71 - 3.82 (m, 3 H) 3.90 - 4.11 (m, 8 H) 4.16 - 4.30 (m, 2 H) 4.35 - 4.46 (m, 1 H) 4.46 - 4.54 (m, 4 H) 4.61 (br d, J=13.73 Hz, 2 H) 4.78 (br d, J=10.07 Hz, 2 H) 4.94 - 5.12 (m, 3 H) 5.15 - 5.19 (m, 2 H) 5.21 - 5.37 (m, 4 H) 5.41 - 5.53 (m, 2 H) 5.60 (br d, J=5.49 Hz, 1 H) 5.84 - 5.94 (m, 2 H) 7.04 (br d, J=5.19 Hz, 1 H) 7.13 - 7.23 (m, 3 H) 7.24 - 7.28 (m, 2 H) 7.29 - 7.32 (m, 2 H) 7.53 (br t, J=5.95 Hz, 1 H) 7.93 (br s, 2 H) 8.28 (br s, 1 H); LCMS (ESI) m/z 1054.1 (M + 2H)²⁺.

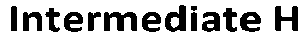

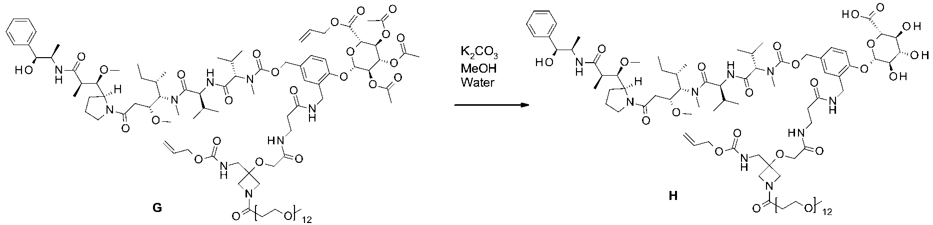

A stirred solution of **Intermediate G** (99g g, 31.6 w/w% in MeCN, 14.9 mmol, 1.0eq) was concentrated to dryness. MeOH (156 mL,5 V) was added and the mixture was again concentrated to dryness. The residue was dissolved in MeOH (313 mL, 10 V) at 5°C and to this solution was added a solution of K₂CO₃ (313 mL, 0.35M aq, 7.5 eq). The solution was stirred for 10 h at 23°C, then cooled to 0°C. Citric acid (20% aq, 250.4 mL, 8V) was added to adjust the pH to 3-5. The reaction mixture was washed twice with iPrOAc (2 x 313 mL; 2 x 10 V); Note product was in the aqueous layer. The aqueous phase was purified by reverse phase HPLC to give (2*S*,3*S*,4*S*,5*R*,6*S*)-6-[2-[[3-[[2-[3-[(allyloxycarbonylamino)methyl]-1-[3-[2-[2-[2-[2-[2-[2-[2-[2-[2-[2-(2-methoxyethoxy)ethoxy]ethoxy]ethoxy]ethoxy]ethoxy]ethoxy]ethoxy]ethoxy]ethoxy]ethoxy]propano yl]azetidin-3-yl]oxyacetyl]amino]propanoylamino]methyl]- 4-[[[(1*S*)-1-[[(1*S*)-1-[[(1*S*,2*R*)-4-[(2*S*)-2-[(1R,2R)-3-[[(1*R*,2*S*)-2-hydroxy-1-methyl-2-phenyl-ethyl]amino]-1-methoxy-2-methyl-3-oxopropyl]pyrrolidin-1-yl]-2-methoxy-1-[(1*S*)-1-methylpropyl]-4-oxo-butyl]-methyl-carbamoyl]-2-methyl-propyl]carbamoyl]-2-methyl-propyl]-methyl-carbamoyl]oxymethyl]phenoxy]-3,4,5-trihydroxy-tetrahydropyran-2-carboxylic acid, **Intermediate H,** as a solid (30.1g, 77.6 w/w%, 81.0 % yield); ¹H NMR (500 MHz, DMSO-d6) δ ppm 0.73 - 0.94 (m, 17 H) 0.95 - 1.06 (m, 6 H) 1.30 (br s, 1 H) 1.44 - 1.60 (m, 2 H) 1.67 - 1.85 (m, 3 H) 1.92 - 2.15 (m, 3 H) 2.23 - 2.45 (m, 6 H) 2.77 - 2.93 (m, 3 H) 2.97 (br s, 1 H) 3.00 - 3.09 (m, 1 H) 3.12 (br s, 2 H) 3.16 - 3.48 (m, 30 H) 3.55 - 3.60 (m, 3 H) 3.69 - 3.80 (m, 2 H) 3.84 - 4.05 (m, 5 H) 4.11 (br d, J=9.16 Hz, 1 H) 4.15 - 4.29 (m, 2 H) 4.33 - 4.51 (m, 4 H) 4.57 (br s, 1 H) 4.97 - 5.18 (m, 3 H) 5.27 (dd, J=17.09, 1.53 Hz, 1 H) 5.45 (br s, 1 H) 5.85 - 5.94 (m, 1 H) 7.06 (br dd, J=8.24, 3.97 Hz, 2 H) 7.12 - 7.29 (m, 5 H) 7.29 - 7.32 (m, 2 H) 7.36 - 7.59 (m 2 H) 7.93 (br s, 1 H)); LCMS (ESI) m/z 971.9 (M + 2H)²⁺.

### Intermediate 42 (Alternative route)

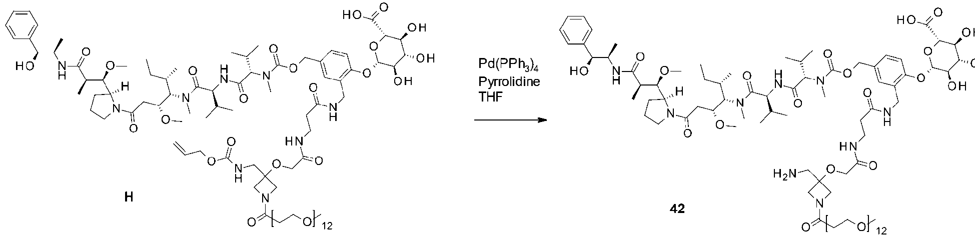

To a stirred solution of **Intermediate H** (17.1 g, 8.89 mMol, 1.0 eq) in THF ( 256.5 mL, 15V) at 0°C was added pyrrolidine (1.57 g, 22.02 mMol, 2.5 eq). The mixture was degassed three times with nitrogen, then Pd(PPh₃)₄ (0.51 g, 0.441 mMol, 0.05 eq) was added. The reaction mixture was stirred at 0°C for 2h. Water was charged (171 mL, 10V) and the pH was adjusted to 4-6 by adding HCl (1M aq, 3.4 mL, 0.2 V). The reaction mixture was extracted twice with toluene (2 x 171 mL, 2 x 10 V), the layers separated and the aqueous layer retained (note: product in aqueous). The aqueous phase was concentrated to dryness by freeze-drying to give (2~{S},3~{S},4~{S},5~{R},6~{S})-6-[2-[[3-[[2-[3-(aminomethyl)-1-[3-[2-[2-[2-[2-[2-[2-[2-[2-[2-[2-(2^{_} methoxyethoxy)ethoxy]ethoxy]ethoxy]ethoxy]ethoxy]ethoxy]ethoxy]ethoxy]ethoxy]ethoxy]propano yl]azetidin-3-yl]oxyacetyl]amino]propanoylamino]methyl]-4-[[[(1~{S})-1-[[(1~{S})-1-[[(1~{S},2~{R})-4-[(2~{S})-2-[(1~{R},2~{R})-3-[[(1~{R},2~{S})-2-hydroxy-1-methyl-2-phenyl-ethyl]amino]-1-methoxy-2-methyl-3-oxo-propyl]pyrrolidin-1-yl]-2-methoxy-1-[(1~{S})-1-methylpropyl]-4-oxo-butyl]-methylcarbamoyl]-2-methyl-propyl]carbamoyl]-2-methyl-propyl]-methyl-carbamoyl]oxymethyl]phenoxy]-3,4,5-trihydroxy-tetrahydropyran-2-carboxylic acid, **Intermediate 42,** as a solid (13.0g, 73.1 w/w%, 75.4 % yield).

### LP2 (Alternative Synthesis)

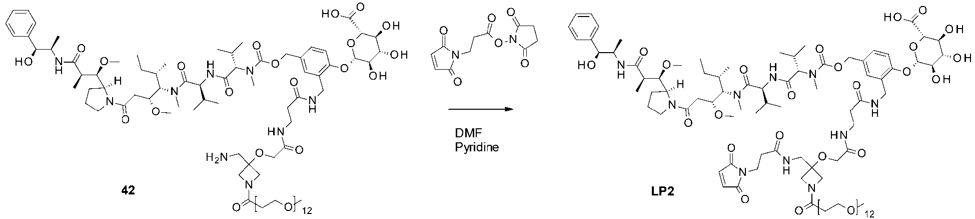

To a stirred solution of **Intermediate 42** (0.75 g, 404 µMol, 1.0 eq) and pyridine (0.38 mL, 0.5 V) in DMF (7.51 mL, 10 V) at 25°C was added (2,5-dioxopyrrolidin-1-yl) 3-(2,5-dioxopyrrol-1-yl)propanoate (215 mg, 809 µMol, 2.0 eq). The reaction mixture was stirred for 2 h at 25°C then purified directly by HPLC. Fractions containing the target compound were combined and isolated by freeze drying to give **LP2** as a solid (300 mg, 96.7 w/w%, 35.7 %yield).

### ADC Conjugation

### General Procedure (LP1-4, LP7 and LP8)

2.5-3.5 equiv. of TCEP (10mM in PBS pH 7.4, 1.25-1.60 nmol, 12.5 mL) was added to a solution of the antibody in reduction buffer containing PBS and 1 mM ethylenediaminetetraacetic acid (EDTA). The reduction mixture was allowed to react at RT for 2 hours with gentle shaking. 5-10 equiv. of Linker-Payload (**LP**) (10mM in DMA, 1-2 nmol, 80-160 mL) was added as a DMA solution to the reduced antibody solution (5mg/mL, 0.5 nmol), for a 10% (v/v) final DMA concentration. The resulting solution was mixed for 1 hour at room temperature. The DAR for the conjugate was assessed on a fully reduced reaction sample (DTT 1M, sodium borate buffer pH 8.5) using a UHPLC analysis on a SHIMADZU PROMINENCE system using a THERMO SCIENTIFIC MABPAC 50 mm x 2.1 mm column eluting with a gradient of water and acetonitrile, measured at 214 nm (see Table 1).

### Procedure for LP6

3.5 equiv. of TCEP (10mM in PBS pH 7.4, 1.65 nmol, 12.5 µL) was added to solution of antibody in reduction buffer containing PBS and 1 mM ethylenediaminetetraacetic acid (EDTA). The reduction mixture was allowed to react at RT for 2 hours with gentle shaking at 37 °C. The pH of the reduced antibody solution was adjusted to 8.5 using 10%v/v of 0.5M TRIS, next 13 equiv. of **LP6** (10mM in DMSO, 2.5 nmol, 170 µL) was added to the reduced antibody solution (5mg/mL, 0.5 nmol), for a 10% (v/v) final DMSO concentration. The resulting solution was mixed overnight at room temperature. The DAR for the conjugate was assessed on a fully reduced reaction sample (DTT 1M, sodium borate buffer pH 8.5) using a UHPLC analysis on a Shimadzu Prominence system using a Thermo Scientific MAbPac 50 mm x 2.1 mm column eluting with a gradient of water and acetonitrile, measured at 214 nm.

**Table 1- ADC Conjugation (LP1-4 and LP6-LP8)**

| ADC | mAb | TCEP equiv. | **LP** | **LP** equiv. | DAR (214nm) |
|---|---|---|---|---|---|
| **ADC1 (Control)** | Herceptin-WT | 2.6 | **LP1** | 6.5 | 4.0 |
| **ADC2** | Herceptin-WT | 3.2 | **LP2** | 5 | 3.7 |
| **ADC3** | Herceptin-WT | 2.5 | **LP3** | 10 | 3.6 |
| **ADC4** | Herceptin-WT | 2.5 | **LP4** | 10 | 3.8 |
| **ADC6** | Herceptin-WT | 3.5 | **LP6** | 13 | 4.2 |
| **ADC7 (Reference)** | Herceptin-WT | 2.5 | **LP7** | 7.5 | 3.6 |
| **ADC8 (Reference)** | Herceptin-WT | 2.5 | **LP8** | 7.5 | 3.7 |

### Procedure for LP5 (ADC5)

Conjugation of **LP5** using the above General Procedure gave a DAR that was below specification (DAR 3.1). Therefore the process was repeated on a further 8 mg scale using the same protocol except with 3.4 eq TCEP for reduction to generate higher DAR material (DAR 5.3) which was pooled to give DAR ^{~} 4.0.

### Procedure for LP9 (ADC9)

**LP9** was added as a DMSO solution (16 molar equivalent/antibody, 1.07 µmole, in 0.159 mL DMSO) to 1.82 mL of the Herceptin-wt antibody solution in PBS, 1 mM EDTA, pH 7.4 (10.0 mg, 66.7 nanomoles) for a 10% (*v*/*v*) final DMSO concentration. The solution left to react at room temperature for 1 hours with gentle shaking. Then the conjugation was quenched by addition of N-acetyl cysteine (5.3 micromoles, 53.40 mL at 100 mM), then purified in in PBS pH 7.4 by SECprep-AKTA and formulated in 20 mM His\His HCl, 240 mM sucrose pH 6.0 by spin filtration using a 15 mL Amicon Ultracell 30 kDa MWCO spin filter, sterile-filtered and analysed. UHPLC analysis on a Shimadzu Prominence system using a Proteomix HIC Butyl-NP5, 5um, non-porous, 4.6x35 mm (Sepax) column eluting with a gradient of 1.5M ammonium sulphate, 25 mM sodium acetate, pH 7.4 and 25 mM sodium acetate, pH 7.4 with 20% acetonitrile (v/v) on a neat sample of ADC at 214 nm showed singly conjugated to **LP9** , consistent with a drug-per-antibody ratio (DAR) of 8 molecules of **LP9** per antibody.

### Linker-Payload enzymatic cleavage

### β-glucuronidase

4 nM β-glucuronidase was added to 40 µM Linker-Payload (1:10,000) in 50 mM sodium acetate, pH 5.0. The mixture was incubated at 37 °C for a duration of 24 hours with intermediate timepoints collected and diluted 1:1 with buffer containing acetonitrile, water and formic acid. The sample was measured by reverse-phase HPLC with data quantified against a standard curve.

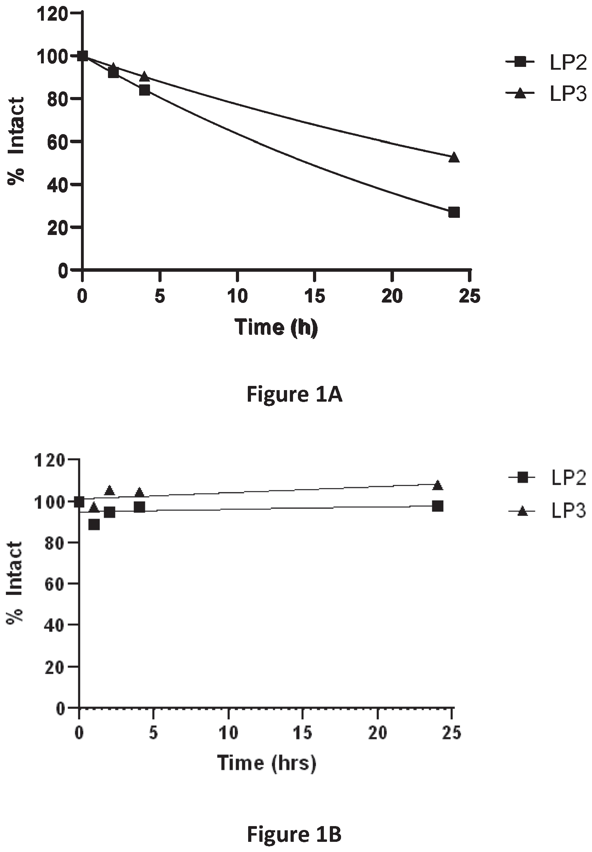

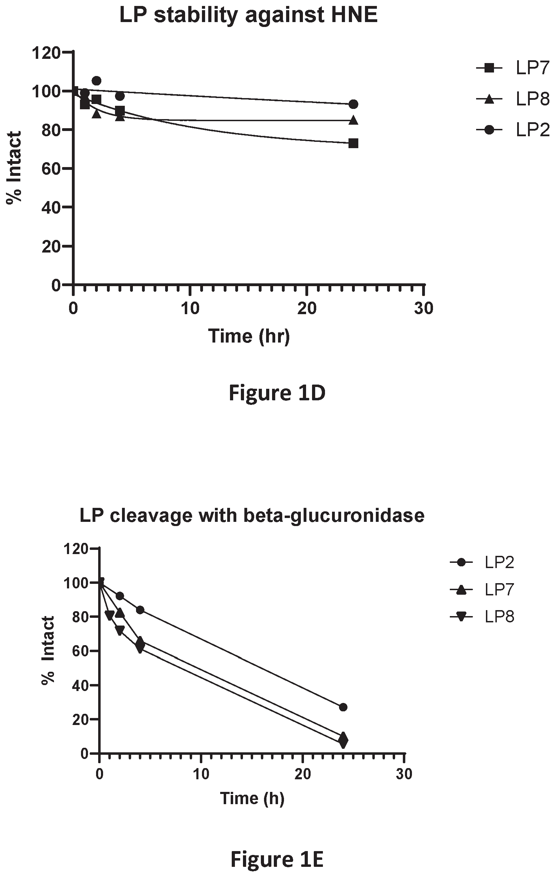

As shown in Figure 1A, both **LP2** and **LP3** underwent significant cleavage in the presence of β-glucuronidase. As shown in Figure 1E, **LP2** underwent slower cleavage than both **LP7** (Reference) and **LP8** (Reference) in the presence of β-glucuronidase.

### Cathepsin B

0.14 µM Cathepsin B was added to 70 µM Linker-Payload (1:500) in 50 mM sodium acetate, 1 mM DTT, pH 5.0. The mixture was incubated at 37 °C for a duration of 24 hours with intermediate timepoints collected and diluted 1:1 with buffer containing acetonitrile, water and formic acid. The sample was measured by reverse-phase HPLC with data quantified against a standard curve.

As shown in Figure 1B, neither **LP2** nor **LP3** underwent rapid cleavage in the presence of cathepsin B.

### Human Neutrophil Elastase

0.67 µM Human neutrophil elastase was added to 67 µM Linker-Payload (1:100) in Tris Buffered Saline, pH 7.4. The mixture was incubated at 37 °C for a duration of 24 hours with intermediate timepoints collected and diluted 1:1 with buffer containing acetonitrile, water and formic acid. The sample was measured by reverse-phase HPLC with data quantified against a standard curve.

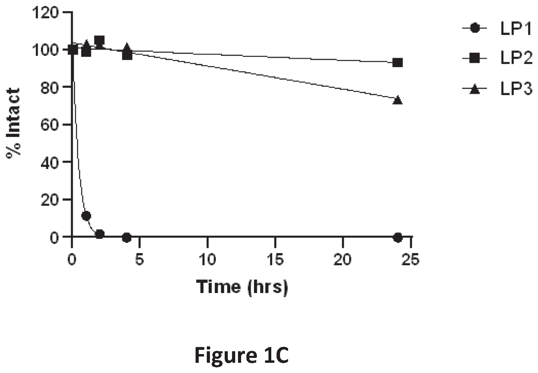

As shown in Figure 1C, **LP2** and **LP3** did not undergo rapid cleavage in the presence of human neutrophil elastase. **LP1** (control) underwent more rapid cleavage in the presence of human neutrophil elastase. As shown in Figure 1D, **LP2, LP7** (Reference) and **LP8** (Reference) did not undergo rapid cleavage in the presence of human neutrophil elastase.

### in vitro cytotoxicity data

Media from SKOV3 WT, SKOV3 GUSB KO (β-Glucuronidase knockout cell line, generated using CRISPR targeting), NCI-N87 and MDAMB468 cells at 80-90% confluency in a T175 flask was aspirated and the flask rinsed with PBS (about 10 ml) and emptied. TrypLE (5ml) Express Enzyme (1x) was added, the flask returned to the 37 °C incubator with 5% CO₂ for about 5 minutes, then the flask was shaken to detach the cells from the bottom. 10 mL cell media (McCoy's 5A supplemented with 50% Fetal Bovine Serum and RPMI 1640) was added to the flask and the cell suspension was transferred to a sterile 50 ml falcon tube, then centrifuged (400g for 5 min). The supernatant was aspirated, and the pellet re-suspended in 10mL culture medium. The cell suspension was well pipetted to break possible aggregates and 10µL solution were mixed with 10µL trypan blue cell-stained cells. 20µL mix were then transferred on a cell counting slide and the cell concentration and viability measured using the LUNA II. According to previous experiments that allowed us to determine the best seeding densities, SKOV3, SKOV3 GUSB KO and MDAMB468 were seeded at 3000cells/well while NCI-N87 at 10000cells/well in a 96-well microplate.

A stock solution (650µL) of antibody drug conjugate (ADC) was made by dilution of filter-sterilised ADC into cell culture medium. A set of 9x 5-fold dilutions of the previous ADC solution were made in a 2mL deep 96 well plate by serial transfer of 65 µl onto 585 µl of cell culture medium. ADC dilution was dispensed (50 µl/ well) into 2 replicate wells of the 96-well plate, containing 50 µl cell suspension seeded 24h (2D) and 48h (3D) before. Control wells received 50 µl cell culture medium. The 96-well plate containing cells and ADCs was incubated at 37 °C in a CO₂-gassed incubator for 6 days. At the end of the incubation period, plates were equilibrated to room temperature for 10min before CELLTITER-GLO^{™} Cell Viability Assay was dispensed (100 µl per well) into each well. Plates were pipette mixed for 5 minutes after which the plates were incubated for 25 minutes at room temperature. Well luminescence was measured, and percentage cell survival was calculated from the mean luminescence in the 2 ADC-treated wells compared to the mean luminescence in the 6 control untreated wells (100%). IC₅₀ was determined from the dose-response data using GRAPHPAD PRISM software v9 (GRAPHPAD, San Diego, CA) using the non-linear regression (curve fit) algorithm: Sigmoidal,4PL, X is log(concentration).

**Table 2- SKOV3 WT 3D and SKOV3 GUSB KO 3D in vitro cytotoxicity data**

| | **IC₅₀ SKOV3 WT 3D (µg/mL)** | **IC₅₀ SKOV3 GUSB KO 3D (µg/mL)** |
|---|---|---|
| **ADC1 (Control)** | 0.037 | 0.051 |
| **ADC2** | 0.071 | 32.4 |
| **ADC6** | 0.014 | N.A. |
| **ADC9** | 0.001 | 5.74 |

| | | |
|---|---|---|
| N.A.: data not available | | |

**Table 3- SKOV3 WT 2D, SKOV3 GUSB KO 2D, NCI-N87 2D and MDAMB468 2D in vitro cytotoxicity data for ADC1 and ADC2**

| | **IC₅₀ SKOV3 WT 2D (µg/mL)** | **IC₅₀ SKOV3 GUSB KO 2D (µg/mL)** | **IC₅₀ NCI-N87 2D (µg/mL)** | **IC₅₀ MDAMB468 2D (Her2- control) (µg/mL)** |
|---|---|---|---|---|
| **ADC1 (Control)** | 0.0080 | 0.012 | 0.015 | 6.53 |
| **ADC2** | 0.005 | >50 | 0.009 | 14.01 |

**Table 4- NCI-N87 2D and MDAMB468 2D in vitro cytotoxicity data for ADC4-ADC9**

| | **IC₅₀ NCI-N87 2D (µg/mL)** | **IC₅₀ MDAMB468 2D (Her2-control) (µg/mL)** |
|---|---|---|
| **ADC4** | 0.01 | 52.39 |
| **ADC5** | 0.006 | >50 |
| **ADC6** | 0.004 | 32.42 |
| **ADC7 (Reference)** | 0.004 | >50 |
| **ADC8 (Reference)** | 0.004 | >50 |
| **ADC9** | 0.12 | 9.025 |

### Hydrophobic interaction chromatography (HIC) Analysis

HIC analysis was performed using a Shimadzu Prominence system equipped with a Proteomix HIC Butyl-NP5, 5µm non-porous particles, 4.6x35mm, eluting with 0.8 mL/minute sterile-filtered HIC gradient buffer containing 1.5 M (NH₄)₂SO₄, 25 mM NaPO₄ (pH 7.40) and 80% 25 mM NaPO₄ (pH 7.40), *20% v*/*v* MeCN. Sample were diluted to 1mg/mL and further diluted 1:1 in HIC buffer (1:1 buffer A and B), elution profile was recorded at multiple wavelengths and reported at 214nm.

**Table 5**

| **DAR** | **ADC2** | | **ADC7 (Reference)** | | **ADC8 (Reference)** | |
|---|---|---|---|---|---|---|
| | **Rt** | **%Area** | **Rt** | **%Area** | **Rt** | **%Area** |
| **Run 1** | | | | | | |
| 0 | 2.495 | 3.356 | 2.489 | 8.817 | 2.487 | 8.404 |
| 1 | 2.785 | 3.231 | 2.824 | 2.457 | 2.821 | 2.803 |
| 2 | 3.33 | 17.395 | 3.371 | 35.864 | 3.367 | 34.847 |
| 3 | 3.845 | 7.116 | 3.906 | 6.045 | 3.905 | 6.509 |
| 4 | 4.339 | 32.267 | 4.444 | 35.191 | 4.439 | 34.668 |
| 5 | 4.663 | 3.099 | 5.097 | 4.442 | 5.086 | 4.689 |
| 6 | 5.14 | 23.91 | 5.235 | 5.442 | 5.229 | 5.57 |
| 7 | 5.419 | 4.796 | 5.441 | 0.836 | 5.425 | 1.406 |
| 8 | 5.629 | 4.83 | 5.694 | 0.906 | 5.687 | 1.104 |

| **Run 2** | | | | | | |
|---|---|---|---|---|---|---|
| 0 | 2.499 | 3.462 | 2.493 | 8.533 | 2.5 | 8.393 |
| 1 | 2.786 | 3.239 | 2.825 | 2.643 | 2.83 | 2.89 |
| 2 | 3.331 | 17.618 | 3.368 | 34.701 | 3.373 | 34.462 |
| 3 | 3.847 | 6.98 | 3.916 | 5.982 | 3.922 | 6.37 |
| 4 | 4.338 | 32.479 | 4.432 | 34.615 | 4.434 | 34.279 |
| 5 | 4.669 | 3.091 | 5.077 | 5.273 | 5.083 | 5.368 |
| 6 | 5.139 | 23.919 | 5.226 | 5.277 | 5.234 | 5.012 |
| 7 | 5.418 | 4.934 | 5.417 | 1.574 | 5.401 | 1.846 |
| 8 | 5.627 | 4.278 | 5.685 | 1.401 | 5.695 | 1.38 |

| **Run 3** | | | | | | |
|---|---|---|---|---|---|---|
| 0 | 2.501 | 3.526 | 2.496 | 9.188 | 2.499 | 8.448 |
| 1 | 2.788 | 3.336 | 2.827 | 2.664 | 2.829 | 3.036 |
| 2 | 3.332 | 17.768 | 3.368 | 36.346 | 3.368 | 34.441 |
| 3 | 3.849 | 6.89 | 3.922 | 5.711 | 3.925 | 6.198 |
| 4 | 4.336 | 32.138 | 4.427 | 35.017 | 4.431 | 34.037 |
| 5 | 4.678 | 3.274 | 5.076 | 4.975 | 5.079 | 5.581 |
| 6 | 5.14 | 23.818 | 5.228 | 4.77 | 5.232 | 5.006 |
| 7 | 5.422 | 4.513 | 5.433 | 0.902 | 5.409 | 1.762 |
| 8 | 5.632 | 4.736 | 5.689 | 0.427 | 5.692 | 1.492 |

The data in Table 5 show that in general, at a specific DAR, **ADC2** is less hydrophobic than **ADC7** (Reference) and **ADC8** (Reference).

The above description of illustrative embodiments is intended only to acquaint others skilled in the art with the Applicant's specification, its principles, and its practical application so that others skilled in the art may readily adapt and apply the specification in its numerous forms, as they may be best suited to the requirements of a particular use. This description and its specific examples, while indicating embodiments of this specification, are intended for purposes of illustration only. This specification, therefore, is not limited to the illustrative embodiments described in this specification, and may be variously modified. In addition, it is to be appreciated that various features of the specification that are, for clarity reasons, described in the context of separate embodiments, also may be combined to form a single embodiment. Conversely, various features of the specification that are, for brevity reasons, described in the context of a single embodiment, also may be combined to form sub-combinations thereof.

### Statements of Disclosure M

1M. A conjugate of Formula (IM)

   Ab - (G^{A}-J^{A}-D^{M})ₖ (IM)

   or a pharmaceutically acceptable salt thereof, wherein
   Ab is an antibody or antigen-binding fragment thereof,
   k is an integer from 1 to 10,
   each G^{A} is independently a conjugation group conjugated to the antibody or antigen-binding fragment thereof,
   each D^{M} is 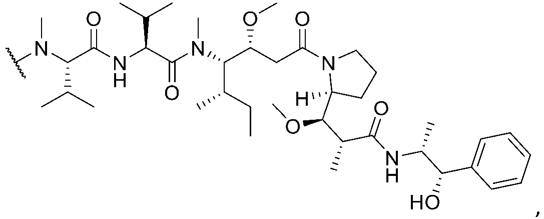
   each J^{A} is independently a group of Formula (IMA) 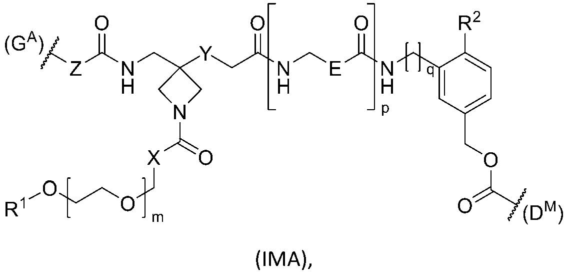
   E is (CH₂)ₙ₁, wherein n1 is 0, 1, 2 or 3,
   R¹ is C₁₋₄ alkyl or H,
   R² is 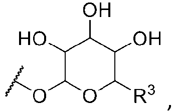 wherein R³ is CO₂H or CH₂OH,
   X is (CH₂)_{n2,} 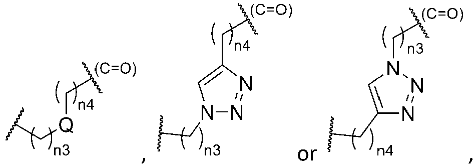 wherein n2 and n4 are 0, 1, 2 or 3, n3 is 1, 2 or 3, and Q is O or NR^{A}, wherein R^{A} is H, C₁₋₄ alkyl or C₃₋₄ cycloalkyl,
   Y is O or NR^{B}, wherein R^{B} is H, C₁₋₄ alkyl or C₃₋₄ cycloalkyl,
   Z is (CH₂)ₙ₅, wherein n5 is 0, 1, 2, 3, 4 or 5,
   m is an integer from 2 to 17,
   p is 1 or 0,
   q is 1 or 0,
   (G^{A}) indicates the point of attachment to G^{A}, and
   (D^{M}) indicates the point of attachment to D^{M.}
2M. A conjugate of Formula (IM) or a pharmaceutically acceptable salt thereof, as disclosed in statement 1M, wherein m is 9, 10, 11, 12 or 13.
3M. A conjugate of Formula (IM) or a pharmaceutically acceptable salt thereof, as disclosed in statement 1M or statement 2M, wherein R¹ is CH₃.
4M. A conjugate of Formula (IM) or a pharmaceutically acceptable salt thereof, as disclosed in any one of statements 1M to 3M, wherein R² is 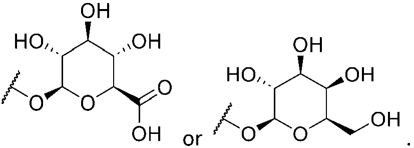
5M. A conjugate of Formula (IM) or a pharmaceutically acceptable salt thereof, as disclosed in any one of statements 1M to 4M, wherein E is CH₂.
6M. A conjugate of Formula (IM) or a pharmaceutically acceptable salt thereof, as disclosed in any one of statements 1M to 5M, wherein X is CH₂.
7M. A conjugate of Formula (IM) or a pharmaceutically acceptable salt thereof, as disclosed in any one of statements 1M to 6M, wherein Y is O.
8M. A conjugate of Formula (IM) or a pharmaceutically acceptable salt thereof, as disclosed in any one of statements 1M to 7, wherein n5 is 1, 2, 3, 4 or 5.
9M. A conjugate of Formula (IM) or a pharmaceutically acceptable salt thereof, as disclosed in any one of statements 1M to 8M, wherein Z is (CH₂)₂.
10M. A conjugate of Formula (IM) or a pharmaceutically acceptable salt thereof, as disclosed in any one of statements 1M to 9M, wherein p is 1.
11M. A conjugate of Formula (IM) or a pharmaceutically acceptable salt thereof, as disclosed in any one of statements 1M to 10M, wherein q is 1.
12M. A conjugate of Formula (IM) or a pharmaceutically acceptable salt thereof, as disclosed in statement 1, wherein each J^{A} is a group of Formula (IMB) 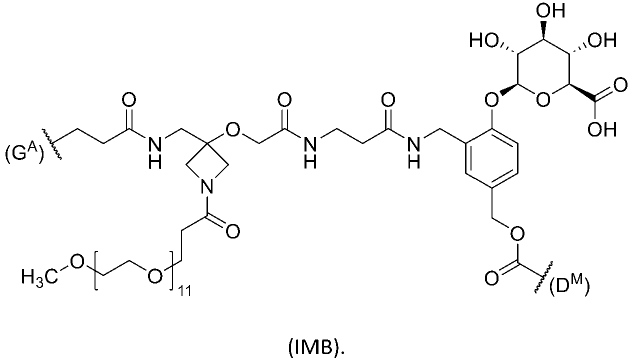
13M. A conjugate of Formula (IM) or a pharmaceutically acceptable salt thereof, as disclosed in any one of statements 1M to 12M, wherein G^{A} is selected from 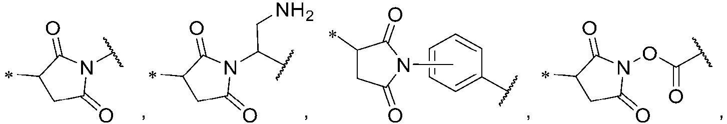 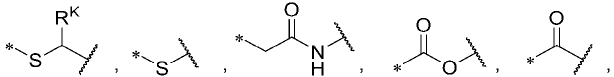 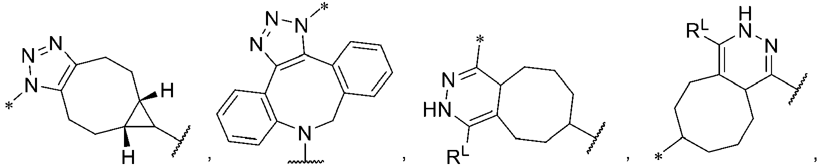 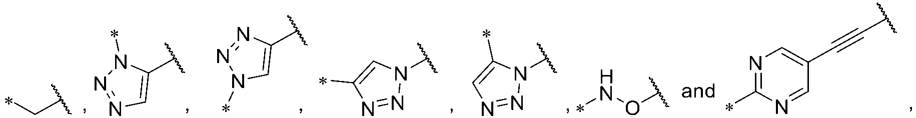 wherein R^{K} is H or CH₃, R^{L} is C₁₋₆ alkyl, and 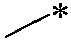 indicates the point of attachment to the antibody or antigen-binding fragment thereof.
14M. A conjugate of Formula (IM) or a pharmaceutically acceptable salt thereof, as disclosed in statement 13M, wherein G^{A} is 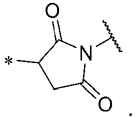
15M. A conjugate of Formula (IM) or a pharmaceutically acceptable salt thereof, as disclosed in statement 1M, wherein each (G^{A}-J^{A}-D^{M}) is 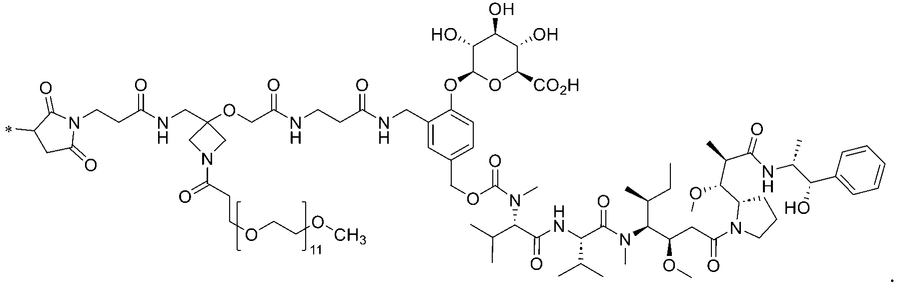
16M. A conjugate of Formula (IM) or a pharmaceutically acceptable salt thereof, as disclosed in statement 1M, wherein each (G^{A}-J^{A}-D^{M}) is 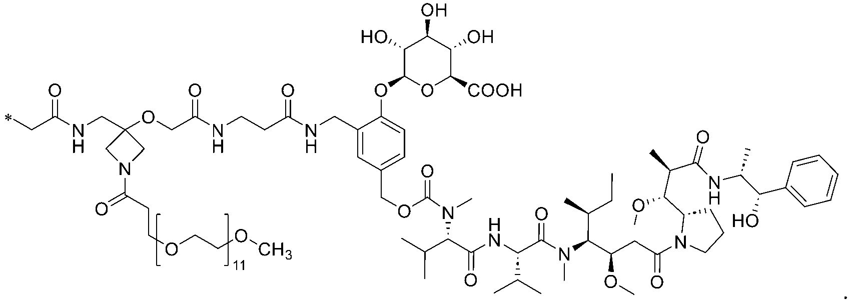
17M. A conjugate of Formula (IM) or a pharmaceutically acceptable salt thereof, as disclosed in any one of statements 1M to 16M, wherein k is an integer from 2 to 8.
18M. A pharmaceutical composition comprising a conjugate of Formula (IM) or a pharmaceutically acceptable salt thereof, as disclosed in any one of statements 1M to 17M, and a pharmaceutically acceptable excipient.
19M. A conjugate of Formula (IM) or a pharmaceutically acceptable salt thereof, as disclosed in any one of statements 1M to 17M, for use in therapy.
20M. A conjugate of Formula (IM) or a pharmaceutically acceptable salt thereof, as disclosed in any one of statements 1M to 17M, for use in the treatment of cancer.
21M. A method of treating cancer in a patient comprising administering to the patient a conjugate of Formula (IM) or a pharmaceutically acceptable salt thereof, as disclosed in any one of statements 1M to 17M.
22M. Use of a conjugate of Formula (IM) or a pharmaceutically acceptable salt thereof, as disclosed in any one of statements 1M to 17M, in the manufacture of a medicament for the treatment of cancer.
23M. A compound of Formula (IIM)

   G^{B}-J^{B}-D^{M} (IIM)

   or a salt thereof, wherein G^{B} is a conjugation group for conjugation to an antibody or antigen-binding fragment thereof,
   J^{B} is a group of Formula (IIMA) 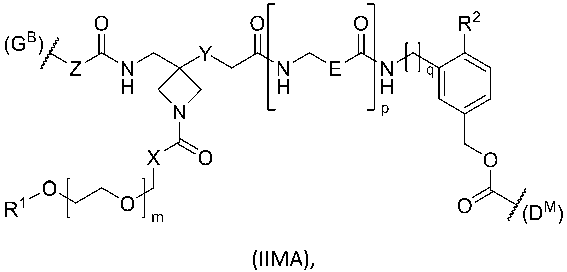
   wherein D^{M}, E, R¹, R², X, Y, Z, m, p and q are as defined for a conjugate of Formula (IM) in any one of statements 1M to 11M, (G^{B}) indicates the point of attachment to G^{B}, and (D^{M}) indicates the point of attachment to D^{M}.
24M. A compound of Formula (IIM) or a salt thereof, as disclosed in statement 20M, wherein G^{B} is selected from 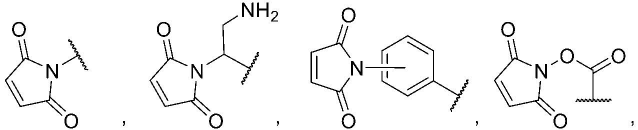 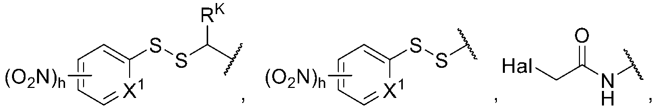 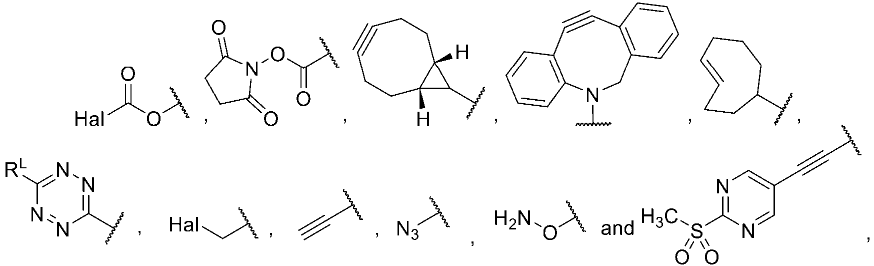
   wherein X¹ is CH or N,
   h is 0 or 1,
   Hal is Cl, Br or I,
   R^{K} is H or CH₃, and
   R^{L} is C₁₋₆ alkyl.
25M. A compound of Formula (IIM) or a salt thereof, as disclosed in statement 23M or statement 24M, wherein G^{B} is 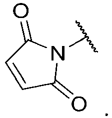
26M. A compound of Formula (IIM) or a salt thereof, as disclosed in statement 23M, that is 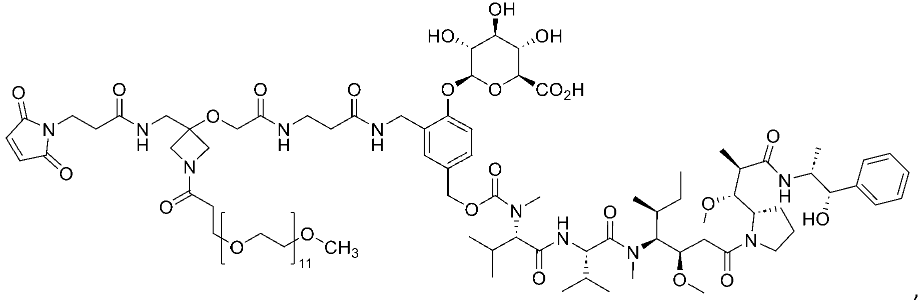 (2S,3S,4S,5R,6S)-6-(4-((5S,8S,11S,12R)-11-((S)-sec-butyl)-12-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-5,8-diisopropyl-4,10-dimethyl-3,6,9-trioxo-2,13-dioxa-4,7,10-triazatetradecyl)-2-((3-(2-((3-((3-(2,5-dioxo-2,5-dihydro-1H-pyrrol-1-yl)propanamido)methyl)-1-(2,5,8,11,14,17,20,23,26,29,32,35-dodecaoxaoctatriacontan-38-oyl)azetidin-3-yl)oxy)acetamido)propanamido)methyl)phenoxy)-3,4,5-trihydroxytetrahydro-2H-pyran-2-carboxylic acid, or a salt thereof.
27M. A compound of Formula (IIM) or a salt thereof, as disclosed in statement 23M, that is 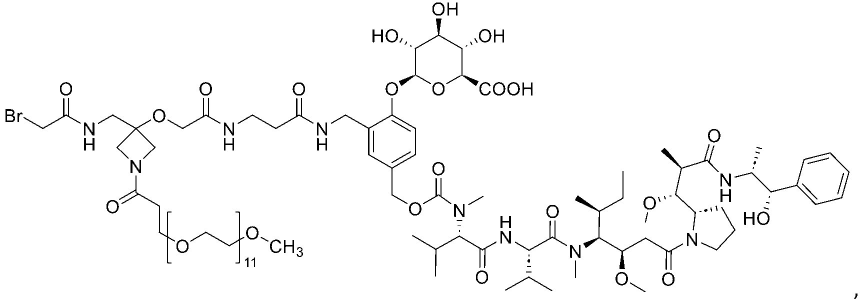 (2S,3S,4S,5R,6S)-6-(2-((3-(2-((3-((2-bromoacetamido)methyl)-1-(2,5,8,11,14,17,20,23,26,29,32,35-dodecaoxaoctatriacontan-38-oyl)azetidin-3-yl)oxy)acetamido)propanamido)methyl)-4-((5S,8S,11S,12R)-11-((S)-sec-butyl)-12-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-5,8-diisopropyl-4,10-dimethyl-3,6,9-trioxo-2,13-dioxa-4,7,10-triazatetradecyl)phenoxy)-3,4,5-trihydroxytetrahydro-2H-pyran-2-carboxylic acid, or a salt thereof.
28M. A compound of Formula (IVM)

   J^{IVM}-D^{M} (IVM)

   or a salt thereof, wherein J^{IVM} is a group of Formula (IVMA) 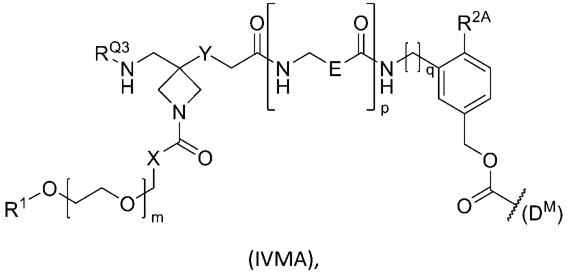
   wherein D^{M}, E, R¹, X, Y, m, p and q are as defined for a conjugate of Formula (IM) in any one of statements 1 to 3, statements 5 to 7, statement 10 or statement 11, R^{2A} is 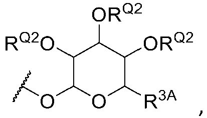
   wherein R^{3A} is CO₂R^{Q1} or CH₂OR^{Q2}, R^{Q1} is H or R^{P1}, each R^{Q2} is independently H or R^{P2}, and R^{Q3} is H or R^{P3}, wherein R^{P1} is a carboxylic acid protecting group, each R^{P2} is independently an alcohol protecting group and R^{P3} is an amine protecting group, and (D^{M}) indicates the point of attachment to D^{M}.

## Claims

1. A conjugate of Formula (I)
Ab - (G^{A}-J^{A}-D^{R})ₖ (I)
or a pharmaceutically acceptable salt thereof, wherein
Ab is an antibody or antigen-binding fragment thereof,
k is an integer from 1 to 10,
each G^{A} is independently a conjugation group conjugated to the antibody or antigen-binding fragment thereof,
each D^{R} is independently a drug comprising a nitrogen atom N^{R},
each J^{A} is independently a group of Formula (IA) 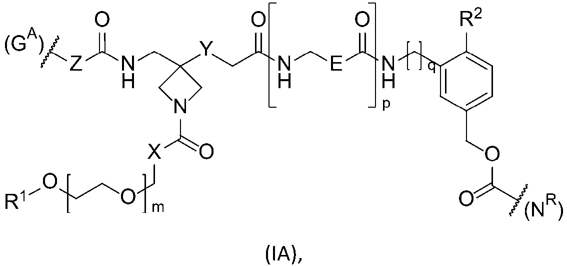
E is (CH₂)ₙ₁, wherein n1 is 0, 1, 2 or 3,
R¹ is C₁₋₄ alkyl or H,
R² is 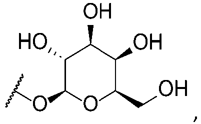
X is (CH₂)ₙ₂, 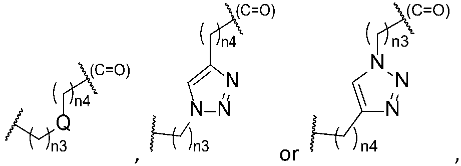 wherein n2 and n4 are 0, 1, 2 or 3, n3 is 1, 2 or 3, and Q is O or NR^{A}, wherein R^{A} is H, C₁₋₄ alkyl or C₃₋₄ cycloalkyl,
Y is O or NR^{B}, wherein R^{B} is H, C₁₋₄ alkyl or C₃₋₄ cycloalkyl,
Z is (CH₂)ₙ₅, wherein n5 is 0, 1, 2, 3, 4 or 5,
m is an integer from 2 to 17,
p is 1 or 0,
q is 1 or 0,
(G^{A}) indicates the point of attachment to G^{A}, and
(N^{R}) indicates the point of attachment to the nitrogen atom N^{R}.

2. A conjugate of Formula (I) or a pharmaceutically acceptable salt thereof, as claimed in claim 1, wherein (i) m is 9, 10, 11, 12 or 13, and/or (ii) R¹ is CH₃.

3. A conjugate of Formula (I) or a pharmaceutically acceptable salt thereof, as claimed in claim 1 or claim 2, wherein (i) E is CH₂, (ii) X is CH₂, and/or (iii) Y is O.

4. A conjugate of Formula (I) or a pharmaceutically acceptable salt thereof, as claimed in any one of claims 1 to 3, wherein Z is (CH₂) or (CH₂)₂.

5. A conjugate of Formula (I) or a pharmaceutically acceptable salt thereof, as claimed in any one of claims 1 to 4, wherein (i) p is 1, and/or (ii) q is 1.

6. A conjugate of Formula (I) or a pharmaceutically acceptable salt thereof, as claimed in claim 1, wherein each J^{A} is a group of Formula (IB3)

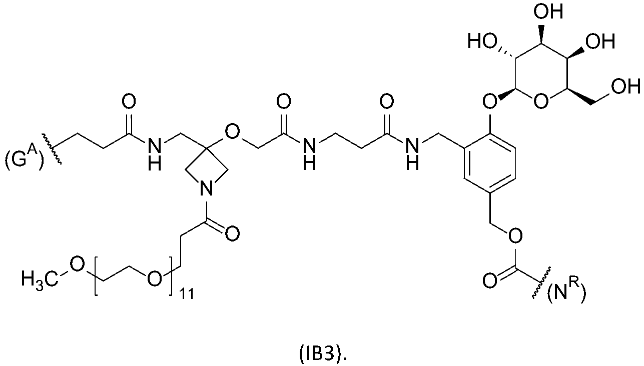

7. A conjugate of Formula (I) or a pharmaceutically acceptable salt thereof, as claimed in any one of claims 1 to 6, wherein G^{A} is selected from 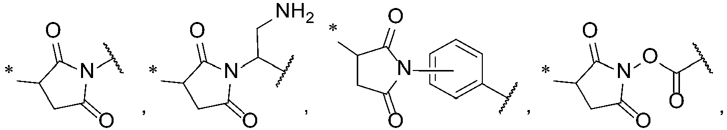 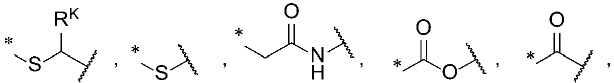 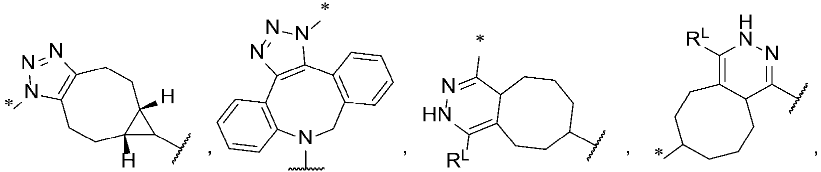 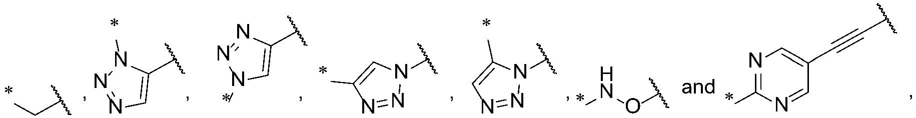 wherein R^{K} is H or CH₃, R^{L} is C₁₋₆ alkyl, and 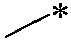 indicates the point of attachment to the antibody or antigen-binding fragment thereof.

8. A conjugate of Formula (I) or a pharmaceutically acceptable salt thereof, as claimed in claim 7, wherein G^{A} is

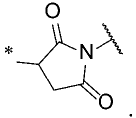

9. A conjugate of Formula (I) or a pharmaceutically acceptable salt thereof, as claimed in any one of claims 1 to 8, wherein k is an integer from 2 to 8.

10. A pharmaceutical composition comprising a conjugate of Formula (I) or a pharmaceutically acceptable salt thereof, as claimed in any one of claims 1 to 9, and a pharmaceutically acceptable excipient.

11. A conjugate of Formula (I) or a pharmaceutically acceptable salt thereof, as claimed in any one of claims 1 to 9, for use in (i) therapy, or (ii) the treatment of cancer.

12. A compound of Formula (II)
G^{B}-J^{B}-D^{R} (II)
or a salt thereof, wherein G^{B} is a conjugation group for conjugation to an antibody or antigen-binding fragment thereof,
D^{R} is a drug comprising a nitrogen atom N^{R},
J^{B} is a group of Formula (IIA) 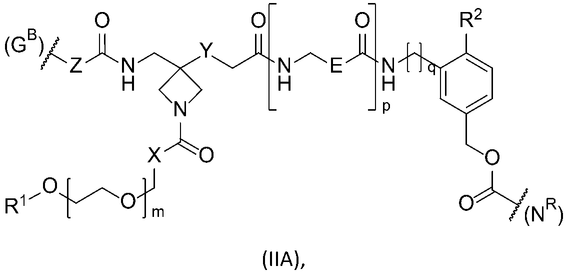
wherein E, R¹, R², X, Y, Z, m, p and q are as defined for a conjugate of Formula (I) in any one of claims 1 to 5, (G^{B}) indicates the point of attachment to G^{B}, and (N^{R}) indicates the point of attachment to the nitrogen atom N^{R}.

13. A compound of Formula (II) or a salt thereof, as claimed in claim 12, wherein G^{B} is selected from 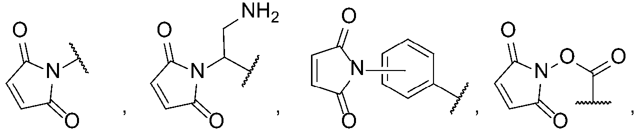 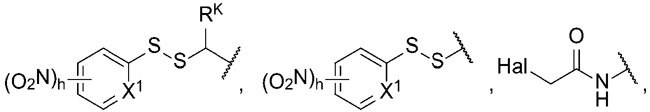 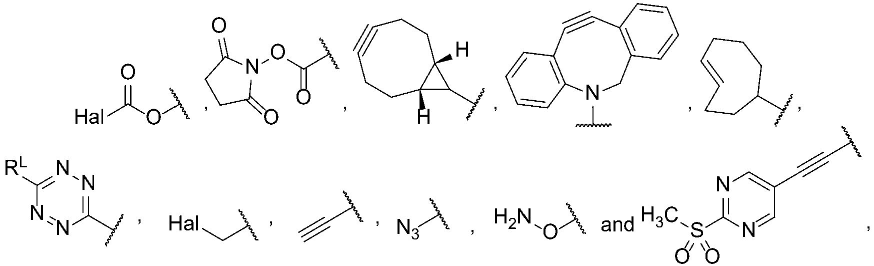
wherein X¹ is CH or N,
h is 0 or 1,
Hal is Cl, Br or I,
R^{K} is H or CH₃, and
R^{L} is C₁₋₆ alkyl.

14. A compound of Formula (II) or a salt thereof, as claimed in claim 12 or claim 13, wherein G^{B} is

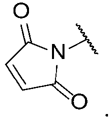

15. A compound of Formula (II) or a salt thereof, as claimed in any one of claims 12 to 14, wherein J^{B} is a group of Formula (IIB3)
